# EUROPEAN PATENT APPLICATION

(11) **EP 3 584 243 A1**
(43) Date of publication of application: **25.12.2019**
(21) Application number: 18754806.0
(22) Date of filing: 13.02.2018
(51) Int. Cl.: C07D 307/91, H01L 51/50

(54) **NOVEL COMPOUND, ORGANIC ELECTROLUMINESCENT ELEMENT USING SAME, AND ELECTRONIC DEVICE**

(30) Priority: 14.02.2017 JP 2017025460
(71) Applicant: Idemitsu Kosan Co., Ltd, Tokyo 100-8321 (JP)
(72) Inventor: NAKANO, Yuki, Sodegaura-shi Chiba 299-0293 (JP); TASAKI, Satomi, Sodegaura-shi Chiba 299-0293 (JP); SEDA, Keita, Sodegaura-shi Chiba 299-0293 (JP); TAKAHASHI, Ryota, Sodegaura-shi Chiba 299-0293 (JP)
(74) Representative: Hoffmann Eitle
(86) International application number: PCT/JP2018/004828
(87) International publication number: WO 2018/151077

(57) **Abstract**

To provide a compound represented by the following formula (1). At least one of R₁ to R₁₀ is a group represented by the following formula (2). At least one of Ar₁ and Ar₂ is a group represented by the following formula (3).

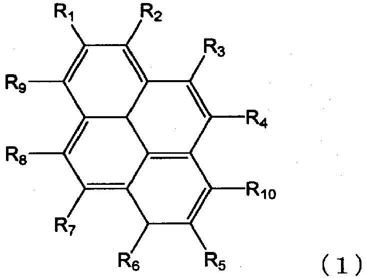

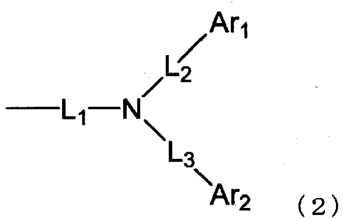

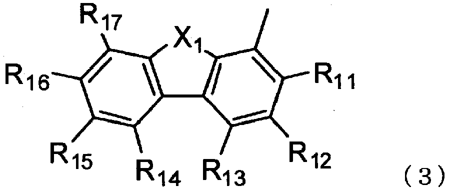

## Description

### Technical Field

The invention relates to a novel compound, an organic electroluminescent element using the same and an electronic device.

### Background Art

When voltage is applied to an organic electroluminescent element (hereinafter, referred to as an organic EL element), holes and electrons are injected into an emitting layer from an anode and a cathode, respectively. Then, thus injected holes and electrons are recombined in the emitting layer, and excitons are formed therein.

The organic EL element includes the emitting layer between the anode and the cathode. Further, the organic EL element has a stacked structure including an organic layer such as a hole-injecting layer, a hole-transporting layer, an electron-injecting layer, and an electron-transporting layer in several cases.

Patent Document 1 discloses a specific aromatic amine derivative as a material for an organic EL element.

### Related Art Documents

### Patent Documents

Patent Document 1: WO2013/077405

### Summary of the Invention

An object of the invention is to provide a compound from which an organic EL element having high luminous efficiency can be obtained, an organic electroluminescent element using the same and an electronic device.

One aspect of the invention provides the following compound.

A compound, represented by the following formula (1): 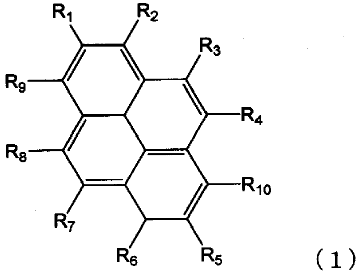 wherein, in the formula (1), R₁ to R₁₀ are independently a hydrogen atom, a halogen atom, a cyano group, a substituted or unsubstituted aryl group having 6 to 50 carbon atoms that form a ring (hereinafter referred to as "ring carbon atoms"), a substituted or unsubstituted heteroaryl group having 5 to 50 atoms that form a ring (hereinafter referred to as "ring atoms"), a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a substituted or unsubstituted alkylthio group having 1 to 50 carbon atoms, a substituted or unsubstituted arylthio group having 6 to 50 ring carbon atoms, or a group represented by the following formula (2); and at least one of R₁ to R₁₀ is a group represented by the following formula (2): 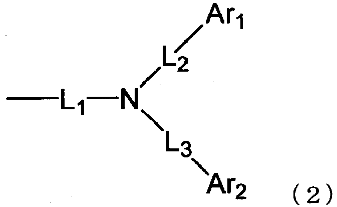
wherein, in the formula (2), Ar₁ and Ar₂ independently represent a substituted or unsubstituted aryl group having 6 to 50 ring carbon atoms, a substituted or unsubstituted heteroaryl group having 5 to 50 ring atoms, or a group represented by the following formula (3), and at least one of Ar₁ and Ar₂ is a group represented by the following formula (3);
L₁, L₂ and L₃ are independently a single bond, a substituted or unsubstituted arylene group having 6 to 30 ring carbon atoms, or a substituted or unsubstituted heteroarylene group having 5 to 30 ring atoms;when the number of Ar₁, Ar₂, L₁, L₂ and L₃ is two or more, two or more of Ar₁, Ar₂, L₁, L₂ and L₃ may be the same with or different from each other: 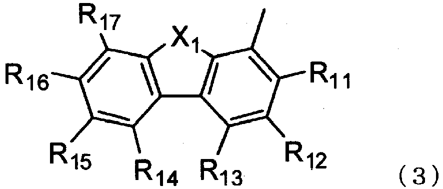
wherein, in the formula (3), R₁₁ represents a substituted or unsubstituted alkyl group having 1 to 50 carbon atoms, a substituted or unsubstituted aryl group having 6 to 50 ring carbon atoms, or a substituted or unsubstituted heteroaryl group having 5 to 50 ring atoms; R₁₂ to R₁₇ independently represent a hydrogen atom, a halogen atom, a cyano group, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a substituted or unsubstituted alkylthio group having 1 to 50 carbon atoms, or a substituted or unsubstituted arylthio group having 6 to 50 ring carbon atoms; and R₁₂ to R₁₇ may form a saturated or unsaturated ring between adjacent substituents; and
X₁ represents an oxygen atom or a sulfur atom.

Another aspect of the invention provides the following organic EL element.

An organic electroluminescent element, comprising:
a cathode;
an anode; and
an emitting layer arranged between the cathode and the anode,
wherein the emitting layer includes the above-described compound.

Another aspect of the invention provides an electronic device, comprising the organic EL element.

According to the invention, a compound from which an organic EL element having high luminous efficiency can be obtained, an organic electroluminescent element using the same and an electronic device can be provided.

### Brief Description of the Drawings

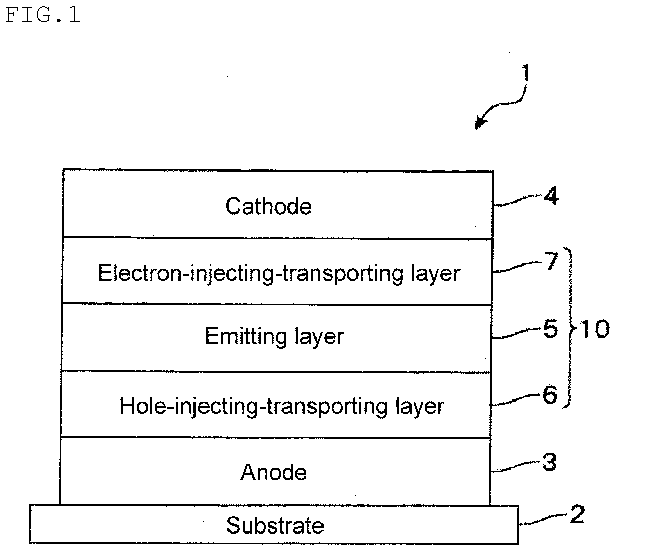

Fig. 1 is a diagram showing a schematic configuration of one embodiment of an organic EL element of the invention.

### Mode for Carrying out the Invention

In the present specification, a hydrogen atom means an atom including isomers different in the number of neutrons, namely, a protium, a deuterium and a tritium.

In the present specification, a term "ring carbon atoms" represents the number of carbon atoms among atoms forming a subject ring itself of a compound having a structure in which atoms are bonded in a ring form (for example, a monocyclic compound, a fused-ring compound, a cross-linked compound, a carbocyclic compound or a heterocyclic compound). When the subject ring is substituted with a substituent, the carbon contained in the substituent is not included in the number of ring carbon atoms. The same shall apply to the "ring carbon atoms" described below, unless otherwise noted. For example, a benzene ring has 6 ring carbon atoms, a naphthalene ring has 10 ring carbon atoms, a pyridinyl group has 5 ring carbon atoms, and a furanyl group has 4 ring carbon atoms. Further, when an alkyl group is substituted as a substituent in the benzene ring or the naphthalene ring, for example, the number of carbon atoms of the alkyl group is not included in the ring carbon atoms. Further, when a fluorene ring is bonded with a fluorene ring as a substituent (including a spirofluorene ring), for example, the number of carbon atoms of the fluorene ring as the substituent is not included in the ring carbon atoms.

In the present specification, a term "ring atoms" represents the number of atoms forming a subject ring itself of a compound having a structure in which atoms are bonded in a ring form (for example, a monocycle, a fused ring and a ring assembly) (for example, a monocyclic compound, a fused-ring compound, a cross-linked compound, a carbocyclic compound or a heterocyclic compound). The term "ring atoms" does not include atoms which do not form the ring (for example, a hydrogen atom which terminates a bonding hand of the atoms forming the ring) or atoms contained in a substituent when the ring is substituted with the substituent. The same shall apply to the "ring atoms" described below, unless otherwise noted. For example, a pyridine ring has 6 ring atoms, a quinazoline ring has 10 ring atoms, and a furan ring has 5 ring atoms. A hydrogen atom bonded with a carbon atom of the pyridine ring or the quinazoline ring or an atom forming the substituent is not included in the number of the ring atoms. Further, when a fluorene ring is bonded with a fluorene ring as a substituent (including a spirofluorene ring), for example, the number of atoms of the fluorene ring as the substituent is not included in the ring atoms.

In the present specification, a term "XX to YY carbon atoms" in an expression of "substituted or unsubstituted ZZ group having XX to YY carbon atoms" represents the number of carbon atoms when the ZZ group is unsubstituted. The number of carbon atoms of a substituent when the ZZ group is substituted with the substituent is not included. Here, "YY" is larger than "XX", and "XX" and "YY" mean an integer of 1 or more.

In the present specification, a term "XX to YY atoms" in an expression of "substituted or unsubstituted ZZ group having XX to YY atoms" represents the number of atoms when the ZZ group is unsubstituted. The number of atoms of a substituent when the group is substituted with the substituent is not included. Here, "YY" is larger than "XX", and "XX" and "YY" each mean an integer of 1 or more.

A term "unsubstituted" in the case of "substituted or unsubstituted" means that the relevant group is not substituted with a substituent, and a hydrogen atom is bonded therewith.

In the present specification, a heteroaryl group also includes the following groups. Further, a heteroarylene group also includes a group in which the following groups are formed into a divalent group.

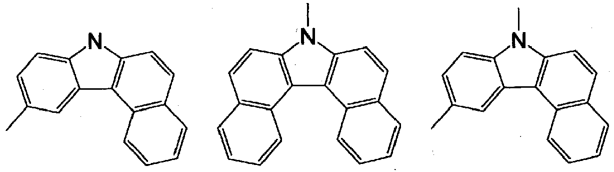

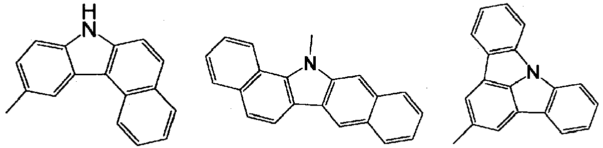

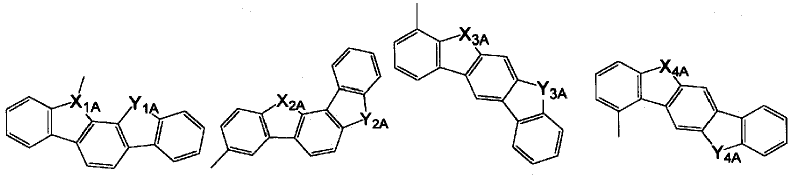

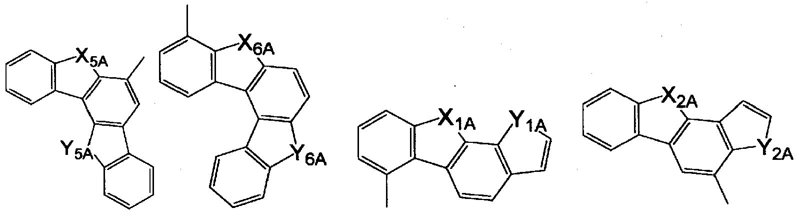

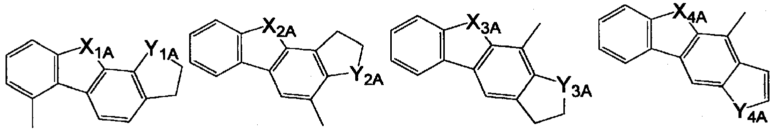

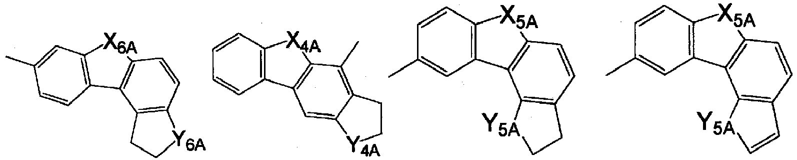

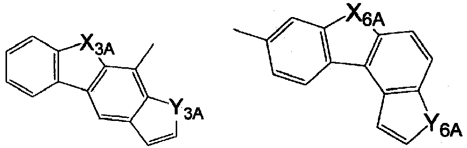

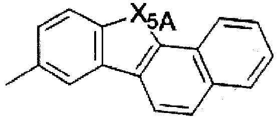

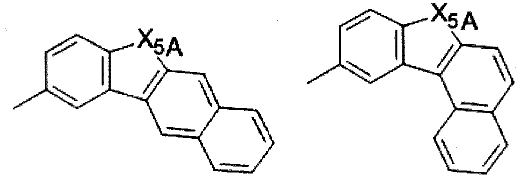

In the formulas, X_{1A} to X_{6A} and Y_{1A} to Y_{6A} are independently an oxygen atom, a sulfur atom, a -NZ-group or a -NH- group. Z is a substituted or unsubstituted aryl group having 6 to 50 ring carbon atoms, a substituted or unsubstituted heteroaryl group having 5 to 50 ring atoms, or a substituted or unsubstituted alkyl group having 1 to 50 carbon atoms. When the number of Z is two or more, two or more of Z may be the same with or different from each other.

Specific examples of the aryl group having 6 to 50 ring carbon atoms of Z include a group similar to an aryl group having 6 to 50 ring carbon atoms of R₁ to R₁₀ and the like described later.

Specific examples of the heteroaryl group having 5 to 50 ring atoms of Z include a group similar to a heteroaryl group having 5 to 50 ring atoms of R₁ to R₁₀ and the like described later.

Specific examples of the alkyl group having 1 to 50 carbon atoms of Z include a group similar to an alkyl group having 1 to 50 carbon atoms of R₁ to R₁₀ and the like described later.

One aspect of the compound of the invention is represented by the following formula (1): 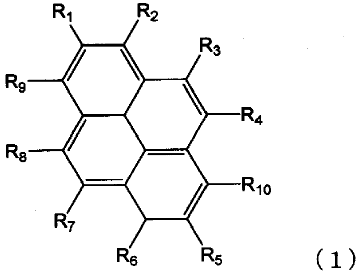 wherein, in the formula (1), R₁ to R₁₀ independently represent a hydrogen atom, a halogen atom, a cyano group, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a substituted or unsubstituted alkylthio group having 1 to 50 carbon atoms, a substituted or unsubstituted arylthio group having 6 to 50 ring carbon atoms, or a group represented by the following formula (2); and at least one (preferably two) of R₁ to R₁₀ is a group represented by the following formula (2): 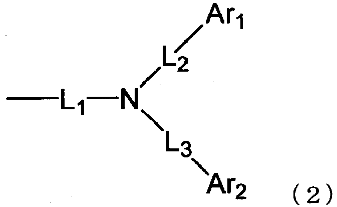
wherein, in the formula (2), Ar₁ and Ar₂ independently represent a substituted or unsubstituted aryl group having 6 to 50 ring carbon atoms, a substituted or unsubstituted heteroaryl group having 5 to 50 ring atoms, or a group represented by the following formula (3), and at least one (or both) of Ar₁ and Ar₂ is a group represented by the following formula (3);
L₁, L₂ and L₃ are independently a single bond, a substituted or unsubstituted arylene group having 6 to 30 ring carbon atoms, or a substituted or unsubstituted heteroarylene group having 5 to 30 ring atoms; and
when the number of Ar₁, Ar₂, L₁, L₂ and L₃ is two or more, two or more of Ar₁, Ar₂, L₁, L₂ and L₃ may be the same with or different from each other: 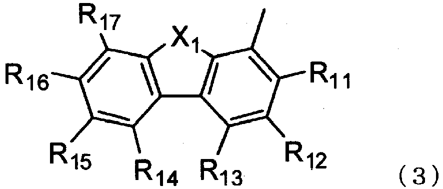
   wherein, in the formula (3), R₁₁ represents a substituted or unsubstituted alkyl group having 1 to 50 carbon atoms, a substituted or unsubstituted aryl group having 6 to 50 ring carbon atoms, or a substituted or unsubstituted heteroaryl group having 5 to 50 ring atoms; R₁₂ to R₁₇ independently represent a hydrogen atom, a halogen atom, a cyano group, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a substituted or unsubstituted alkylthio group having 1 to 50 carbon atoms, or a substituted or unsubstituted arylthio group having 6 to 50 ring carbon atoms; and R₁₂ to R₁₇ may form a saturated or unsaturated ring (preferably a saturated or unsaturated and substituted or unsubstituted 5-membered ring or 6-membered ring, and more preferably a benzene ring) between adjacent substituents; and
   X₁ represents an oxygen atom or a sulfur atom.

Thus, an organic EL element having high luminous efficiency can be obtained.

X₁ is preferably an oxygen atom.

L₁ is preferably a single bond.

Ar₁ is preferably a group represented by the formula (3), and Ar₂ is preferably a substituted or unsubstituted aryl group having 6 to 50 ring carbon atoms.

L₂ and L₃ are preferably a single bond.

Two of R₁ to R₁₀ are preferably a group represented by the formula (2).

R₁₂ to R₁₇ preferably do not form a saturated or unsaturated ring between adjacent substituents.

A compound represented by the formula (1) is preferably represented by the following formula (4A). 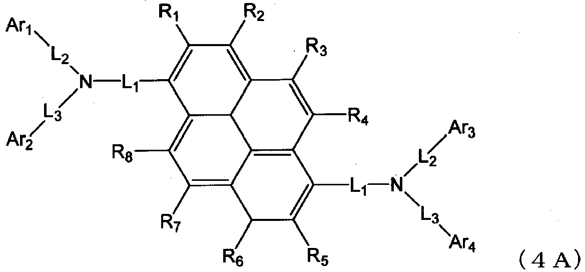
Wherein, in the formula (4A), R₁ to R₈ are as defined in the formula (1); Ar₁, Ar₂, L₁, L₂ and L₃ are as defined in the formula (2); and
Ar₃ and Ar₄ independently represent a substituted or unsubstituted aryl group having 6 to 50 ring carbon atoms, a substituted or unsubstituted heteroaryl group having 5 to 50 ring atoms, or a group represented by the formula (3), and
at least one of Ar₃ and Ar₄ is a group represented by the formula (3).

A compound represented by the formula (1) is preferably represented by the following formula (4B). 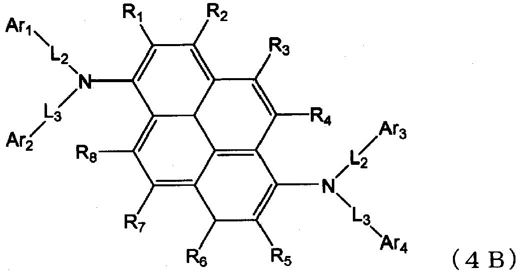
Wherein, in the formula (4B), R₁ to R₈ are as defined in the formula (1); Ar₁, Ar₂, L₂ and L₃ are as defined in the formula (2); and
Ar₃ and Ar₄ independently represent a substituted or unsubstituted aryl group having 6 to 50 ring carbon atoms, a substituted or unsubstituted heteroaryl group having 5 to 50 ring atoms, or a group represented by the formula (3), and
at least one of Ar₃ and Ar₄ is a group represented by the formula (3).

A compound represented by the formula (1) is preferably represented by the following formula (4). 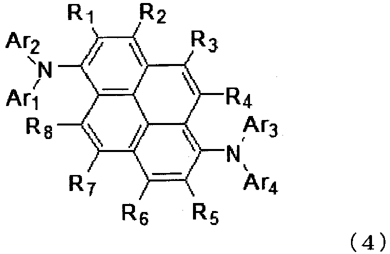
Wherein, in the formula (4), R₁ to R₈ are as defined in the formula (1); Ar₁ and Ar₂ are as defined in the formula (2); and
Ar₃ and Ar₄ independently represent a substituted or unsubstituted aryl group having 6 to 50 ring carbon atoms, a substituted or unsubstituted heteroaryl group having 5 to 50 ring atoms, or a group represented by the formula (3), and
at least one (or both) of Ar₃ and Ar₄ is a group represented by the formula (3).

It is preferable that R₁ to R₈ are independently a hydrogen atom, or a substituted or unsubstituted alkyl group having 1 to 50 carbon atoms;
Ar₁ and Ar₃ are independently a group represented by the formula (3);
Ar₂ and Ar₄ are independently a substituted or unsubstituted aryl group having 6 to 50 ring carbon atoms;
R₁₁ is independently a substituted or unsubstituted alkyl group having 1 to 50 carbon atoms, or a substituted or unsubstituted aryl group having 6 to 50 ring carbon atoms; and
R₁₂ to R₁₇ are independently a hydrogen atom, a substituted or unsubstituted alkyl group having 1 to 50 carbon atoms, or a substituted or unsubstituted aryl group having 6 to 50 ring carbon atoms.

It is preferable that R₁ to R₈ are independently a hydrogen atom, or a substituted or unsubstituted alkyl group having 1 to 18 carbon atoms; Ar₁ and Ar₃ are independently a group represented by the formula (3); Ar₂ and Ar₄ are independently a substituted or unsubstituted aryl group having 6 to 18 ring carbon atoms; R₁₁ is independently a substituted or unsubstituted alkyl group having 1 to 18 carbon atoms, or a substituted or unsubstituted aryl group having 6 to 18 ring carbon atoms; and R₁₂ to R₁₇ are independently a hydrogen atom, a substituted or unsubstituted alkyl group having 1 to 18 carbon atoms, or a substituted or unsubstituted aryl group having 6 to 18 ring carbon atoms.

It is preferable that Ar₂ and Ar₄ are independently a substituted or unsubstituted phenyl group, a substituted or unsubstituted biphenyl group, or a substituted or unsubstituted naphthyl group.

As a substituent when Ar₂ and Ar₄ have the substituent, an alkyl group having 1 to 18 (preferably 1 to 8) carbon atoms is preferable, in which 1 or 2 or more of the alkyl groups may be substituted. Specific examples of Ar₂ and Ar₄ when Ar₂ and Ar₄ have the substituent include a methylphenyl group, a dimethylphenyl group and a methylbiphenyl group.

R₁₁ is preferably a substituted or unsubstituted alkyl group having 1 to 18 carbon atoms. R₁₁ is more preferably a substituted or unsubstituted alkyl having 1 to 8 carbon atoms.

R₁ to R₈ are preferably a hydrogen atom, or a substituted or unsubstituted alkyl group having 1 to 18 (preferably 1 to 8) carbon atoms. Among R₁ to R₈, R₂ and R₆ may be a substituted or unsubstituted alkyl group having 1 to 18 (preferably 1 to 8) carbon atoms, and R₁, R₃ to R₅, R₇ and R₈ may be a hydrogen atom.

R₁₂ to R₁₇ are preferably a hydrogen atom, or a substituted or unsubstituted alkyl group having 1 to 18 (preferably 1 to 8) carbon atoms. Among R₁₂ to R₁₇, R₁₇ may be a substituted or unsubstituted alkyl group having 1 to 18 (preferably 1 to 8) carbon atoms, and R₁₂ to R₁₆ may be a hydrogen atom.

A compound represented by the formula (1) is preferably represented by any one of the following formulas (5A) to (5D): 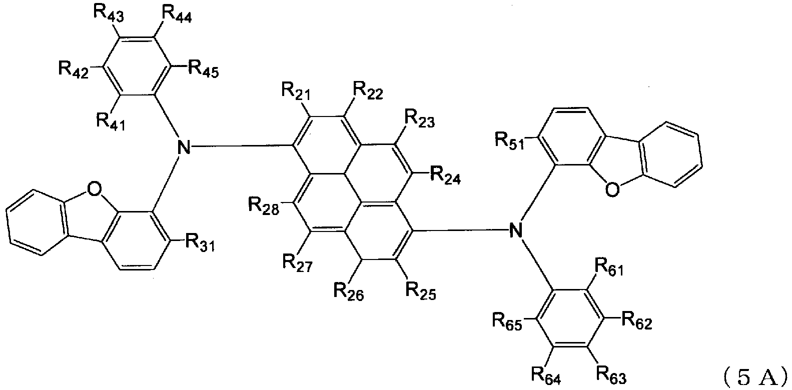
wherein, in the formula (5A), R₂₁ to R₂₈ are independently a hydrogen atom, or a substituted or unsubstituted alkyl group having 1 to 50 carbon atoms;
R₃₁ is a substituted or unsubstituted alkyl group having 1 to 50 carbon atoms;
R₄₁ to R₄₅ are independently a hydrogen atom, or a substituted or unsubstituted alkyl group having 1 to 50 carbon atoms;
R₅₁ is a substituted or unsubstituted alkyl group having 1 to 50 carbon atoms; and
R₆₁ to R₆₅ are independently a hydrogen atom, or a substituted or unsubstituted alkyl group having 1 to 50 carbon atoms.

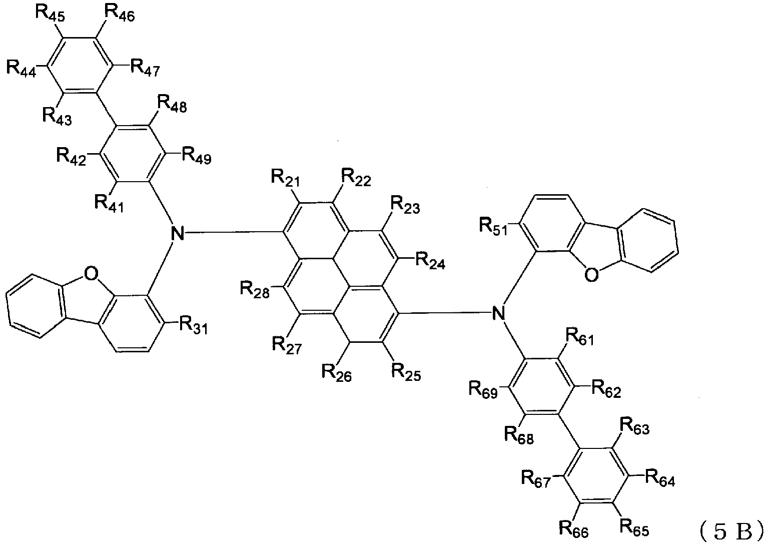

Wherein, in the formula (5B), R₂₁ to R₂₈ are independently a hydrogen atom, or a substituted or unsubstituted alkyl group having 1 to 50 carbon atoms;
R₃₁ is a substituted or unsubstituted alkyl group having 1 to 50 carbon atoms;
R₄₁ to R₄₉ are independently a hydrogen atom, or a substituted or unsubstituted alkyl group having 1 to 50 carbon atoms;
R₅₁ is a substituted or unsubstituted alkyl group having 1 to 50 carbon atoms; and
R₆₁ to R₆₉ are independently a hydrogen atom, or a substituted or unsubstituted alkyl group having 1 to 50 carbon atoms.

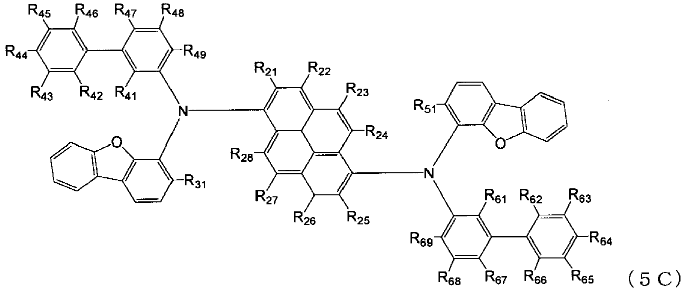

Wherein, in the formula (5C), R₂₁ to R₂₈ are independently a hydrogen atom, or a substituted or unsubstituted alkyl group having 1 to 50 carbon atoms;
R₃₁ is a substituted or unsubstituted alkyl group having 1 to 50 carbon atoms;
R₄₁ to R₄₉ are independently a hydrogen atom, or a substituted or unsubstituted alkyl group having 1 to 50 carbons;
R₅₁ is a substituted or unsubstituted alkyl group having 1 to 50 carbon atoms; and
R₆₁ to R₆₉ are independently a hydrogen atom, or a substituted or unsubstituted alkyl group having 1 to 50 carbon atoms.

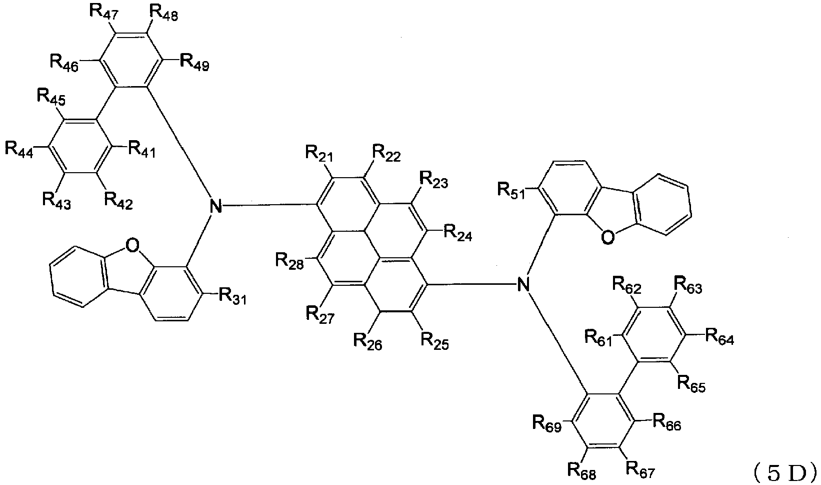

Wherein, in the formula (5D), R₂₁ to R₂₈ are independently a hydrogen atom, or a substituted or unsubstituted alkyl group having 1 to 50 carbon atoms;
R₃₁ is a substituted or unsubstituted alkyl group having 1 to 50 carbon atoms;
R₄₁ to R₄₉ are independently a hydrogen atom, or a substituted or unsubstituted alkyl group having 1 to 50 carbon atoms;
R₅₁ is a substituted or unsubstituted alkyl group having 1 to 50 carbon atoms; and
R₆₁ to R₆₉ are independently a hydrogen atom, or a substituted or unsubstituted alkyl group having 1 to 50 carbon atoms.

The number of carbon atoms of the alkyl group of R₂₁ to R₂₈, R₃₁, R₄₁ to R₄₉, R₅₁ and R₆₁ to R₆₉ is preferably 1 to 18, and more preferably 1 to 8.

One aspect of the compound of the invention is preferably a material for an organic electroluminescent element.

Specific examples of the halogen atom of R₁ to R₁₀, R₁₂ to R₁₇, and R₁₀₁ to R₁₁₀ described later include a fluorine atom, a chlorine atom, a bromine atom and an iodine atom.

Specific examples of the aryl group having 6 to 50 (preferably 6 to 18) ring carbon atoms of R₁ to R₁₇, Ar₁ to Ar₄, R₁₀₁ to R₁₁₀ described later, and Ar₁₀₁ described later include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl-4-yl group, a p-terphenyl-3-yl group, a p-terphenyl-2-yl group, a m-terphenyl-4-yl group, a m-terphenyl-3-yl group, a m-terphenyl-2-yl group, an o-tolyl group, a m-tolyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, a 4"-t-butyl-p-terphenyl-4-yl group, a chrysenyl group, a fluoranthenyl group and a fluorenyl group.

Among the above-described groups, the aryl group is preferably a phenyl group, a naphthyl group, a biphenyl group, a terphenyl group, a pyrenyl group, a phenanthryl group and a fluorenyl group, and more preferably a phenyl group, a naphthyl group, a biphenyl group, a terphenyl group, a pyrenyl group and a fluorenyl group.

Specific examples of the heteroaryl group having 5 to 50 (preferably 5 to 18) ring atoms of R₁ to R₁₇, Ar₁ to Ar₄, R₁₀₁ to R₁₁₀ described later, and Ar₁₀₁ described later include a pyrrolyl group, a pyrazinyl group, a pyridinyl group, an indolyl group, an isoindolyl group, a furyl group, a benzofuranyl group, an isobenzofuranyl group, a dibenzofuranyl group, a dibenzothiophenyl group, a quinolyl group, an isoquinolyl group, a quinoxalinyl group, a carbazolyl group, a phenanthridinyl group, an acridinyl group, a phenanthrolinyl group, and a thienyl group, and a monovalent group formed of a pyridine ring, a pyrazine ring, a pyrimidine ring, a pyridazine ring, a triazine ring, an indole ring, a quinoline ring, an acridine ring, a pyrrolidine ring, a dioxane ring, a piperidine ring, a morpholine ring, a piperazine ring, a carbazole ring, a furan ring, a thiophene ring, an oxazole ring, an oxadiazole ring, a benzoxazole ring, a thiazole ring, a thiadiazole ring, a benzothiazole ring, a triazole ring, an imidazole ring, a benzimidazole ring, a pyran ring, a dibenzofuran ring, a benzo[a]dibenzofuran ring, a benzo[b]dibenzofuran ring and a benzo[c]dibenzofuran ring.

Specific examples of the alkyl group having 1 to 50 (preferably 1 to 18) carbon atoms of R₁ to R₁₇, R₂₁ to R₂₈, R₃₁, R₄₁ to R₄₉, R₅₁, R₆₁ to R₆₉, and R₁₀₁ to R₁₁₀ described later include a methyl group, an ethyl group, a propyl group, an isopropyl group, a n-butyl group, a s-butyl group, an isobutyl group, a t-butyl group, a n-pentyl group, a n-hexyl group, a n-heptyl group, a n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group and a 1,2,3-trinitropropyl group.

Specific examples of the alkenyl group having 2 to 50 (preferably 2 to 18) carbon atoms of R₁ to R₁₀, R₁₂ to R₁₇, and R₁₀₁ to R₁₁₀ described later include a vinyl group, an allyl group, a 1-butenyl group, a 2-butenyl group, a 3-butenyl group, a 1,3-butanedienyl group, a 1-methylvinyl group, a 1-methylallyl group, a 1,1-dimethylallyl group, a 2-methylallyl group and a 1, 2-dimethylallyl group.

Specific examples of the alkynyl group having 2 to 50 carbon atoms of R₁ to R₁₀, R₁₂ to R₁₇, and R₁₀₁ to R₁₁₀ described later include an ethynyl group.

Specific examples of the cycloalkyl group having 3 to 50 (preferably 3 to 18) ring carbon atoms of R₁ to R₁₀, R₁₂ to R₁₇, and R₁₀₁ to R₁₁₀ described later include a cyclopropyl group, a cyclobutyl group, a cyclopentyl group, a cyclohexyl group, a 4-methylcyclohexyl group, a 1-adamantyl group, a 2-adamantyl group, a 1-norbornyl group and a 2-norbornyl group.

Specific examples of the alkoxy group having 1 to 50 (preferably 1 to 18) carbon atoms of R₁ to R₁₀, R₁₂ to R₁₇, and R₁₀₁ to R₁₁₀ described later include a group in which an oxygen atom is bonded with the above-described alkyl group having 1 to 50 carbon atoms.

Specific examples of the aralkyl group having 7 to 50 (preferably 7 to 18) carbon atoms of R₁ to R₁₀, R₁₂ to R₁₇, and R₁₀₁ to R₁₁₀ described later include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, a 2-β-naphthylisopropyl group, a 1-pyrrolylmethyl group, a 2-(1-pyrrolyl)ethyl group, a p-methylbenzyl group, a m-methylbenzyl group, an o-methylbenzyl group, a p-chlorobenzyl group, a m-chlorobenzyl group, an o-chlorobenzyl group, a p-bromobenzyl group, a m-bromobenzyl group, an o-bromobenzyl group, a p-iodobenzyl group, a m-iodobenzyl group, an o-iodobenzyl group, a p-hydroxybenzyl group, a m-hydroxybenzyl group, an o-hydroxybenzyl group, a p-aminobenzyl group, a m-aminobenzyl group, an o-aminobenzyl group, a p-nitrobenzyl group, a m-nitrobenzyl group, an o-nitrobenzyl group, a p-cyanobenzyl group, a m-cyanobenzyl group, an o-cyanobenzyl group, a 1-hydroxy-2-phenylisopropyl group and a 1-chloro-2-phenylisopropyl group.

Specific examples of the aryloxy group having 6 to 50 (preferably 6 to 18) ring carbon atoms of R₁ to R₁₀, R₁₂ to R₁₇, and R₁₀₁ to R₁₁₀ described later include a group in which an oxygen atom is bonded with the above-described aryl group having 6 to 50 ring carbon atoms.

Specific examples of the substituted or unsubstituted silyl group of R₁ to R₁₀, R₁₂ to R₁₇, and R₁₀₁ to R₁₁₀ described later include an unsubstituted silyl group (-SiH₃), a trialkylsilyl group and a triarylsilyl group. The alkyl group and the aryl group are as arbitrary substituents described later.

Specific examples of the alkylthio group having 1 to 50 (preferably 1 to 18) carbon atoms of R₁ to R₁₀, R₁₂ to R₁₇, and R₁₀₁ to R₁₁₀ described later include a group in which a sulfur atom is bonded with the above-described alkyl group having 1 to 50 carbon atoms.

Specific examples of the arylthio group having 6 to 50 (preferably 6 to 18) ring carbon atoms of R₁ to R₁₀, R₁₂ to R₁₇, and R₁₀₁ to R₁₁₀ described later include a group in which a sulfur atom is bonded with the above-described aryl group having 6 to 50 ring carbon atoms.

Specific examples of the arylene group having 6 to 30 (preferably 6 to 18) ring carbon atoms of L₁ to L₃, and L₁₀₁ described later include a phenylene group (for example, a m-phenylene group), a naphthylene group, a biphenylene group, an anthranylene group and a pyrenylene group.

Specific examples of the heteroarylene group having 5 to 30 (preferably 5 to 18) ring atoms of L₁ to L₃, and L₁₀₁ described later include a non-fused heteroarylene group and a fused heteroarylene group, and more specifically, include a pyrrolyl group, a pyrazinyl group, a pyridinyl group, an indolyl group, an isoindolyl group, a furyl group, a benzofuranyl group, an isobenzofuranyl group, a dibenzofuranyl group, a dibenzothiophenyl group, a quinolyl group, an isoquinolyl group, a quinoxalinyl group, a carbazolyl group, a phenanthridinyl group, an acridinyl group, a phenanthrolinyl group, and a thienyl group, and a divalent group formed of a pyridine ring, a pyrazine ring, a pyrimidine ring, a pyridazine ring, a triazine ring, an indole ring, a quinoline ring, an acridine ring, a pyrrolidine ring, a dioxane ring, a piperidine ring, a morpholine ring, a piperazine ring, a carbazole ring, a furan ring, a thiophene ring, an oxazole ring, an oxadiazole ring, a benzoxazole ring, a thiazole ring, a thiadiazole ring, a benzothiazole ring, a triazole ring, an imidazole ring, a benzimidazole ring, a pyran ring, a dibenzofuran ring, a benzo[a]dibenzofuran ring, a benzo[b]dibenzofuran ring and a benzo[c]dibenzofuran ring.

In the present specification, specific examples of the substituent in the case of "substituted or unsubstituted" (hereinafter, also referred to as an arbitrary substituent) include, for R₁ to R₁₀ and the like, a halogen atom, a cyano group, an aryl group having 6 to 50 ring carbon atoms, a heteroaryl group having 5 to 50 ring atoms, an alkyl group having 1 to 50 carbon atoms, an alkenyl group having 2 to 50 carbon atoms, an alkynyl group having 2 to 50 carbon atoms, a cycloalkyl group having 3 to 50 ring carbon atoms, an alkoxy group having 1 to 50 carbon atoms, an aralkyl group having 7 to 50 carbon atoms, an aryloxy group having 6 to 50 ring carbon atoms, a silyl group, an alkylthio group having 1 to 50 carbon atoms and an arylthio group having 6 to 50 ring carbon atoms as described above.

Specific examples of each of the substituents are similar to specific examples described above as the substituents of R₁ to R₁₀ and the like, respectively.

In the present specification, a saturated or unsaturated ring (preferably a saturated or unsaturated and substituted or unsubstituted 5-membered ring or 6-membered ring, and more preferably a benzene ring) may be formed between arbitrary adjacent substituents.

In the present specification, the arbitrary substituent may further have a substituent. Specific examples of the substituent that the arbitrary substituent may further have include a substituent similar to the above-described arbitrary substituent.

Specific examples of one aspect of the compound of the invention include compounds shown below.

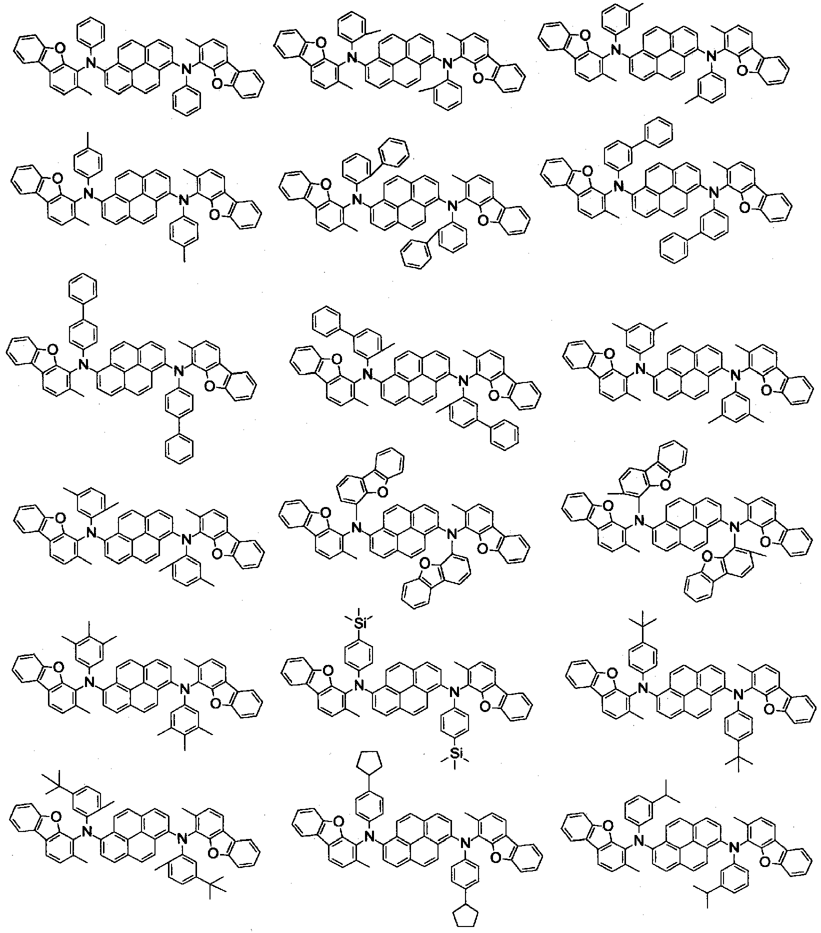

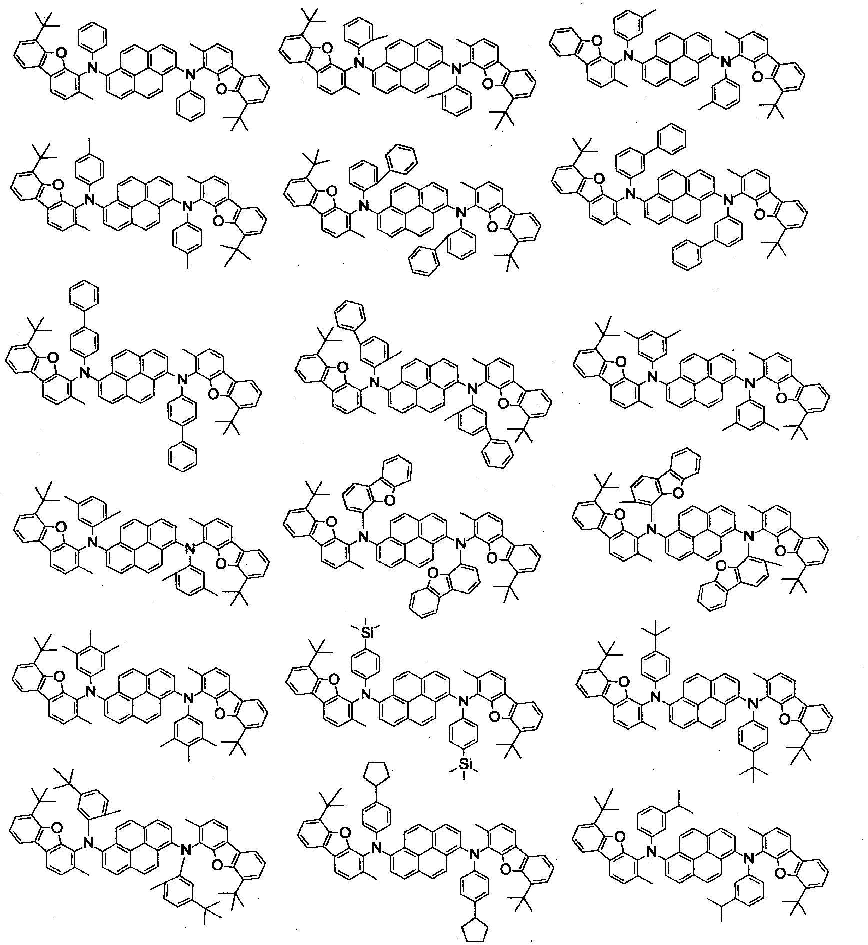

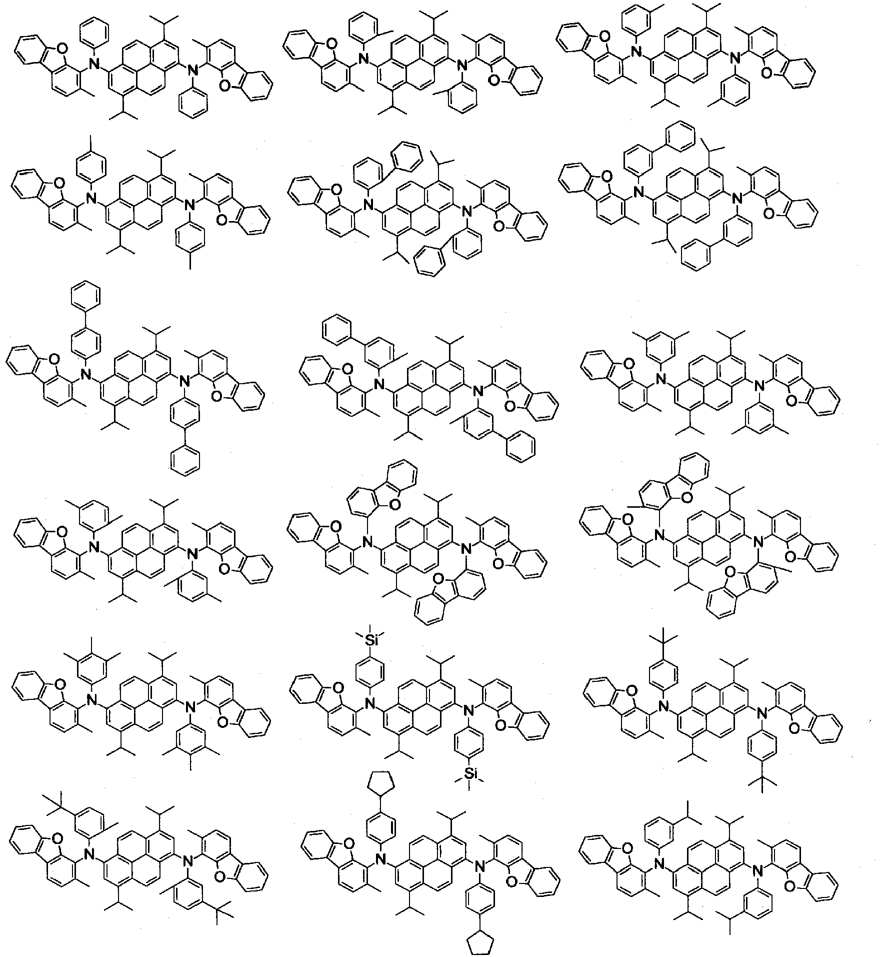

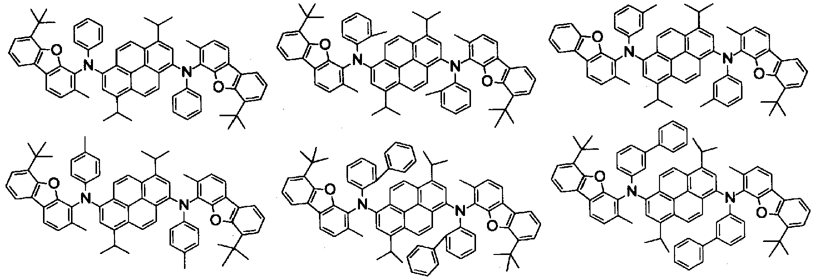

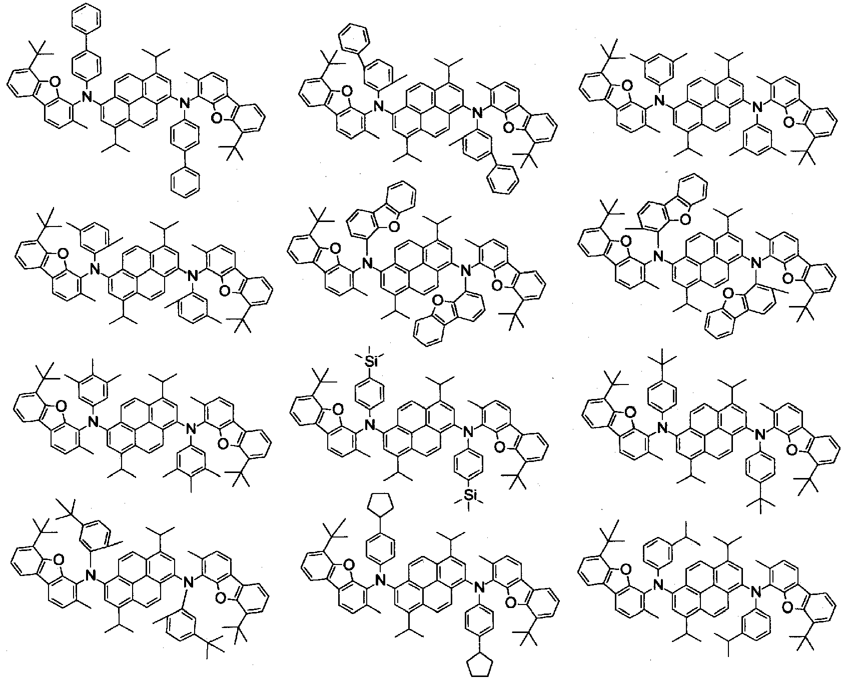

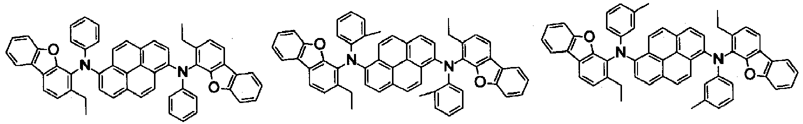

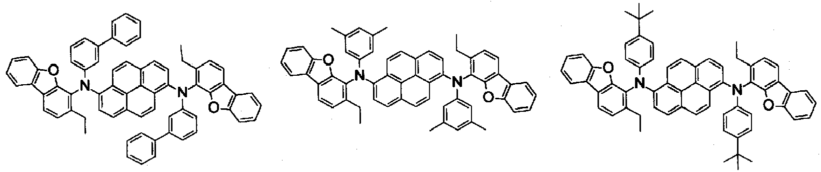

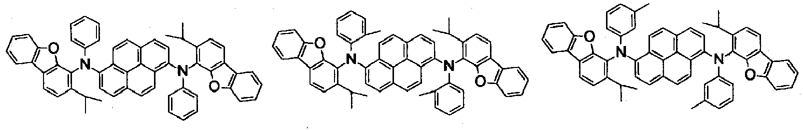

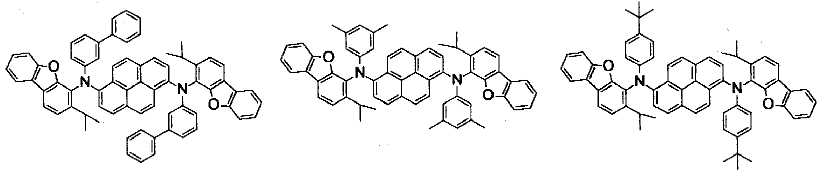

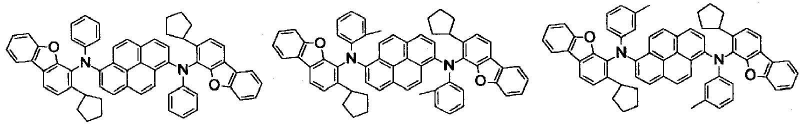

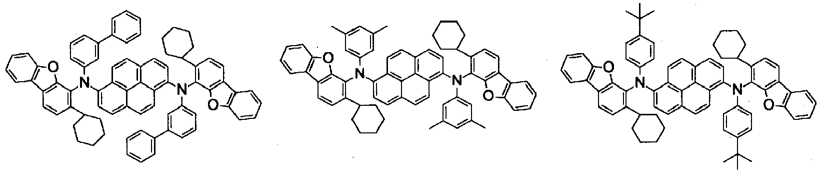

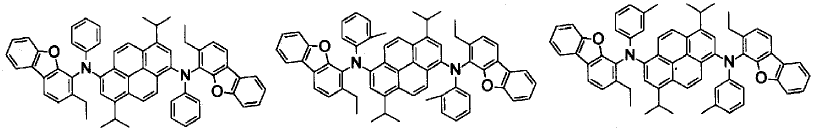

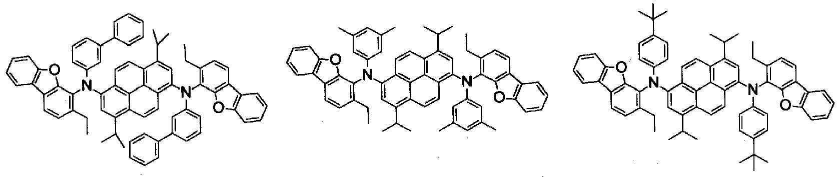

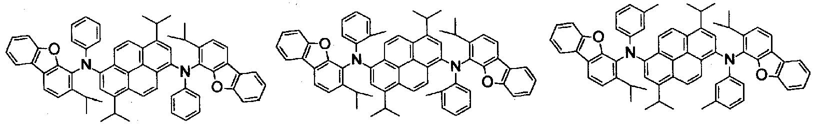

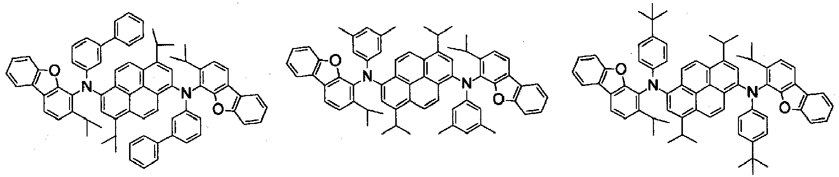

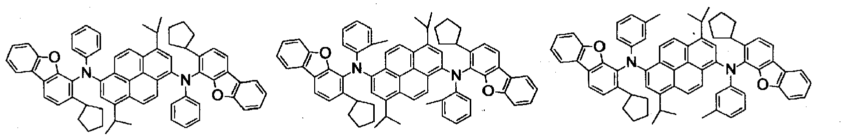

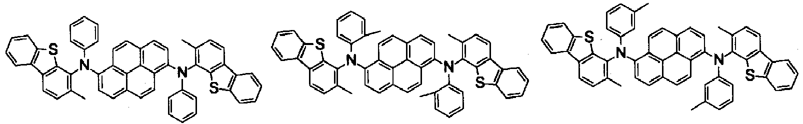

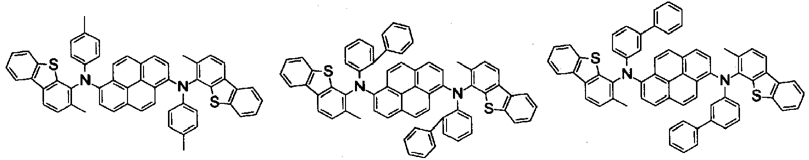

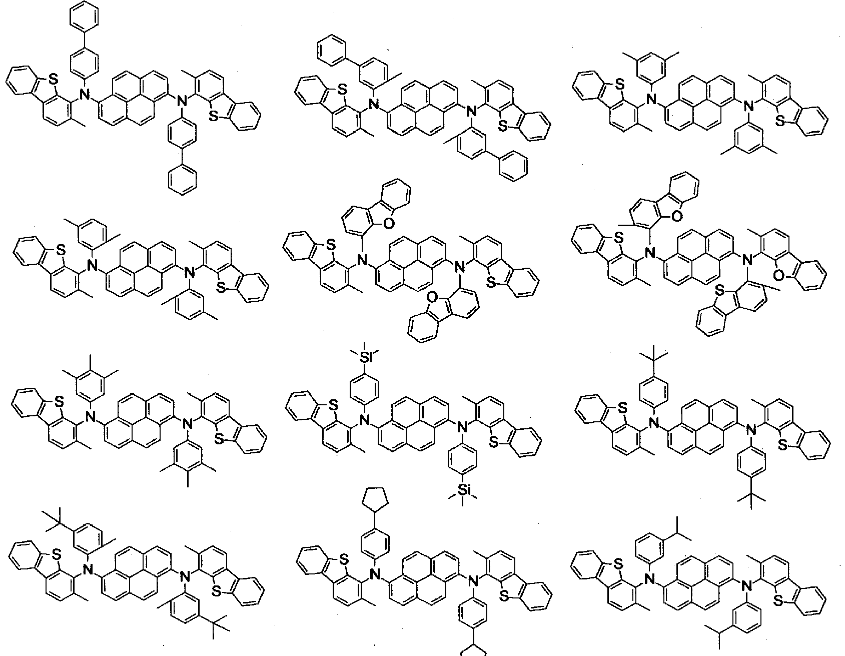

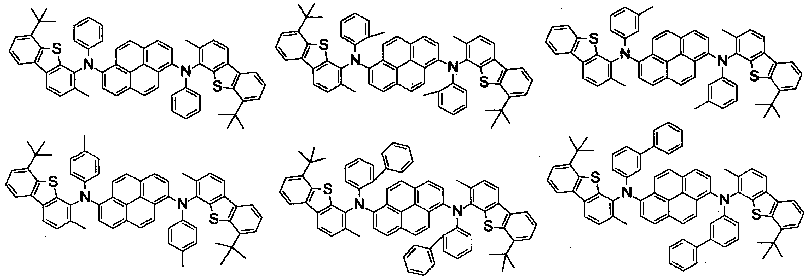

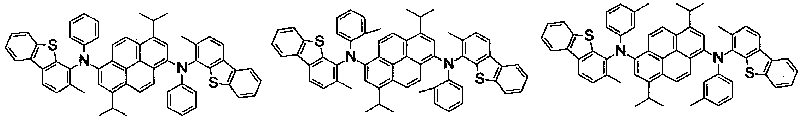

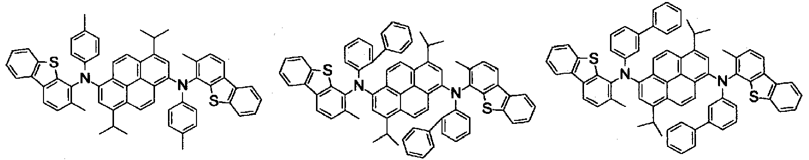

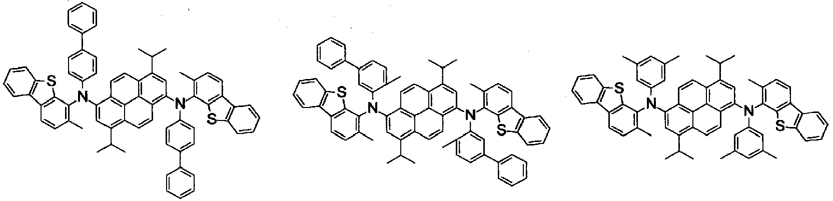

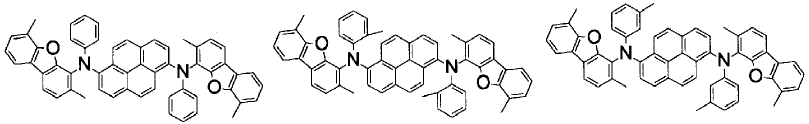

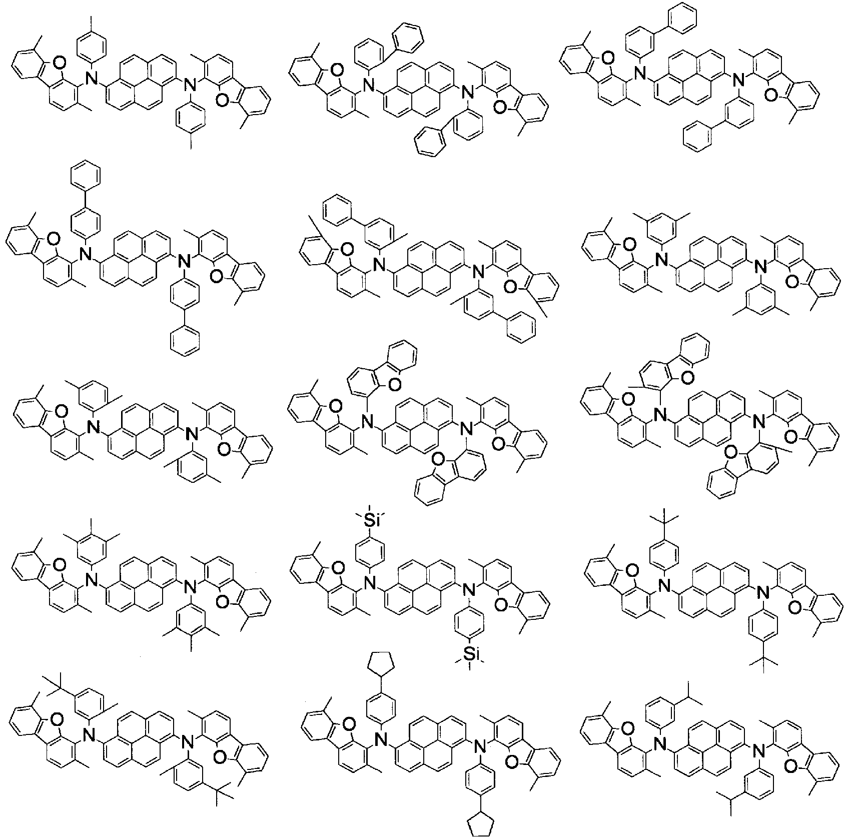

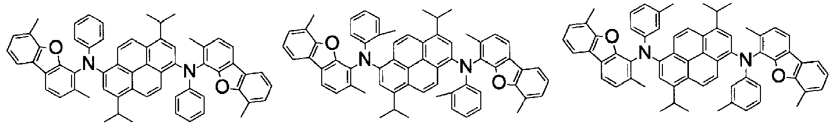

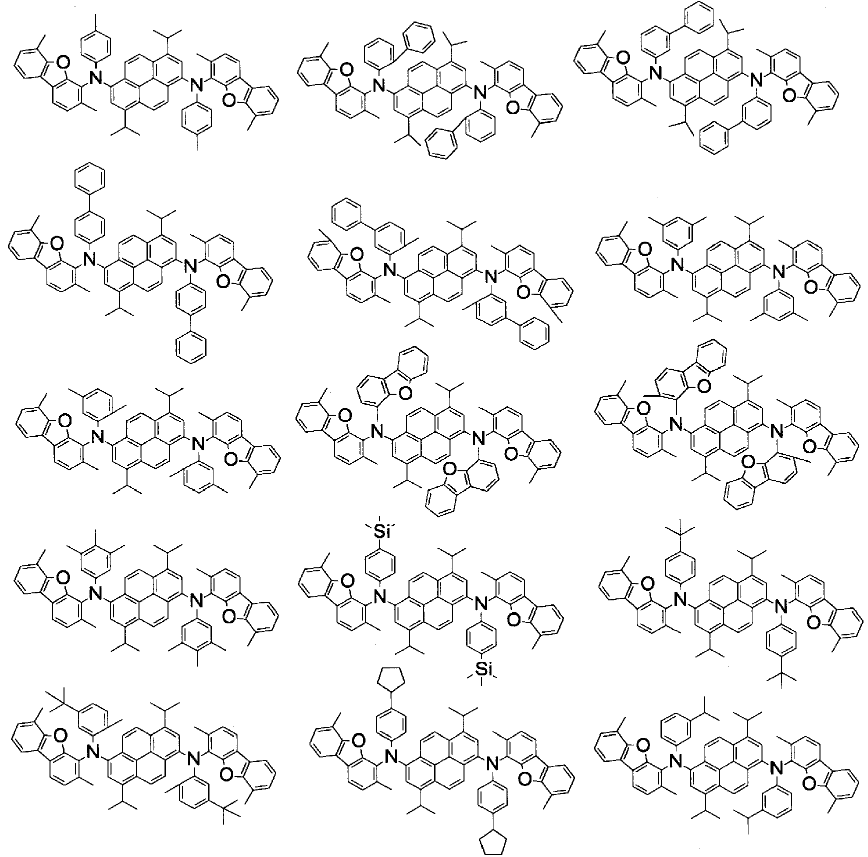

One aspect of the organic EL element of the invention has a cathode, an anode and an emitting layer arranged between the cathode and the anode, wherein the emitting layer includes the above-described compound.

Thus, luminous efficiency can be improved.

The emitting layer preferably further includes a compound represented by the following formula (11): 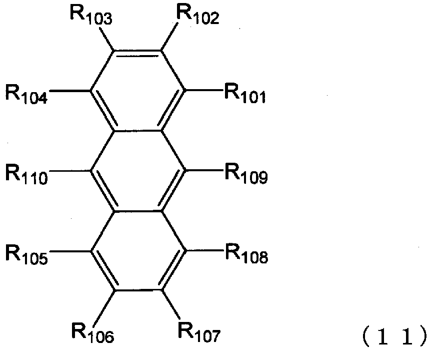
wherein, in the formula (11), R₁₀₁ to R₁₁₀ are independently a hydrogen atom, a halogen atom, a cyano group, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a substituted or unsubstituted alkylthio group having 1 to 50 carbon atoms, a substituted or unsubstituted arylthio group having 6 to 50 ring carbon atoms, or a group represented by -L₁₀₁-Ar₁₀₁,
and at least one of R₁₀₁ to R₁₁₀ is the group represented by -L₁₀₁-Ar₁₀₁, L₁₀₁ is a single bond, a substituted or unsubstituted arylene group having 6 to 30 ring carbon atoms, or a substituted or unsubstituted heteroarylene group having 5 to 30 ring atoms, and Ar₁₀₁ is a substituted or unsubstituted aryl group having 6 to 50 ring carbon atoms, or a substituted or unsubstituted heteroaryl group having 5 to 50 ring atoms, and when the number of L₁₀₁ is two or more, two or more of L₁₀₁ may be the same with or different from each other, and when the number of Ar₁₀₁ is two or more, two or more of Ar₁₀₁ may be the same with or different from each other.

At least one of R₁₀₉ and R₁₁₀ is preferably the group represented by -L₁₀₁-Ar₁₀₁ described above.

R₁₀₉ and R₁₁₀ are preferably independently the group represented by -L₁₀₁-Ar₁₀₁ described above.

Specific examples of the compound represented by the formula (11) include compounds shown below.

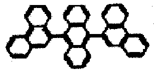

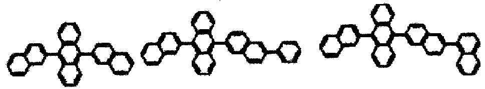

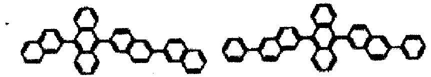

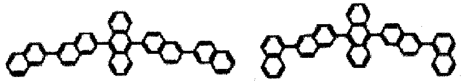

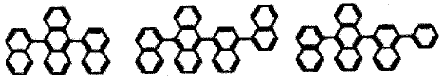

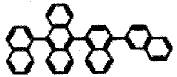

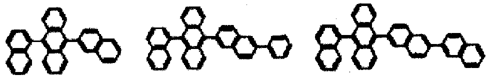

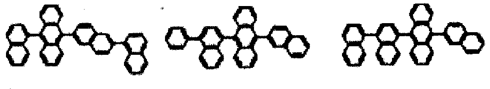

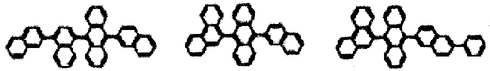

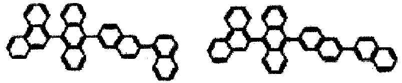

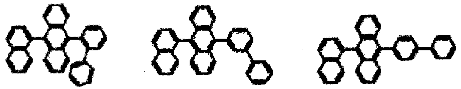

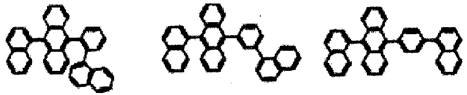

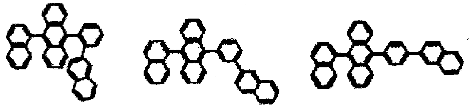

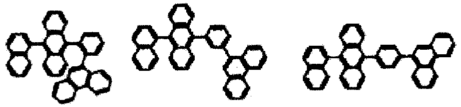

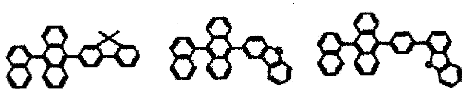

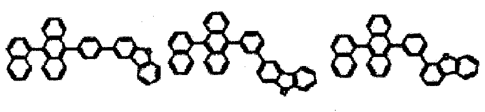

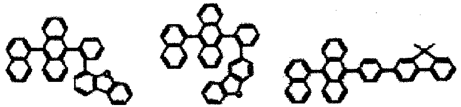

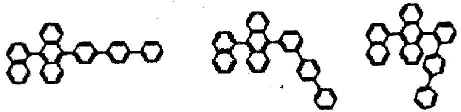

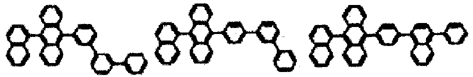

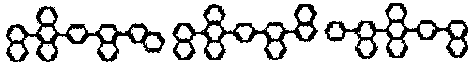

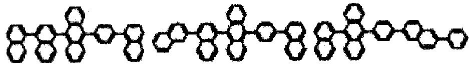

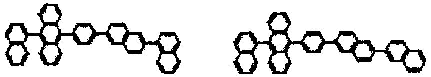

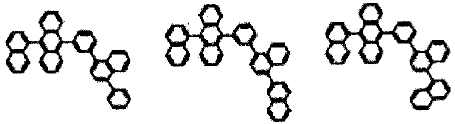

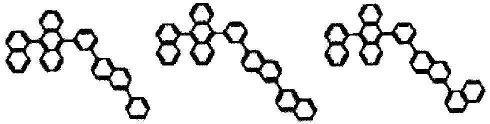

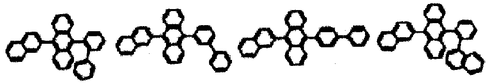

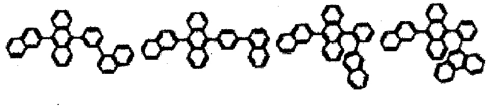

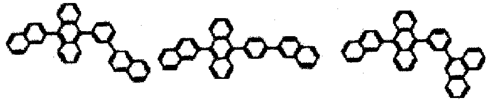

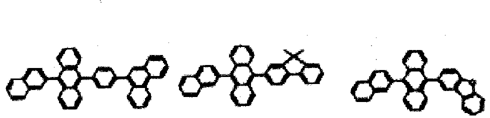

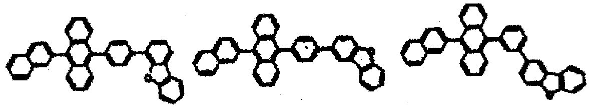

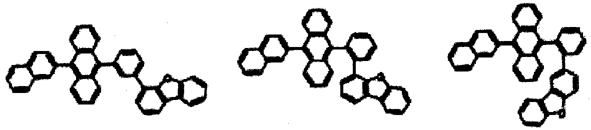

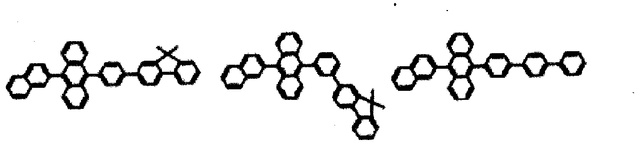

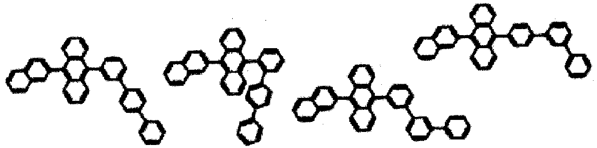

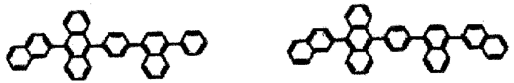

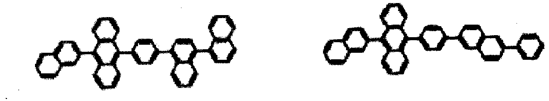

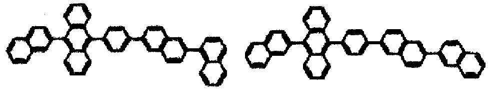

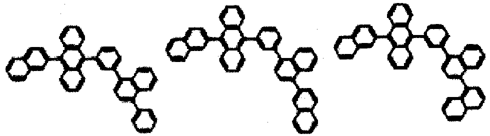

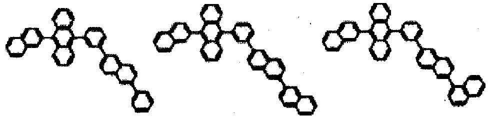

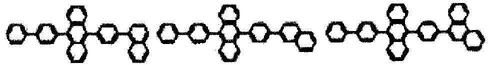

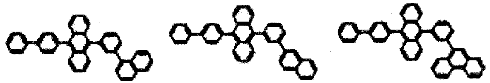

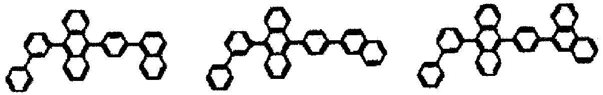

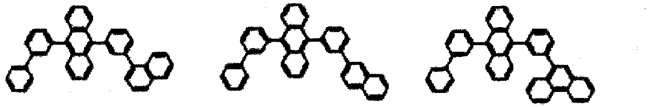

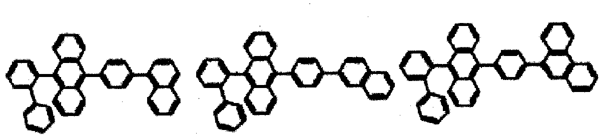

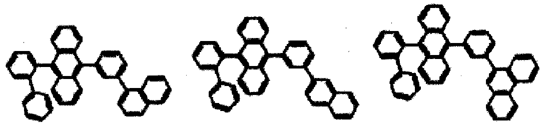

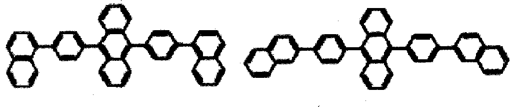

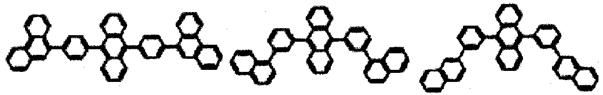

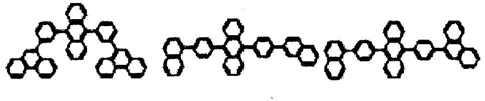

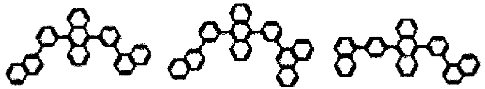

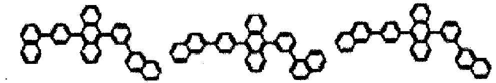

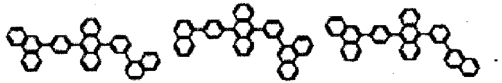

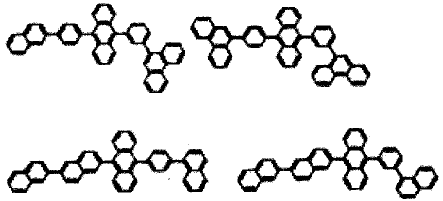

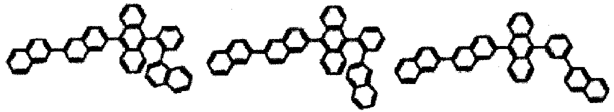

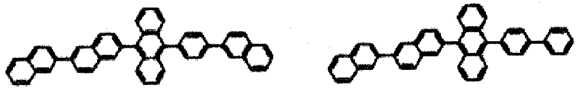

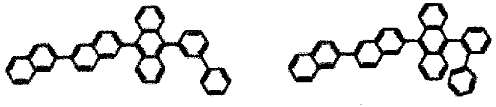

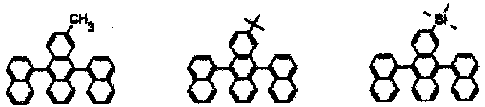

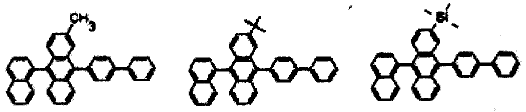

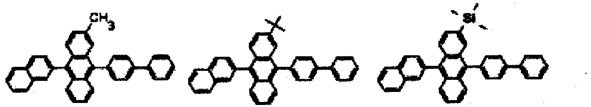

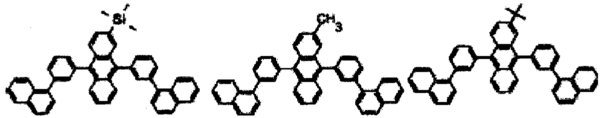

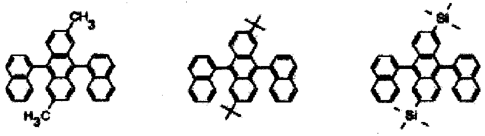

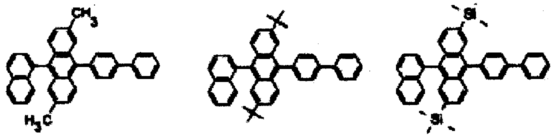

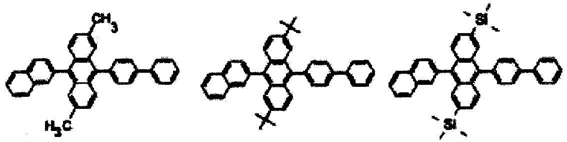

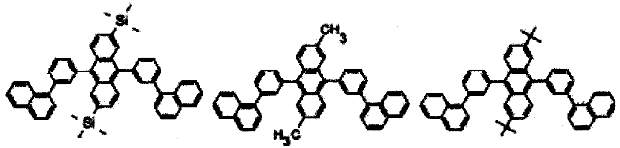

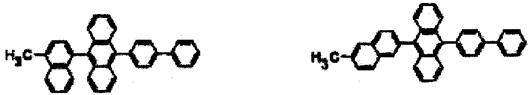

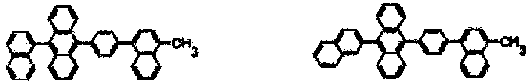

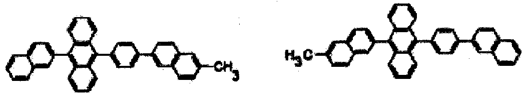

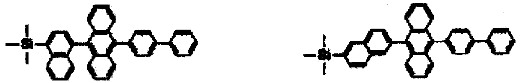

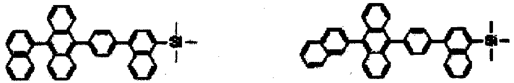

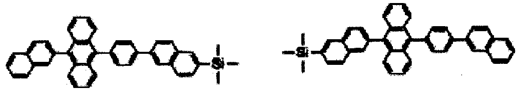

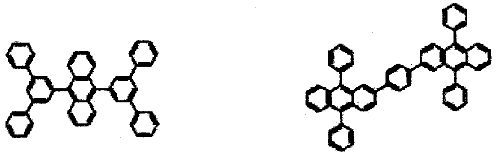

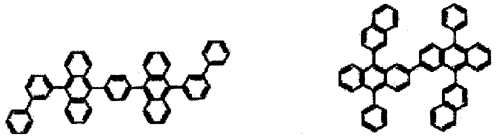

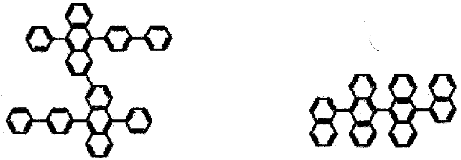

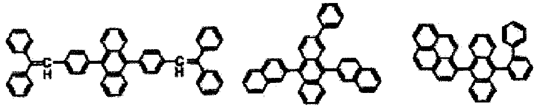

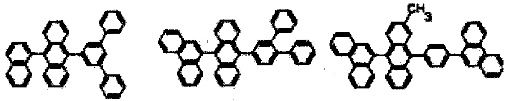

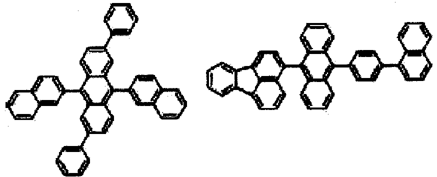

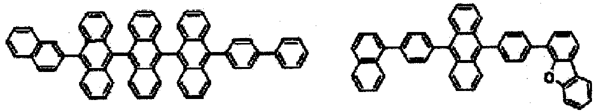

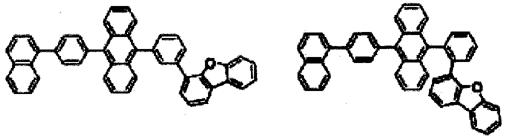

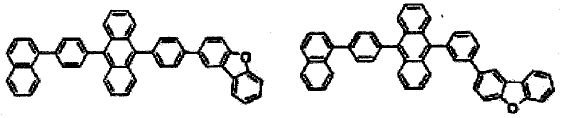

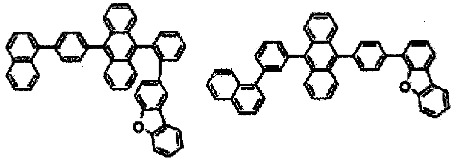

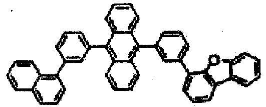

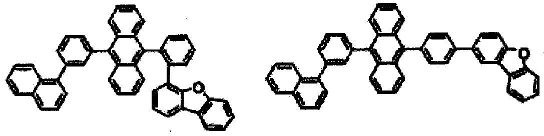

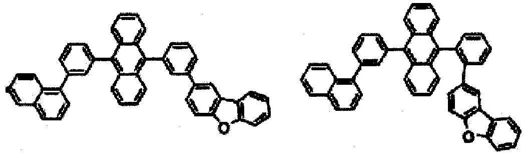

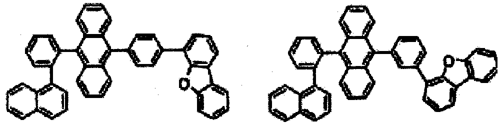

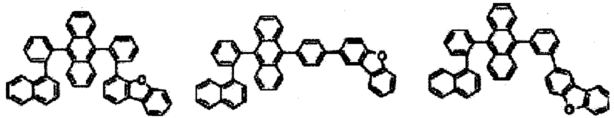

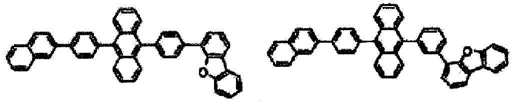

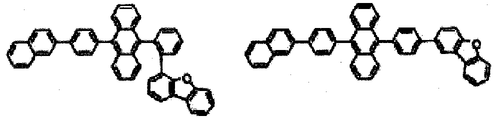

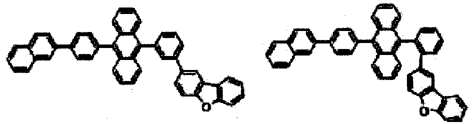

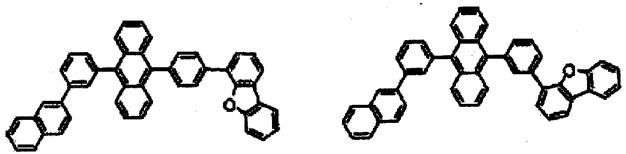

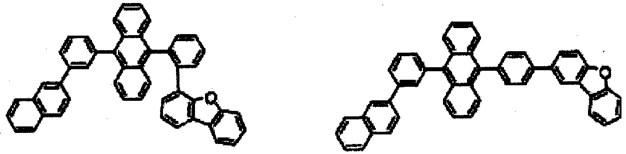

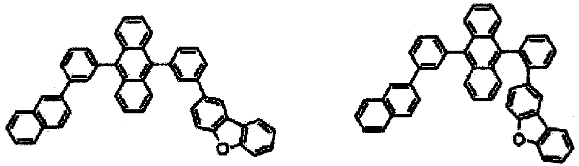

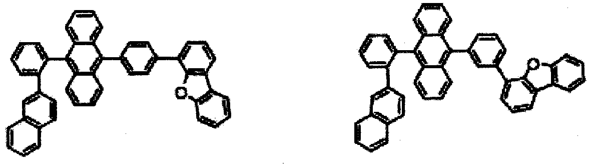

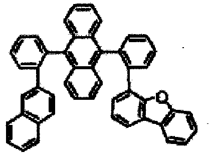

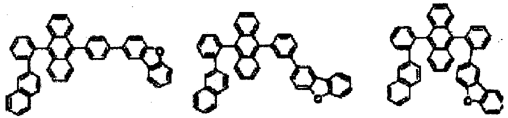

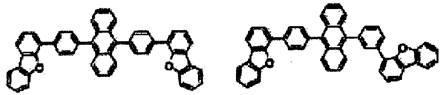

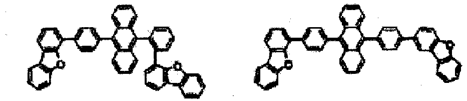

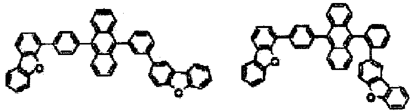

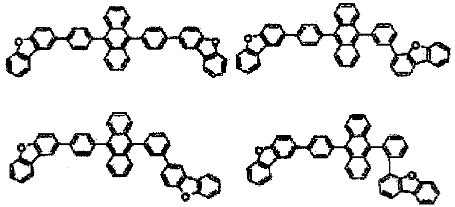

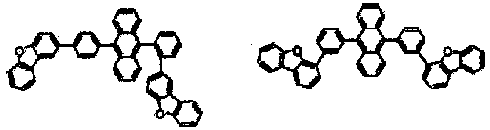

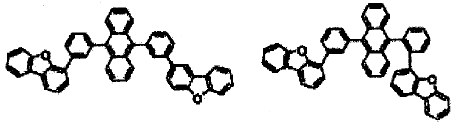

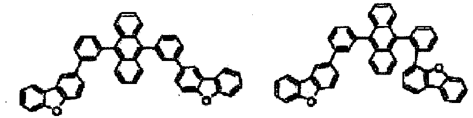

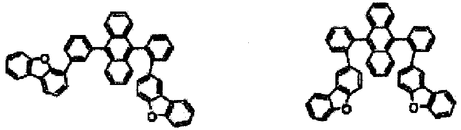

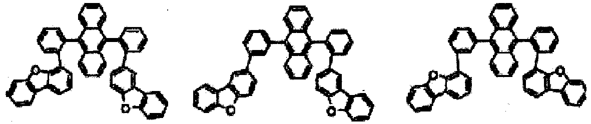

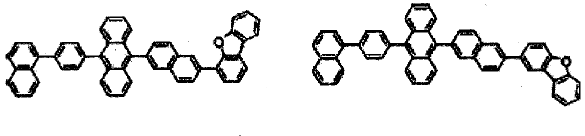

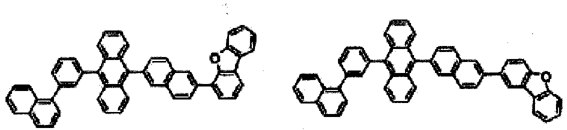

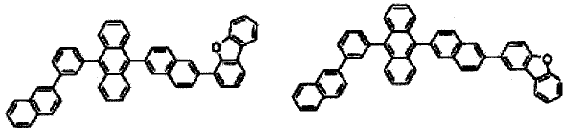

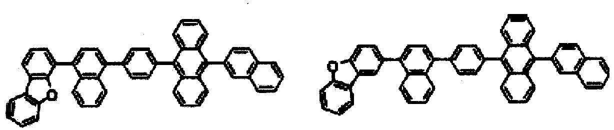

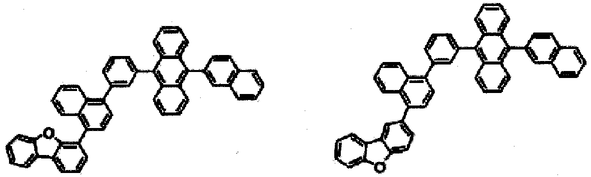

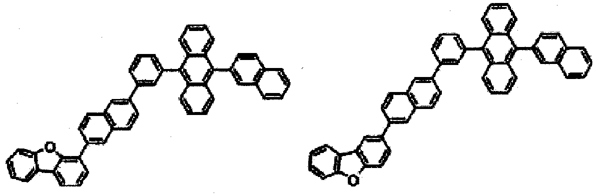

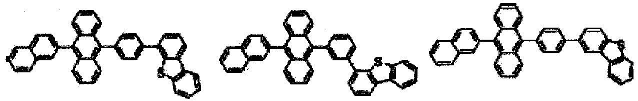

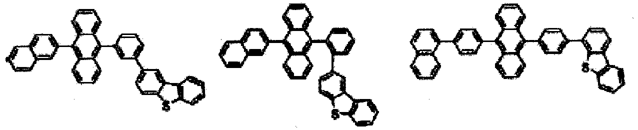

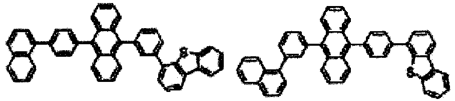

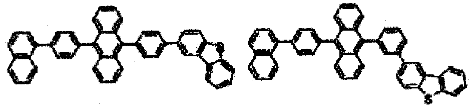

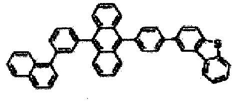

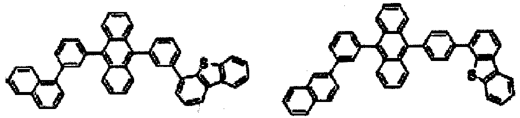

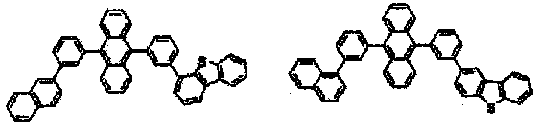

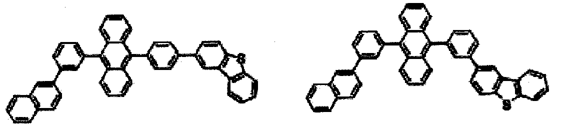

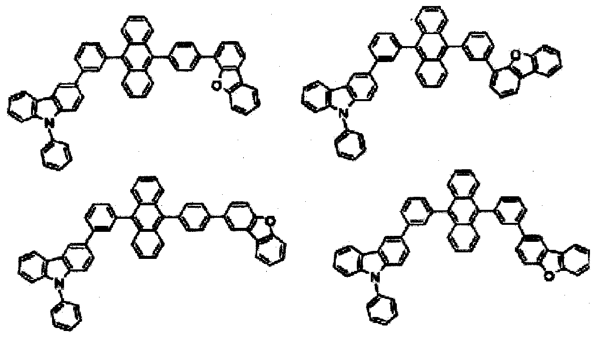

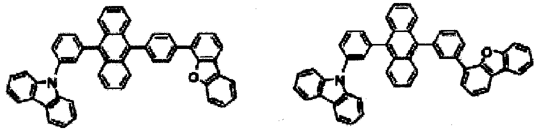

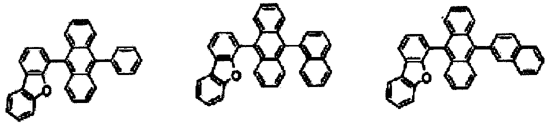

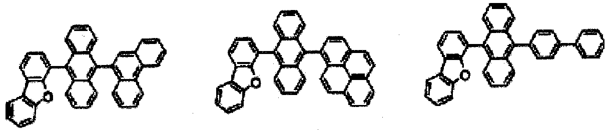

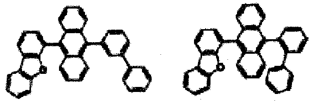

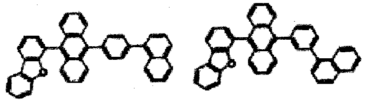

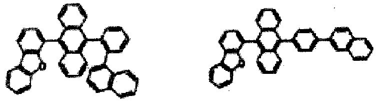

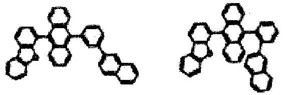

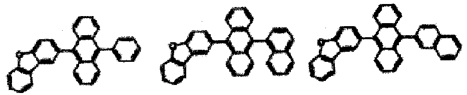

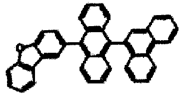

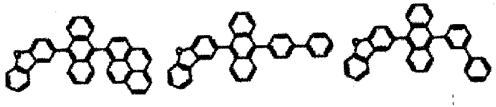

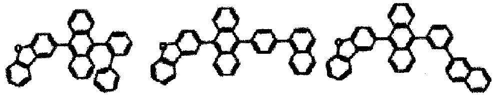

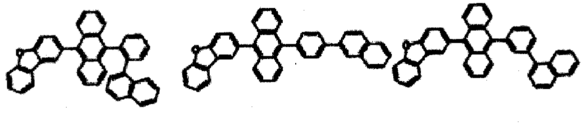

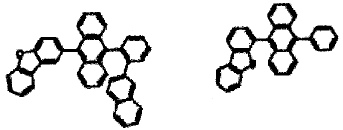

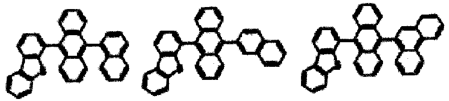

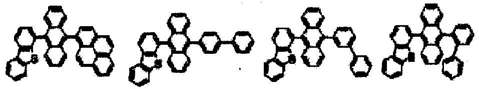

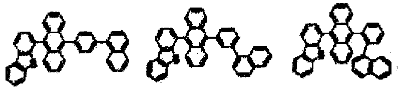

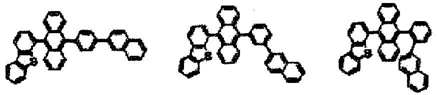

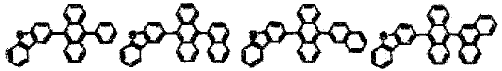

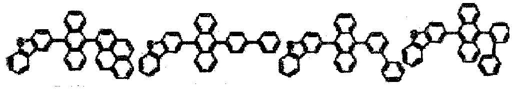

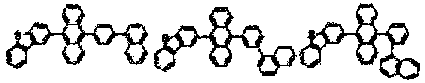

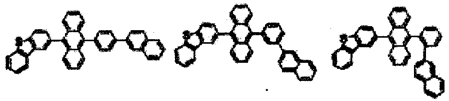

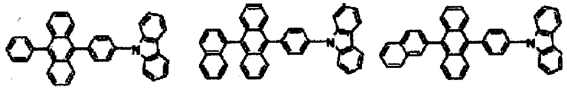

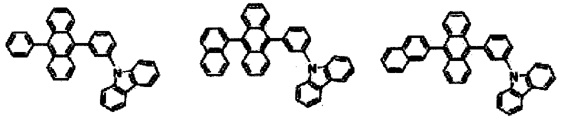

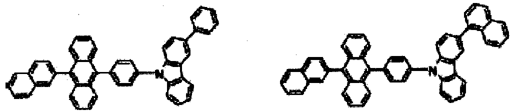

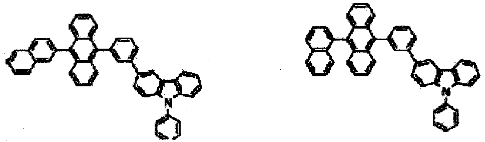

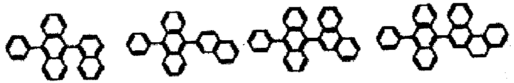

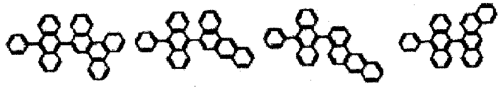

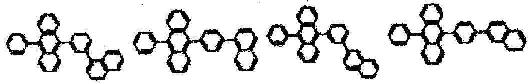

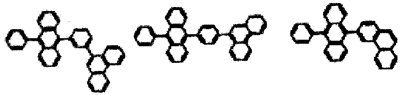

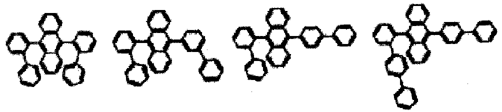

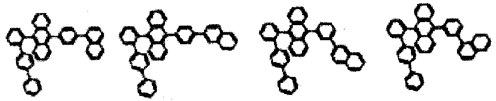

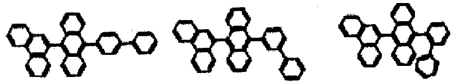

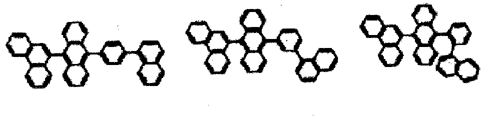

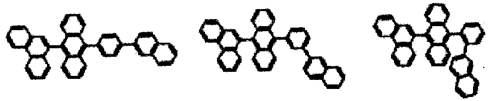

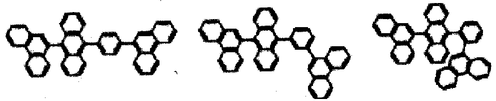

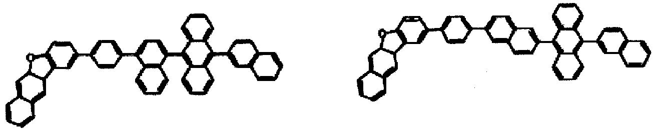

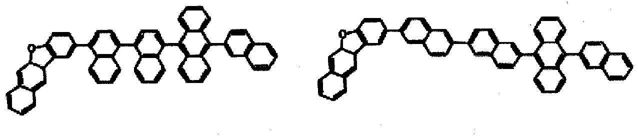

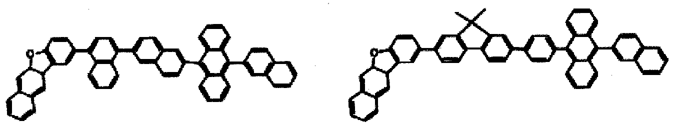

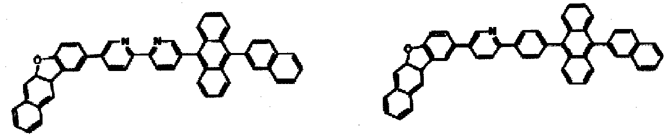

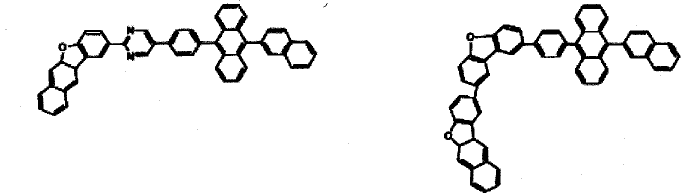

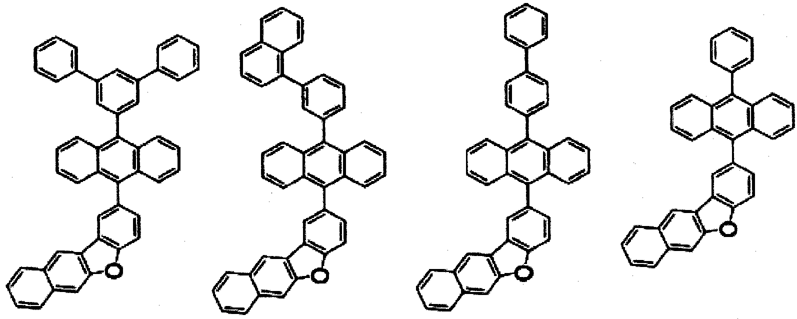

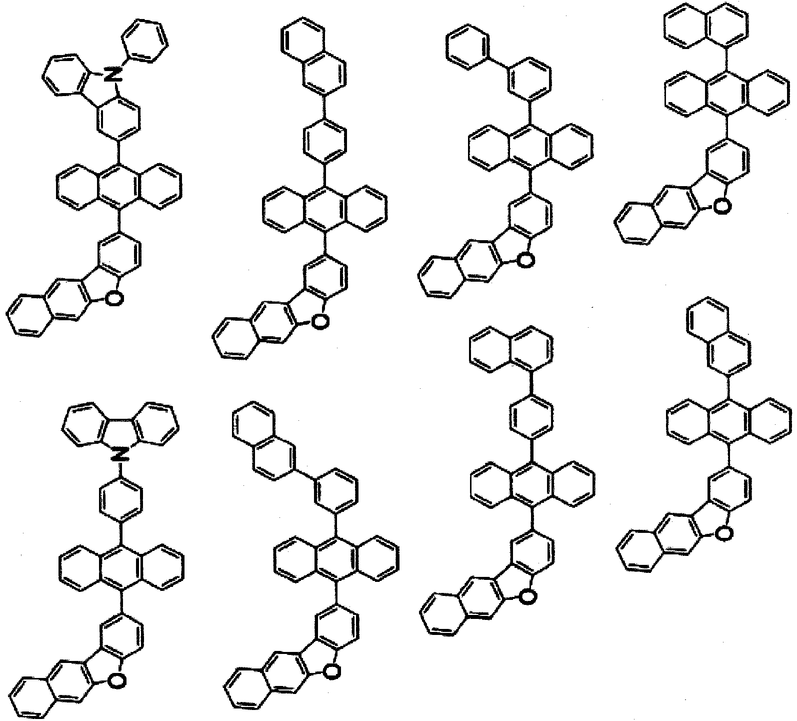

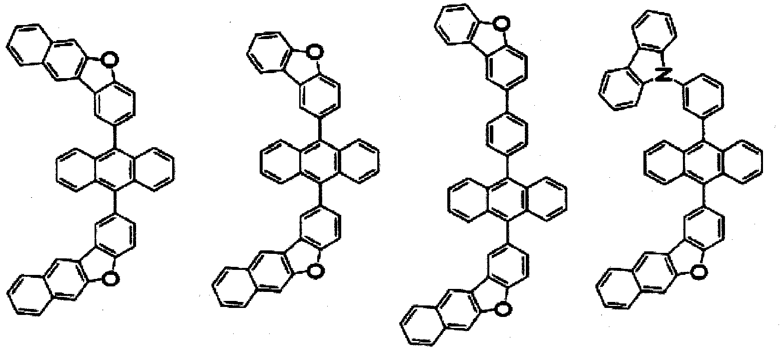

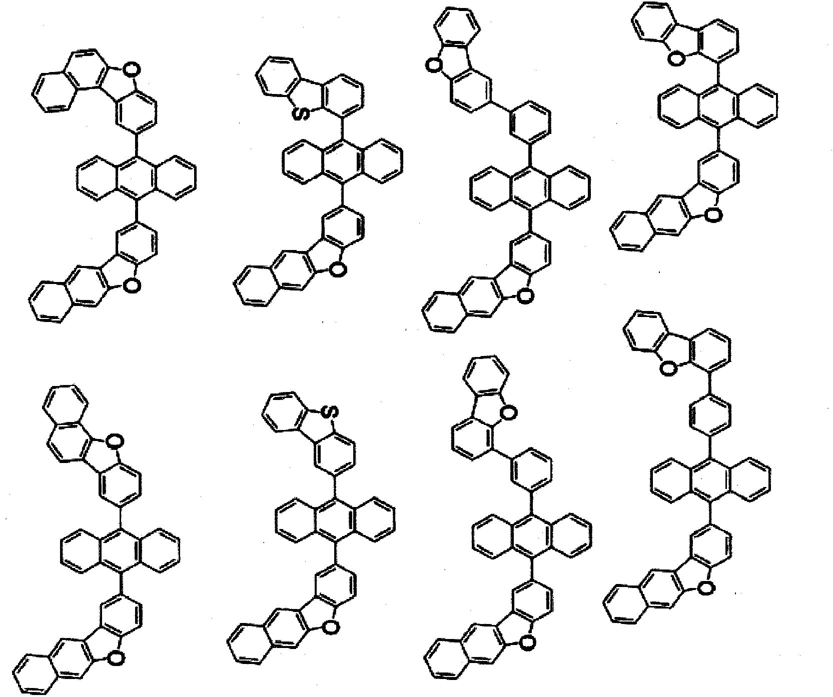

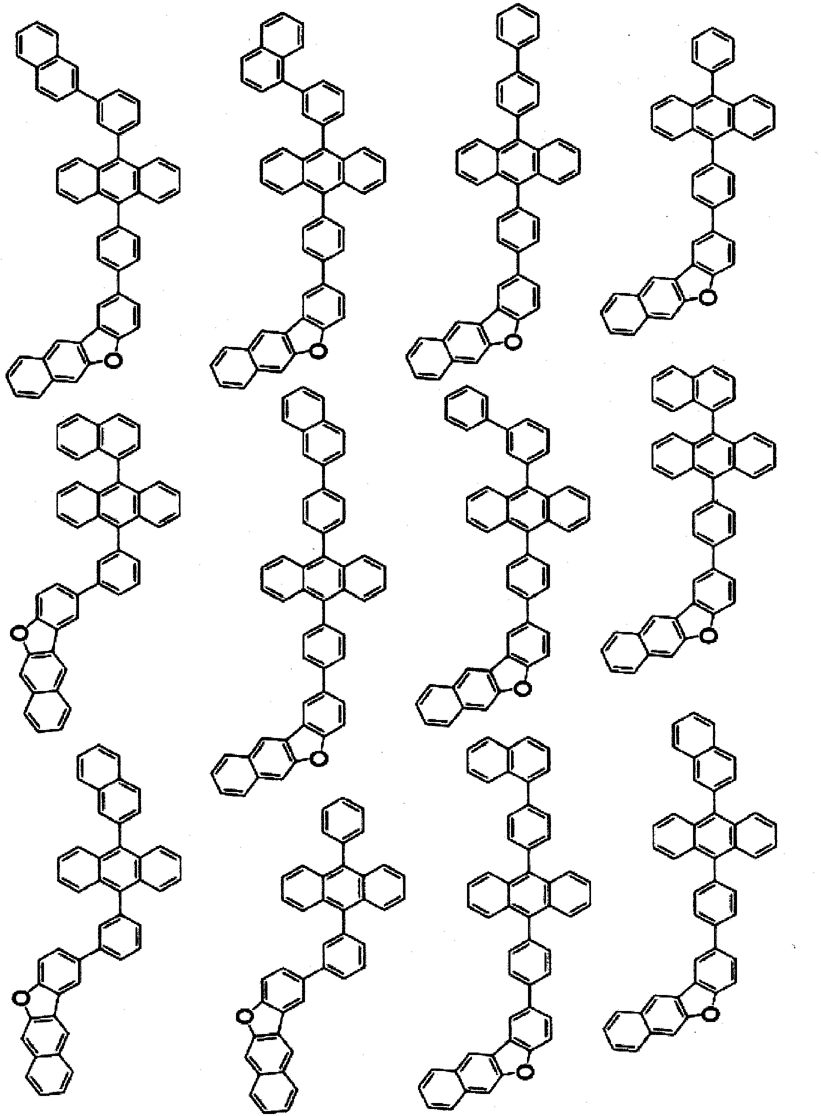

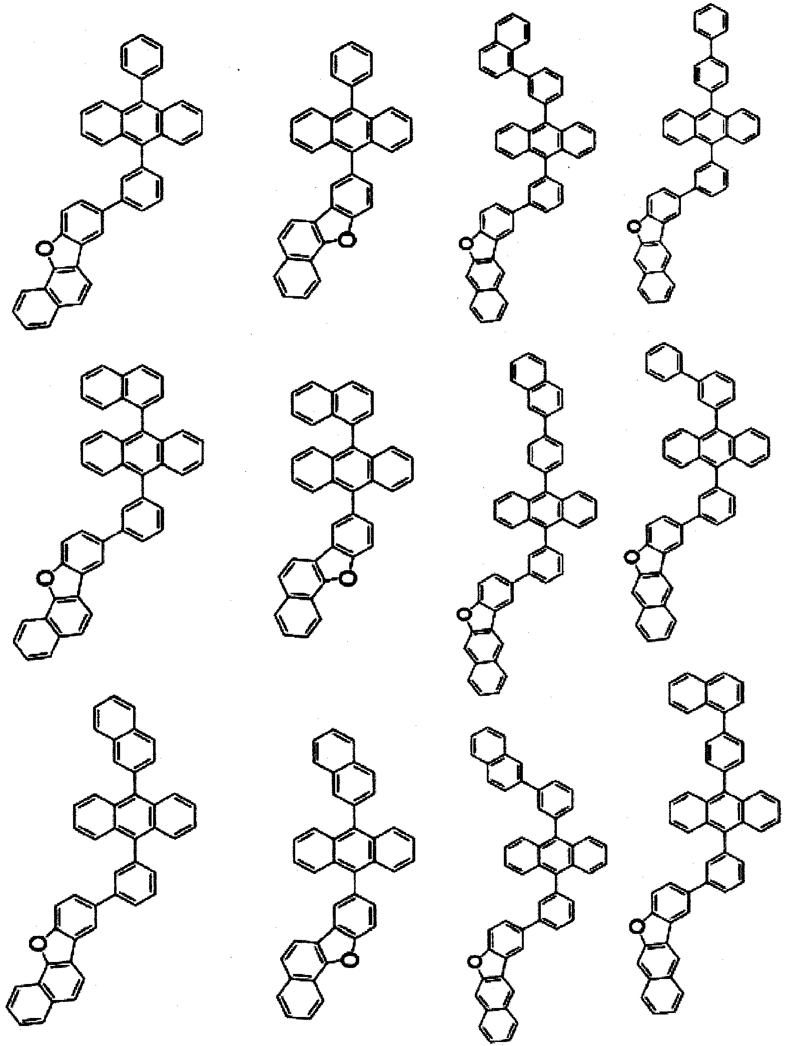

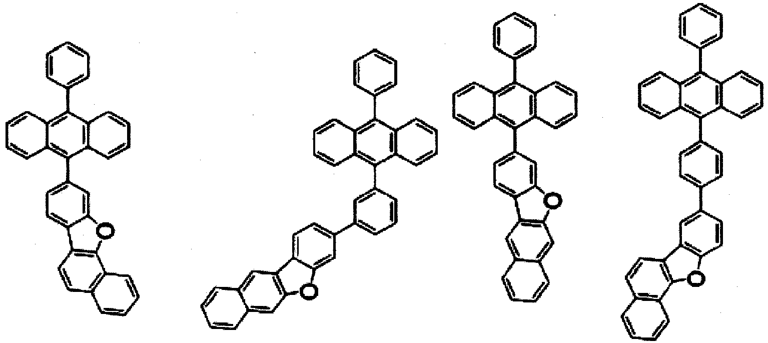

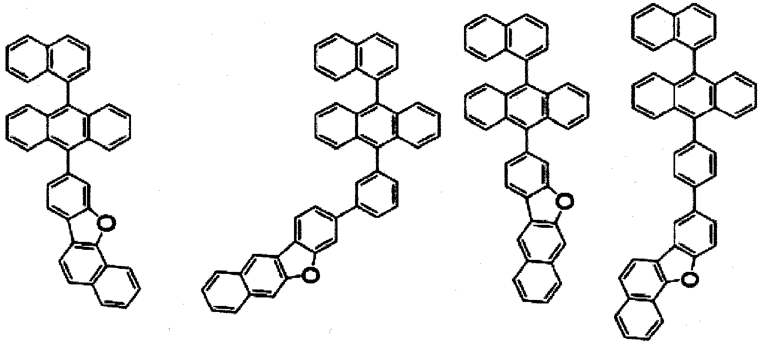

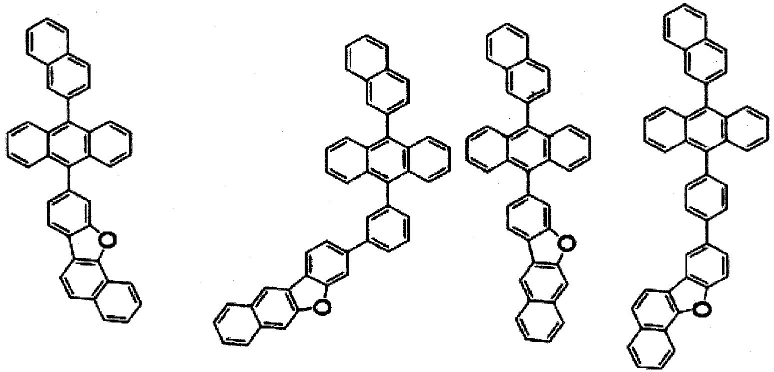

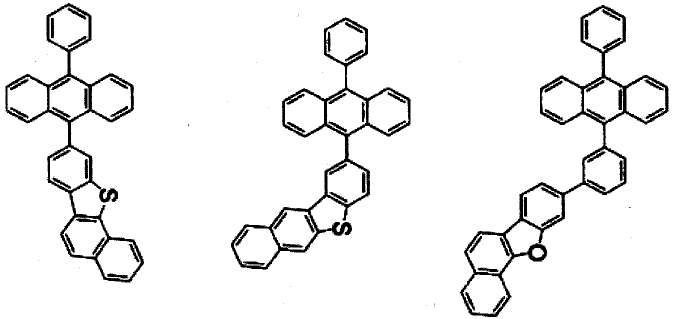

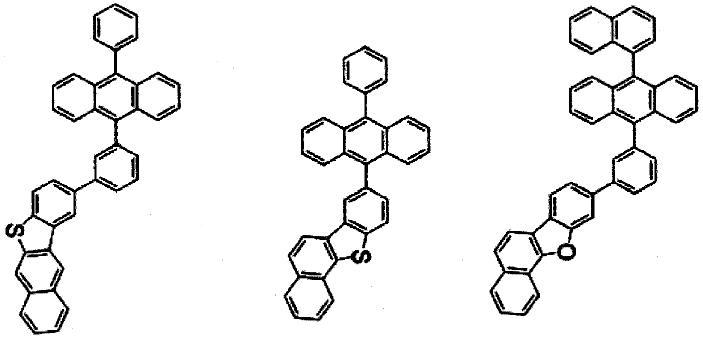

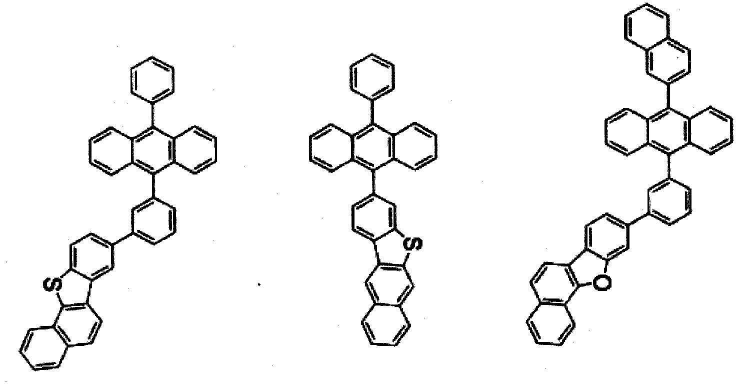

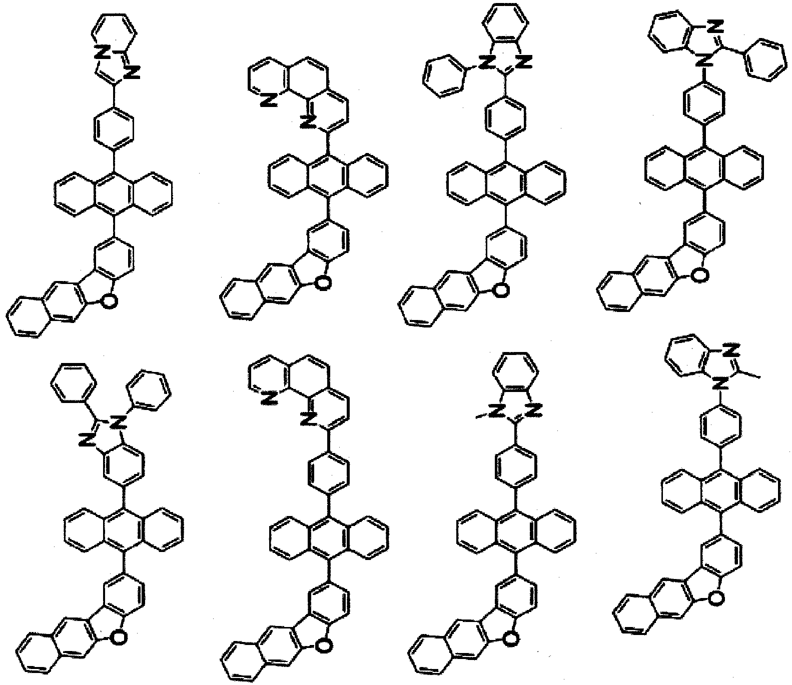

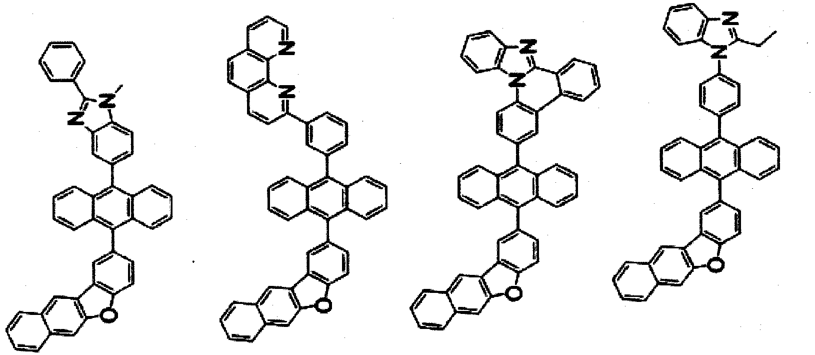

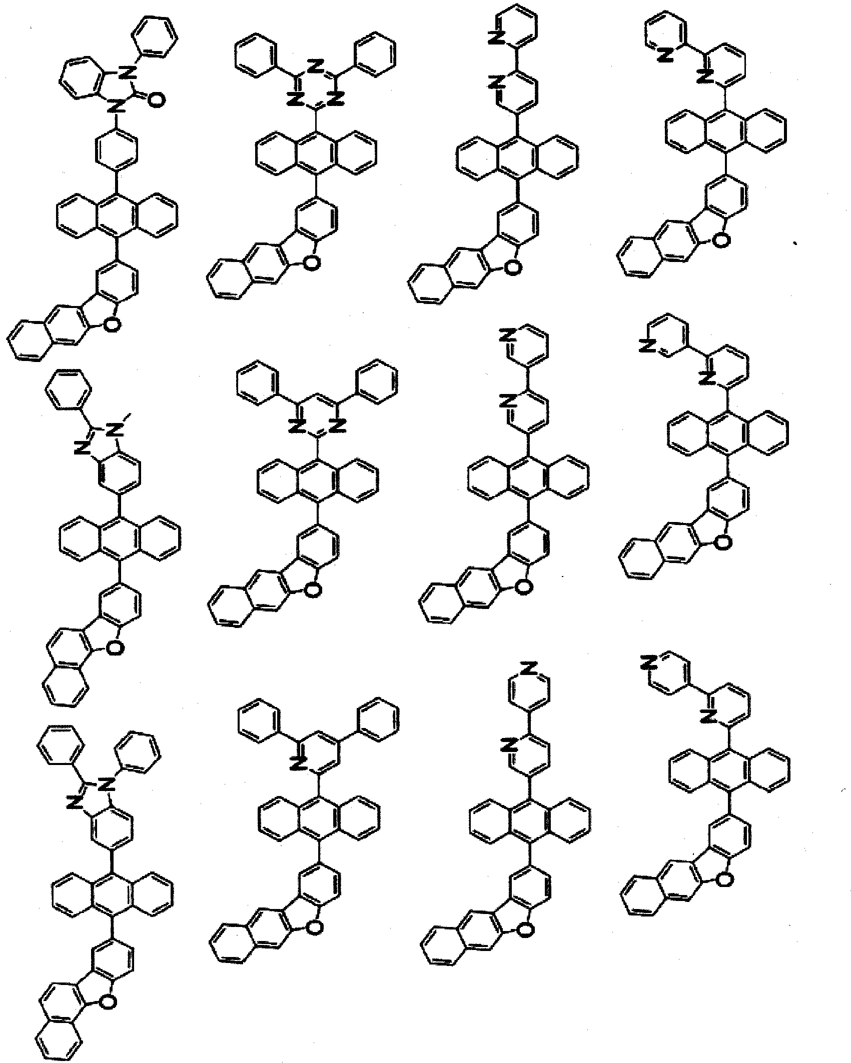

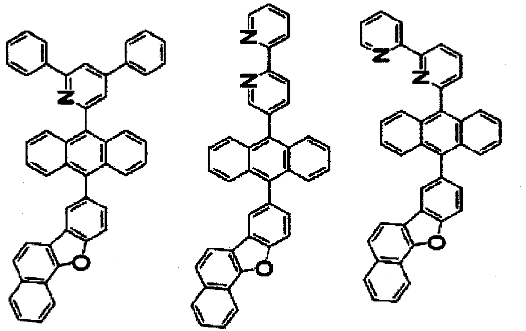

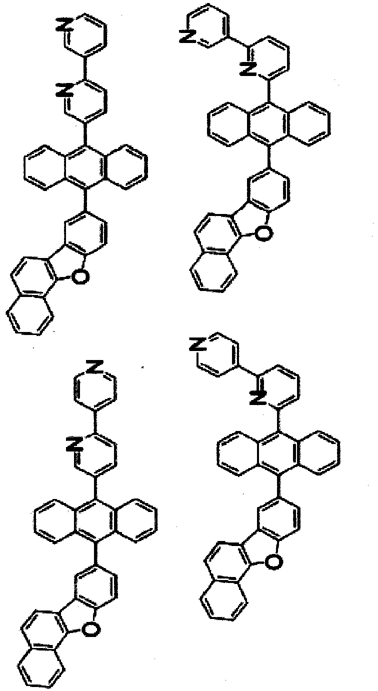

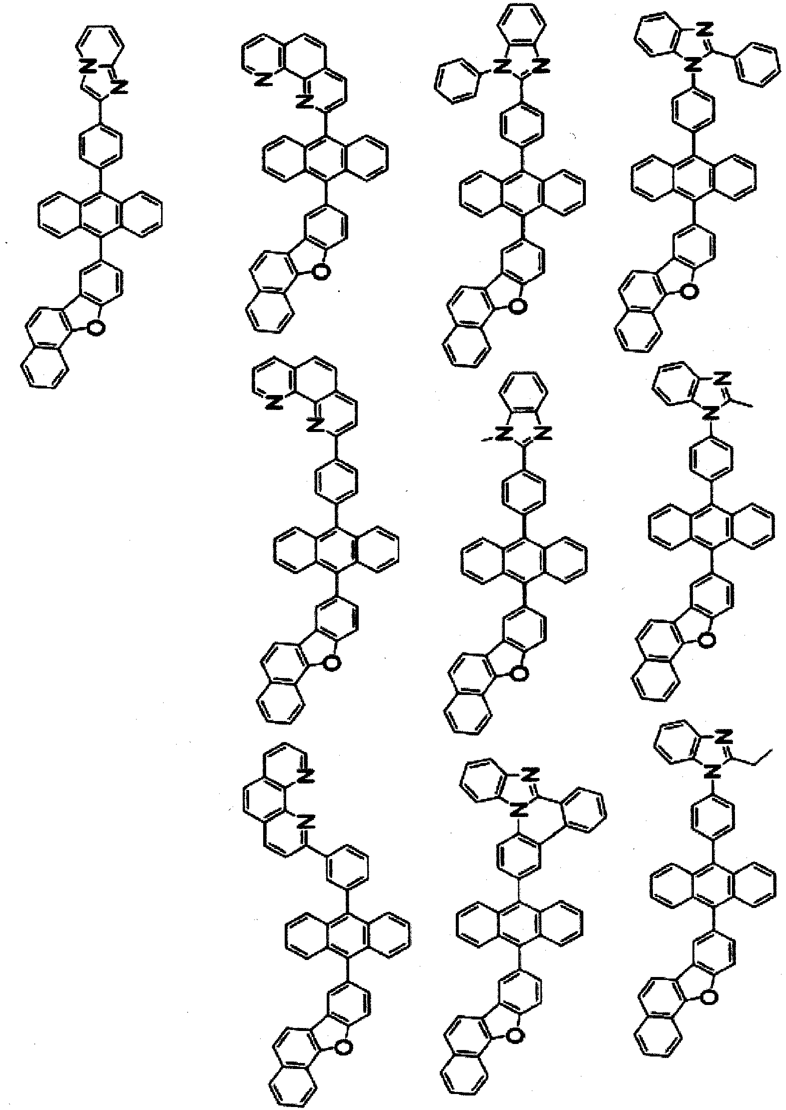

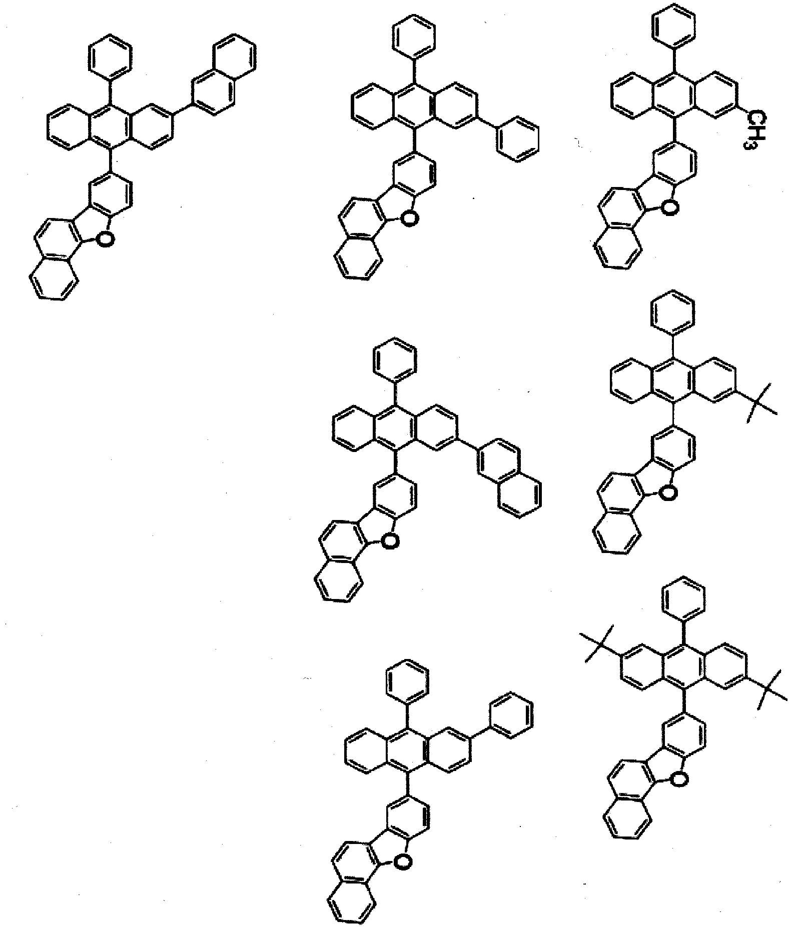

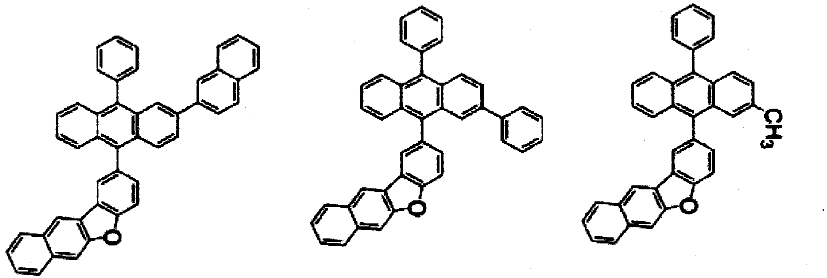

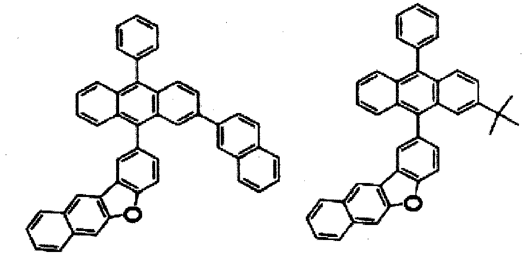

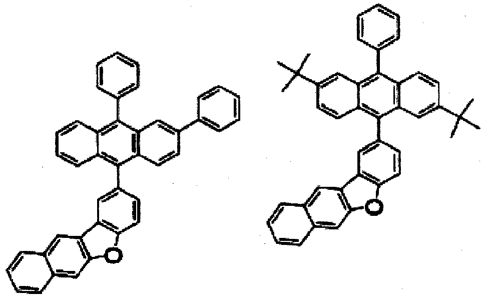

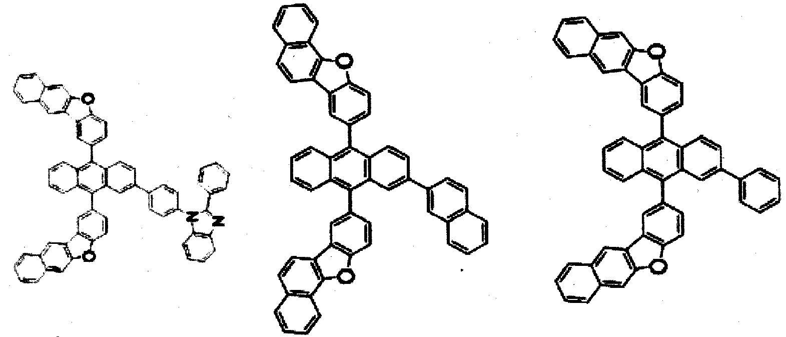

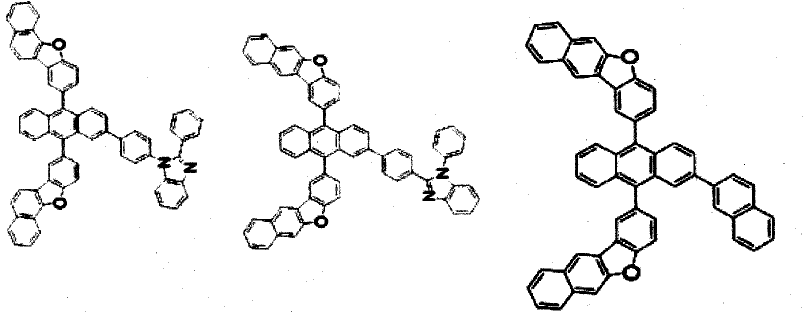

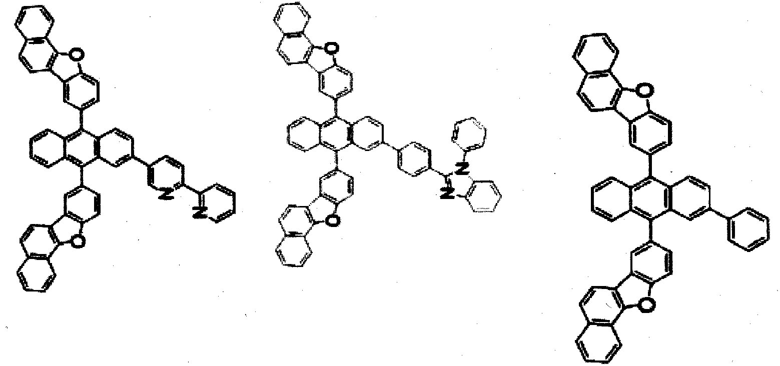

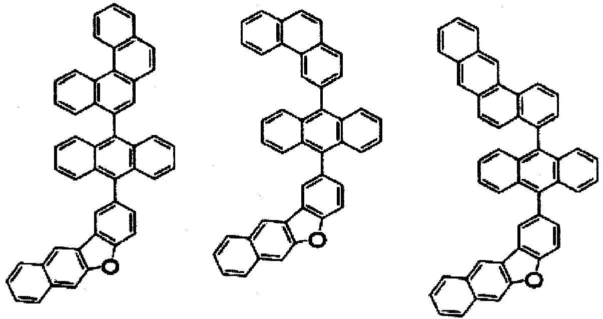

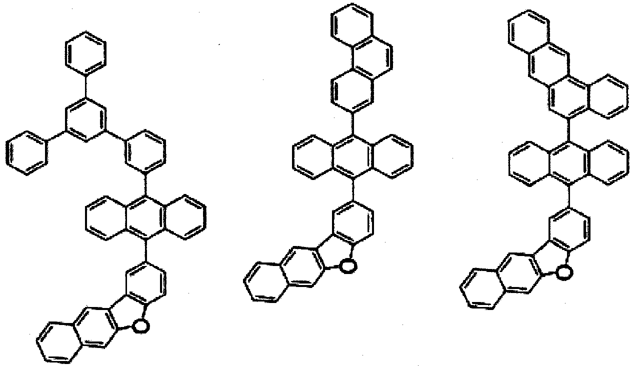

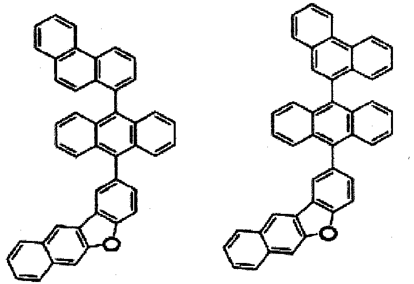

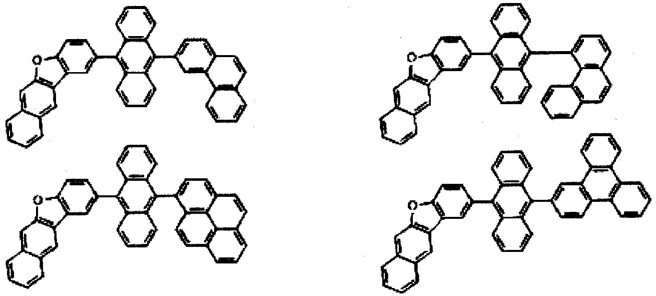

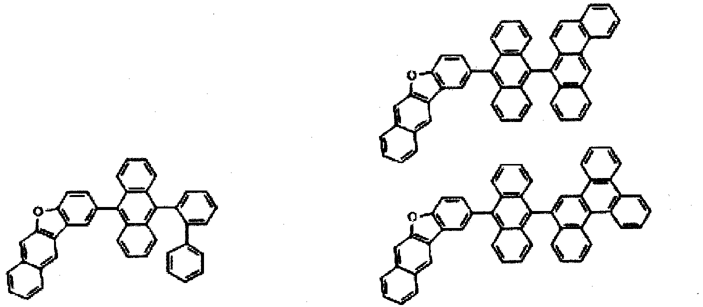

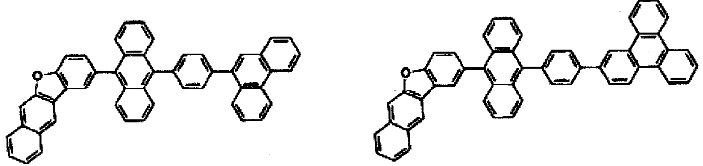

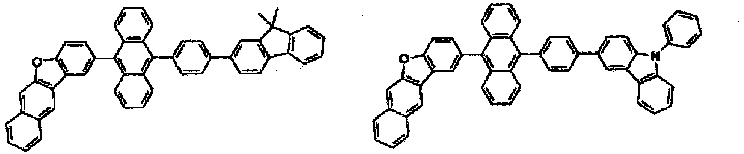

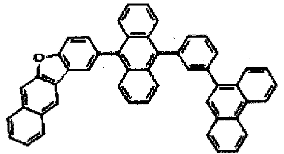

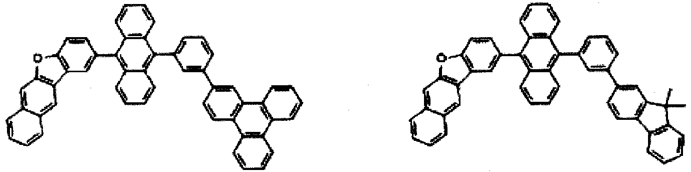

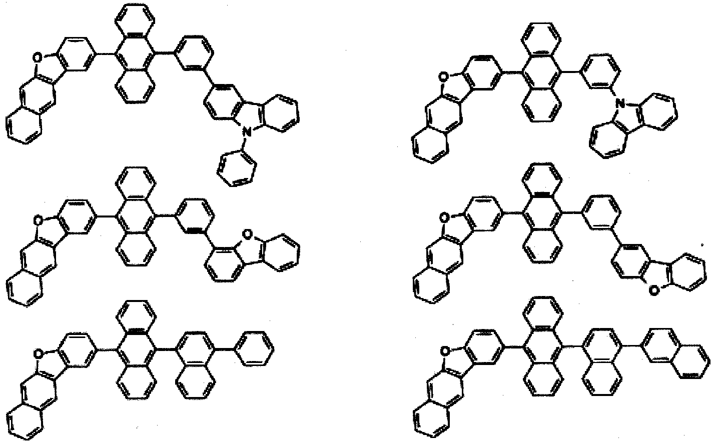

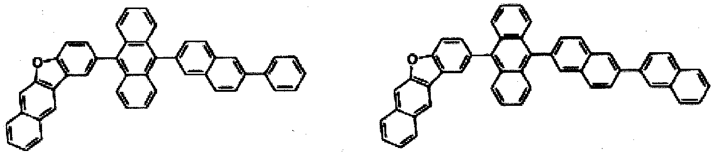

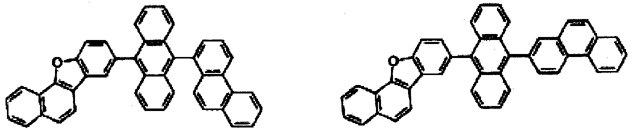

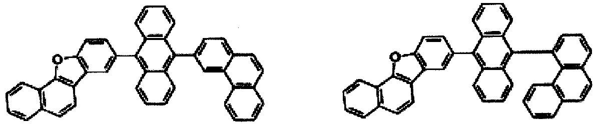

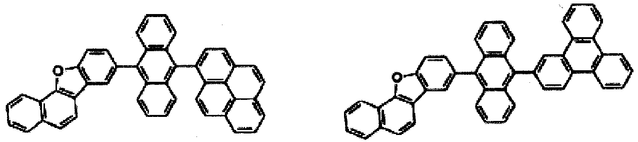

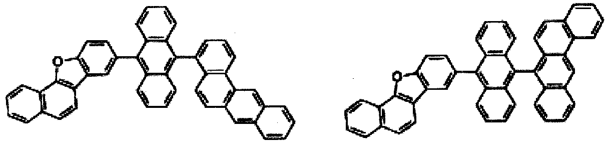

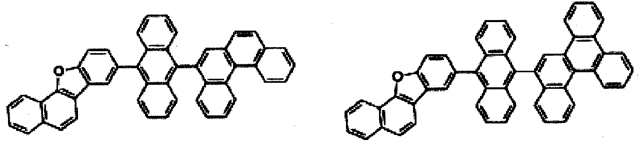

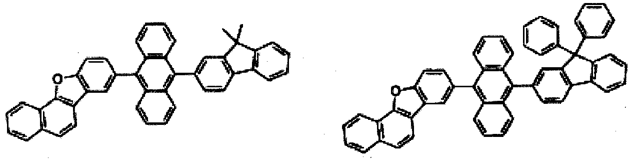

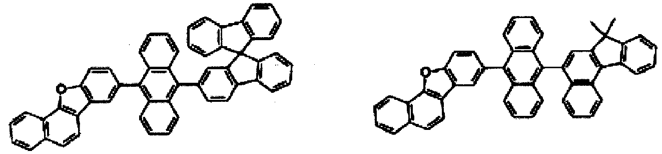

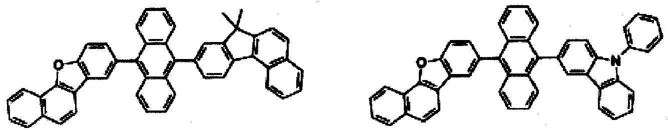

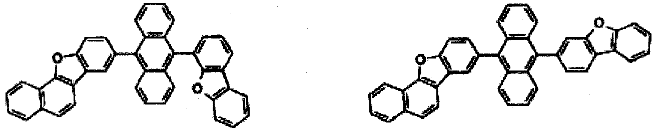

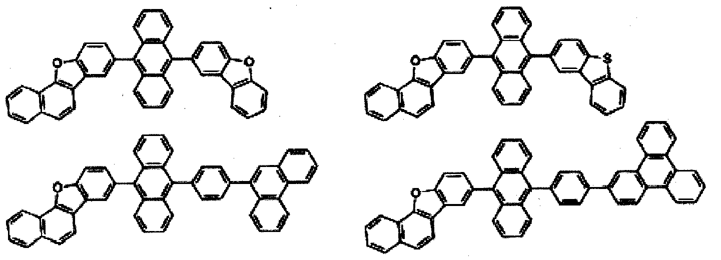

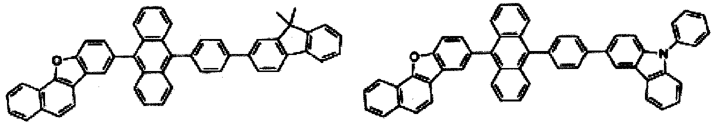

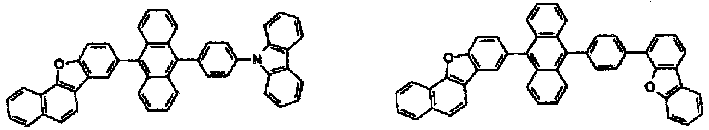

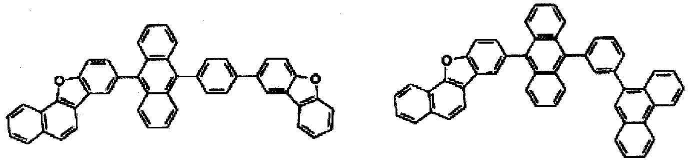

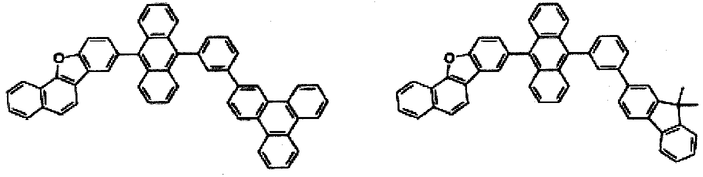

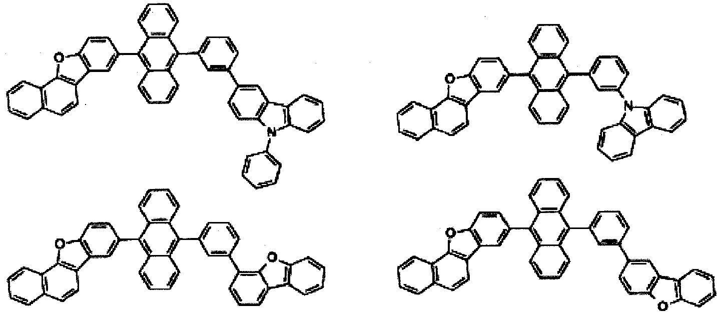

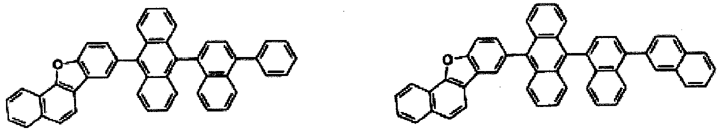

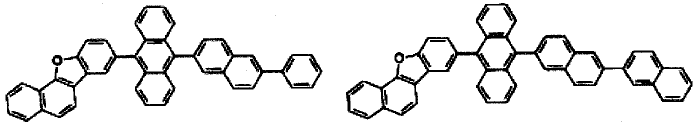

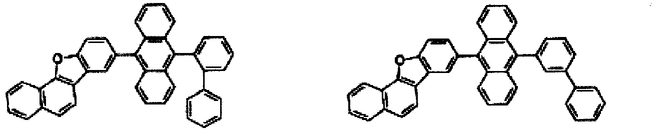

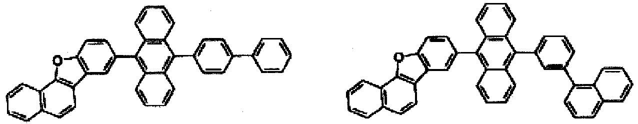

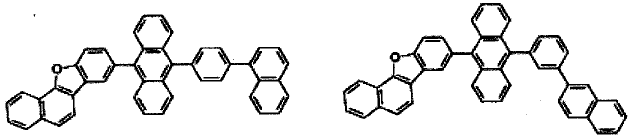

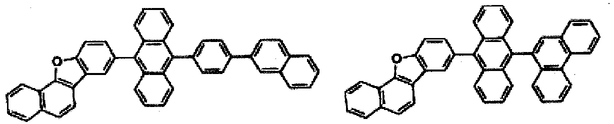

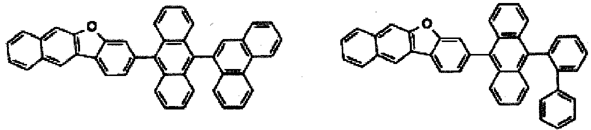

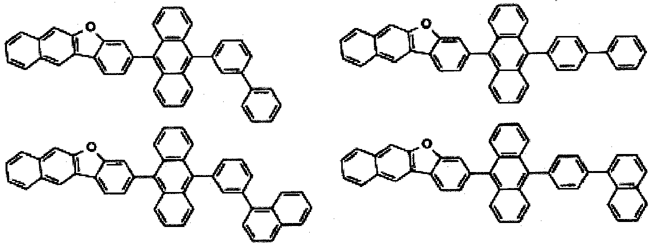

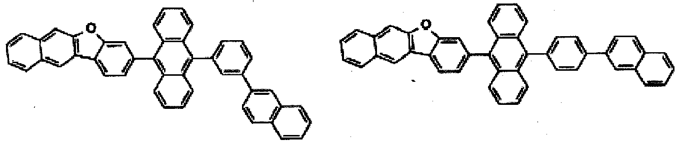

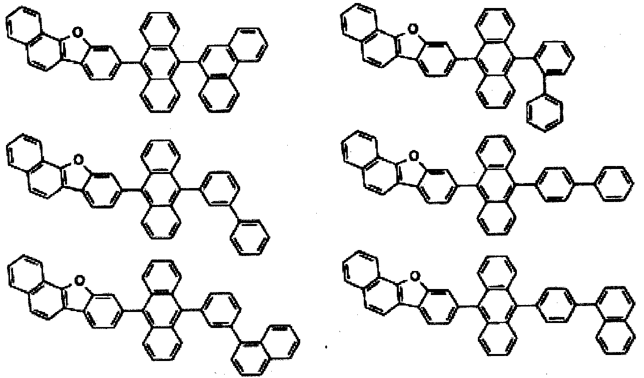

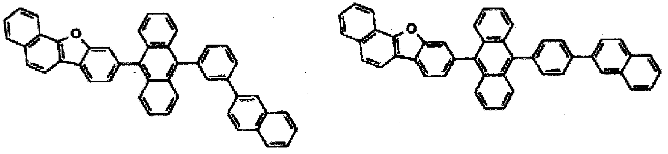

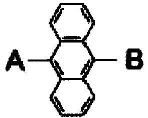

| **A** | **B** |
|---|---|
| 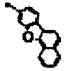 | 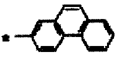 |
| 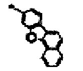 | 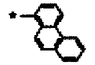 |
| 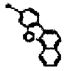 | 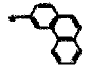 |
| 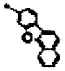 | 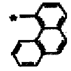 |
| 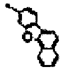 | 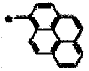 |
| 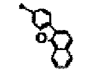 | 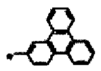 |
| 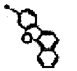 | 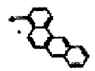 |
| 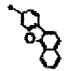 | 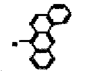 |
| 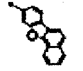 | 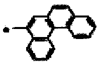 |
| 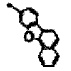 | 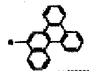 |
| 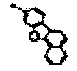 | 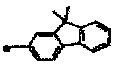 |
| 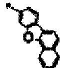 | 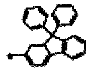 |
| 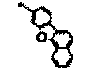 | 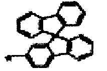 |
| 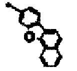 | 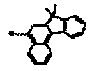 |
| 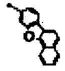 | 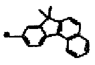 |
| 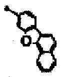 | 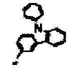 |
| 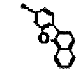 | 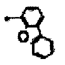 |
| 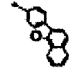 | 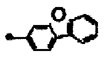 |
| 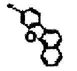 | 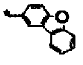 |
| 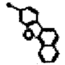 | 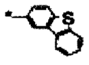 |
| 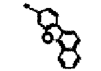 | 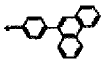 |
| 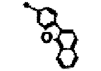 | 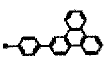 |
| 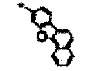 | 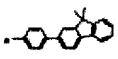 |
| 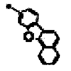 | 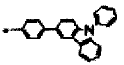 |
| 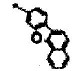 | 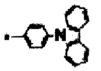 |
| 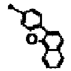 | 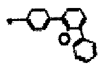 |
| 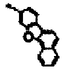 | 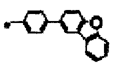 |
| 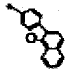 | 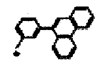 |
| 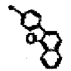 | 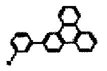 |
| 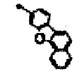 | 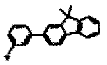 |
| 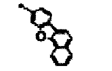 | 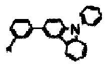 |
| 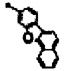 | 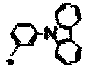 |
| 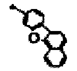 | 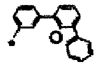 |
| 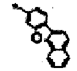 | 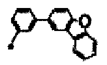 |
| 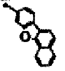 | 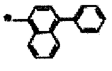 |
| 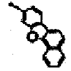 | 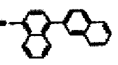 |
| 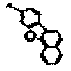 | 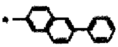 |
| 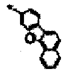 | 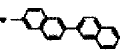 |

| | |
|---|---|
| An asterisk mark (*) in the table each shows a bonding position to an anthracene ring. | |

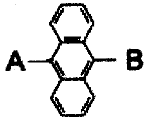

| **A** | **B** |
|---|---|
| 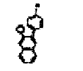 | 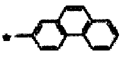 |
| 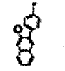 | 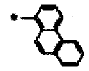 |
| 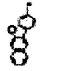 | 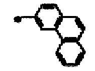 |
| 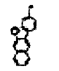 | 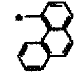 |
| 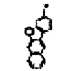 | 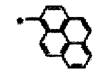 |
| 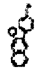 | 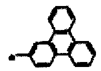 |
| 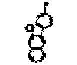 | 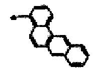 |
| 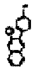 | 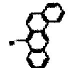 |
| 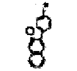 | 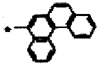 |
| 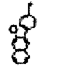 | 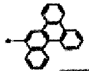 |
| 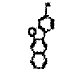 | 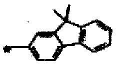 |
| 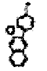 | 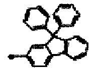 |
| 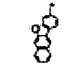 | 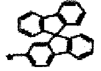 |
| 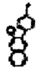 | 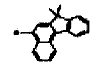 |
| 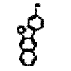 | 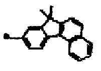 |
| 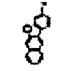 | 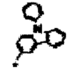 |
| 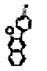 | 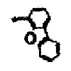 |
| 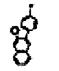 | 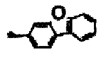 |
| 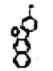 | 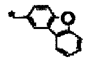 |
| 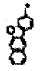 | 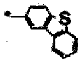 |
| 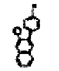 | 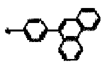 |
| 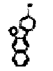 | 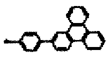 |
| 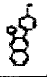 | 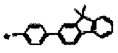 |
| 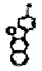 | 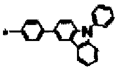 |
| 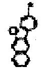 | 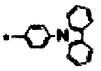 |
| 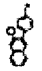 | 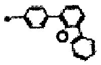 |
| 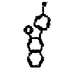 | 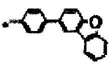 |
| 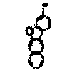 | 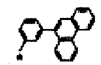 |
| 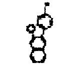 | 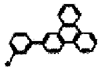 |
| 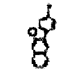 | 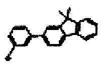 |
| 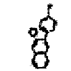 | 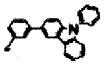 |
| 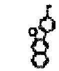 | 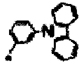 |
| 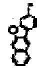 | 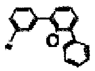 |
| 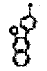 | 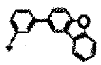 |
| 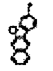 | 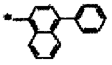 |
| 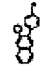 | 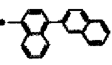 |
| 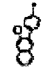 | 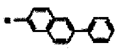 |
| 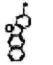 | 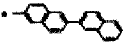 |

| | |
|---|---|
| An asterisk mark (*) in the table each shows a bonding position to an anthracene ring. | |

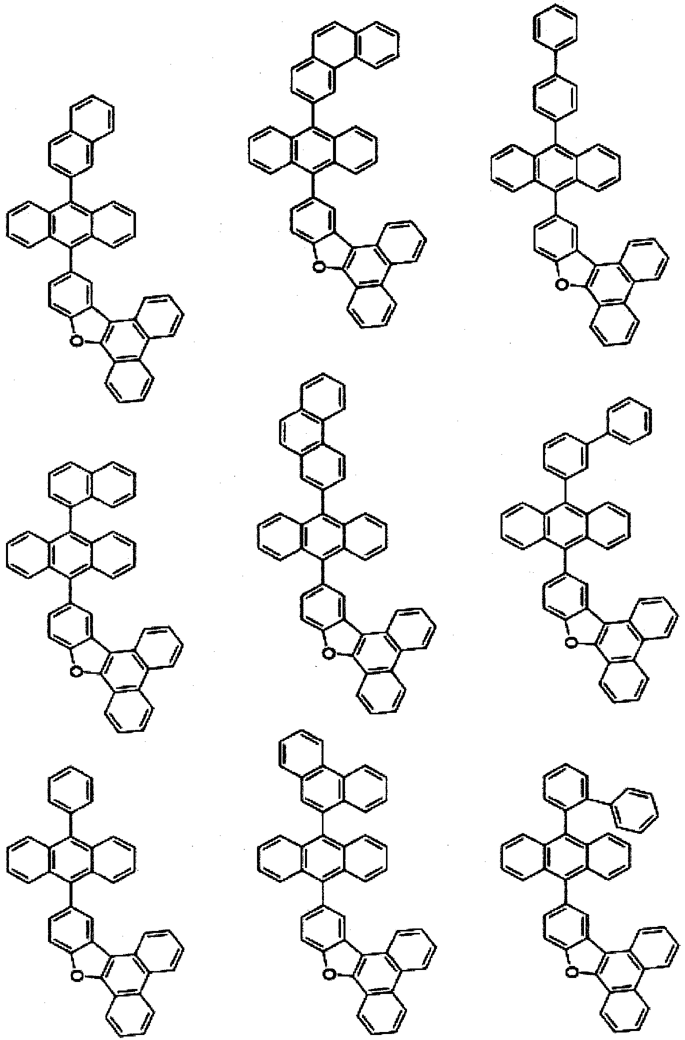

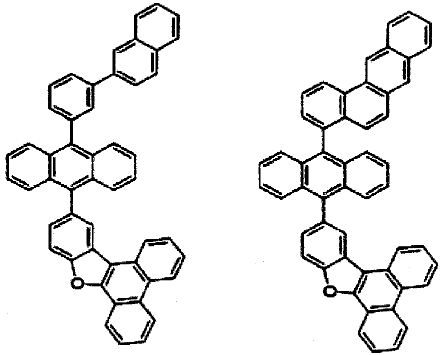

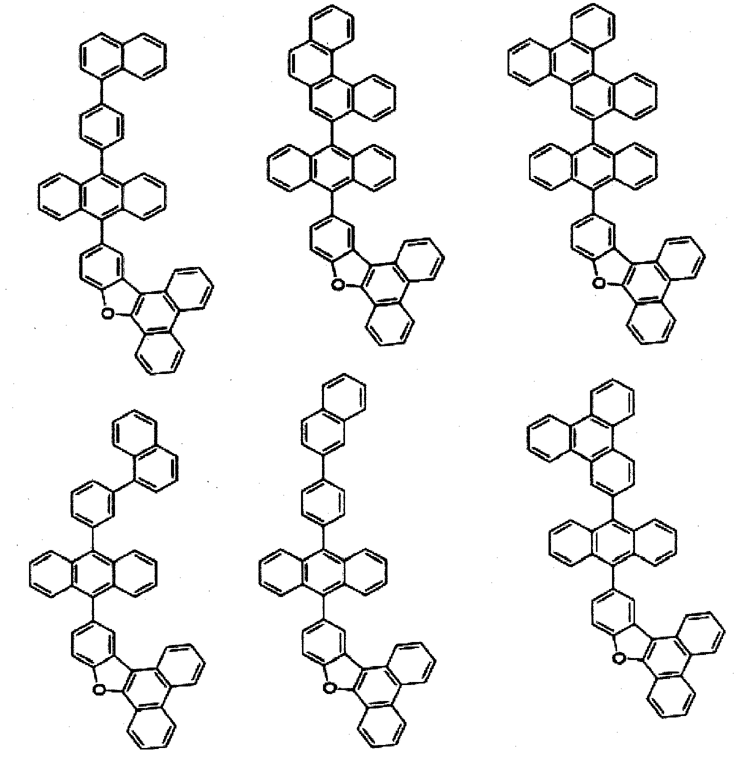

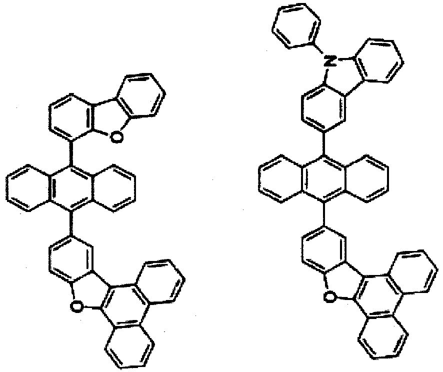

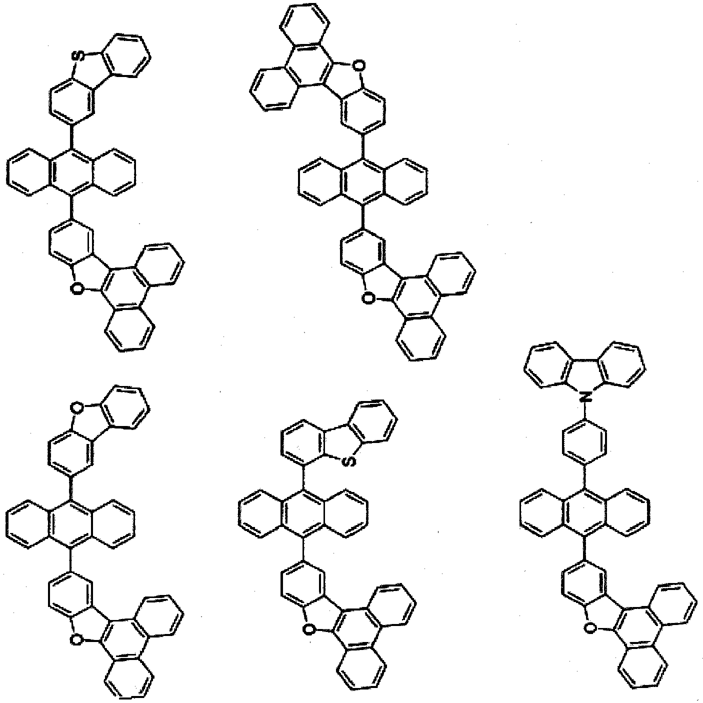

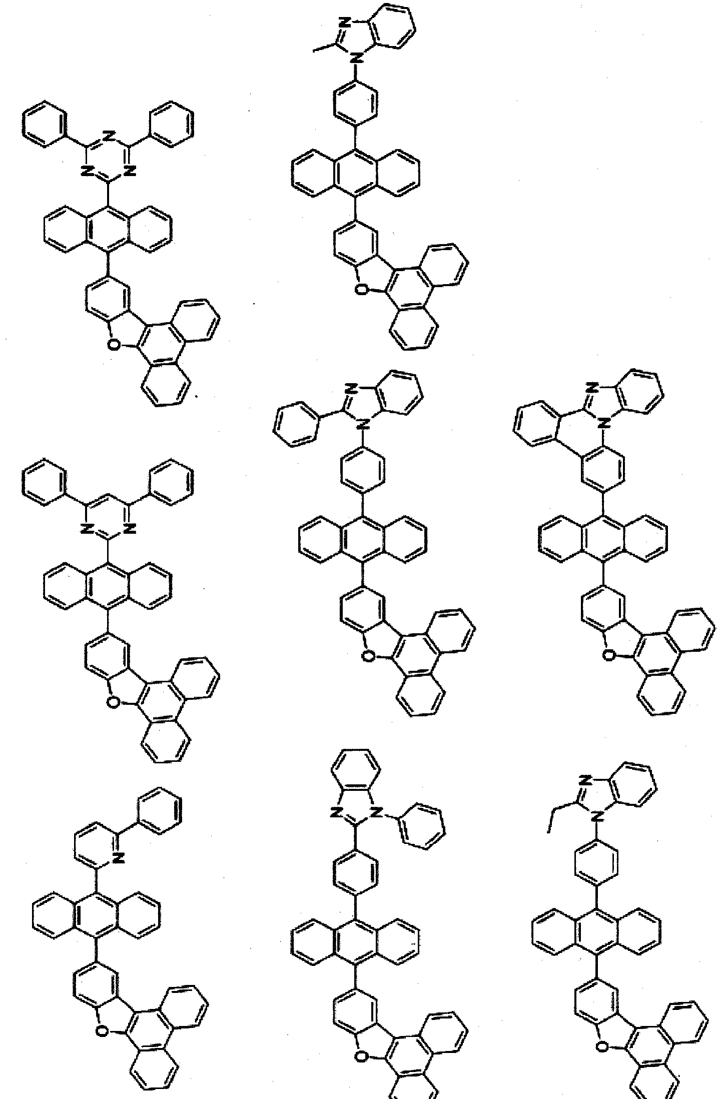

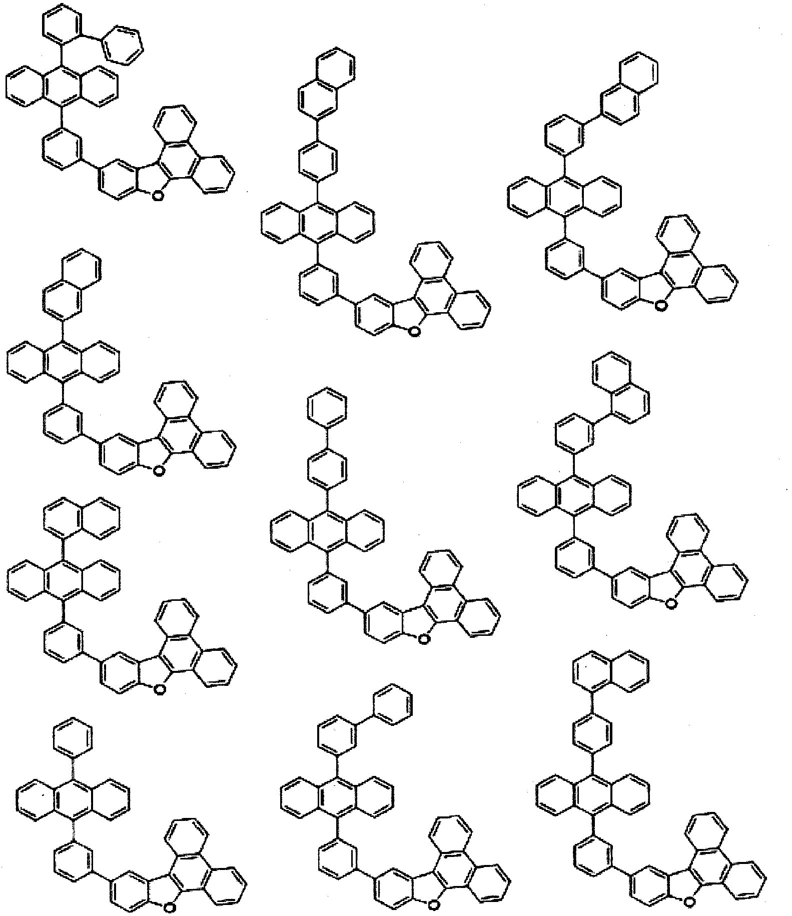

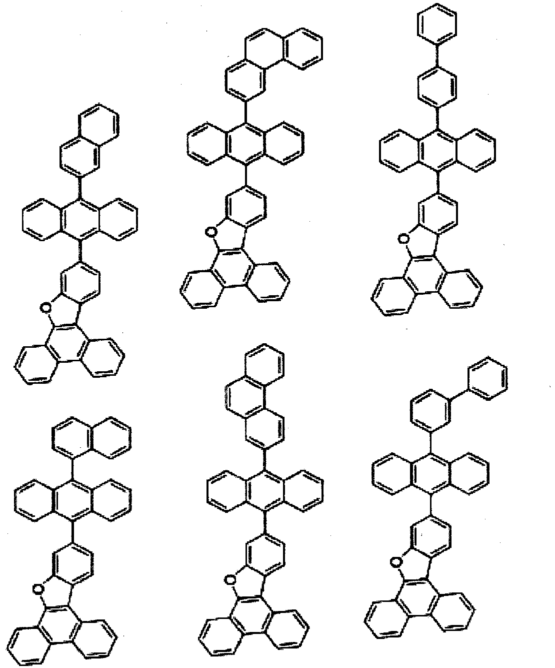

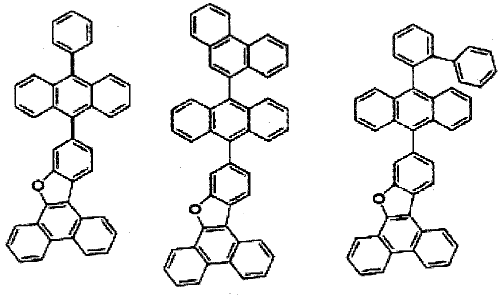

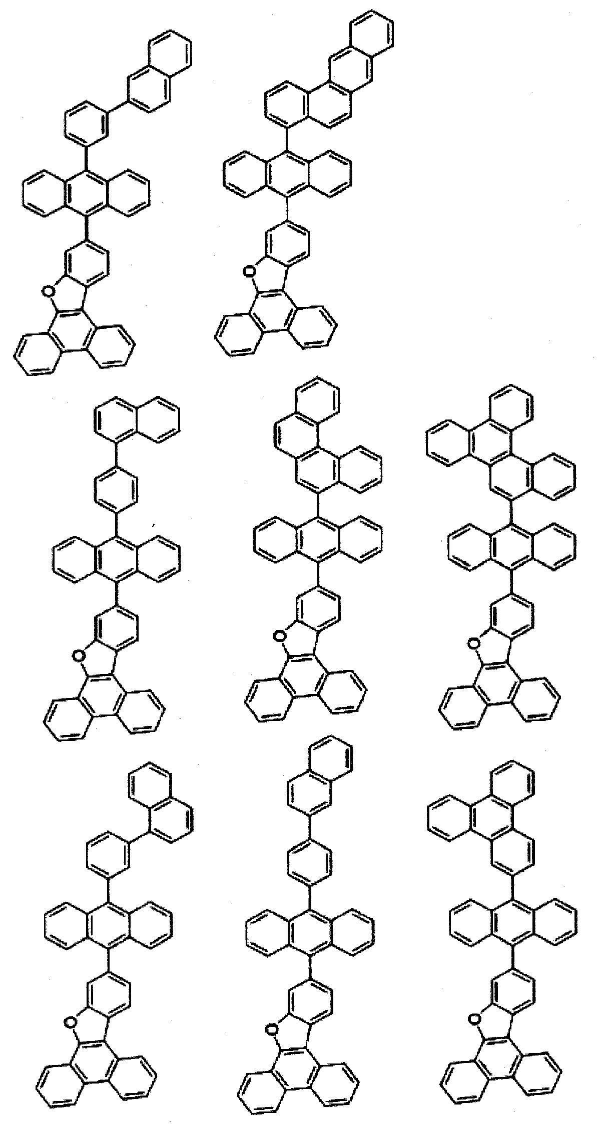

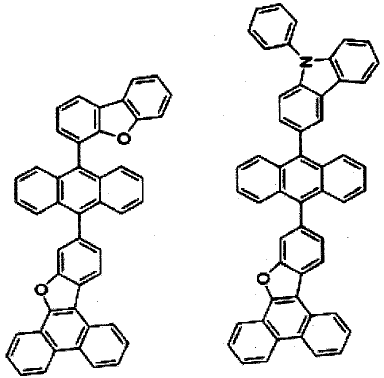

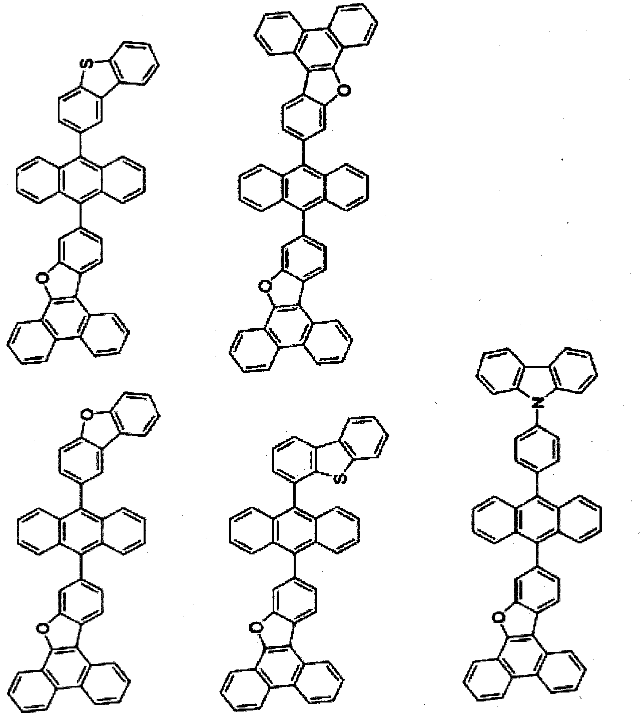

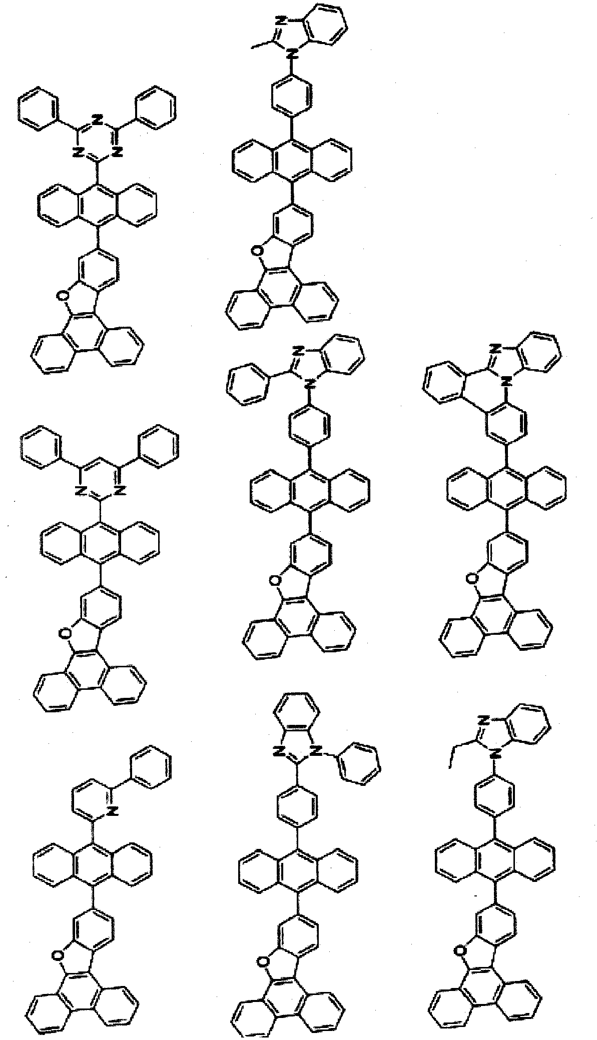

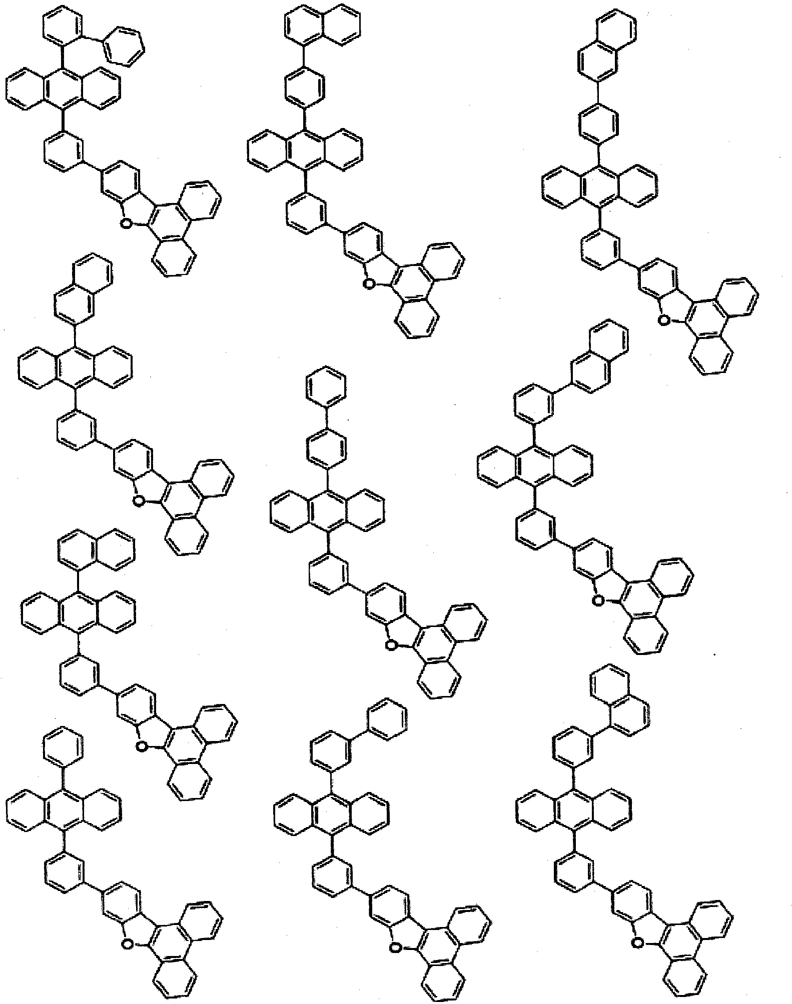

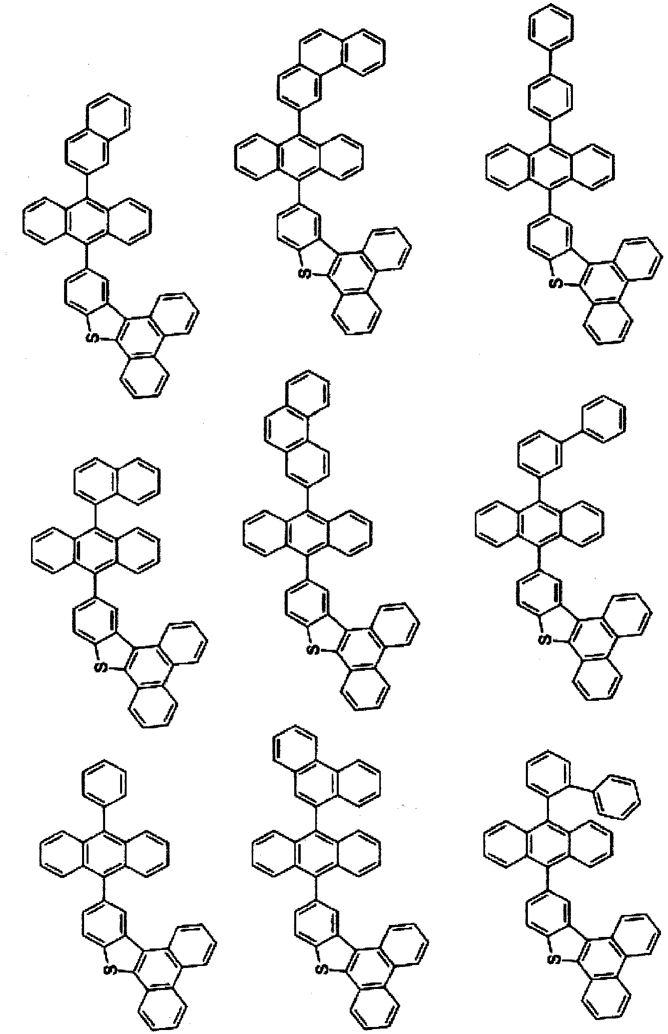

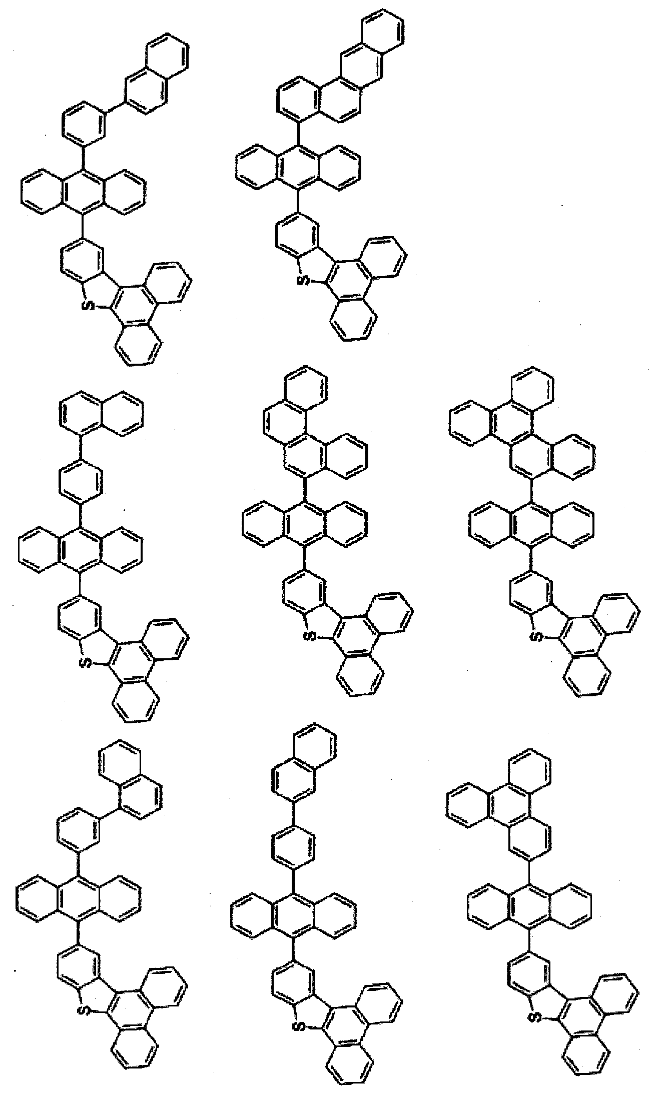

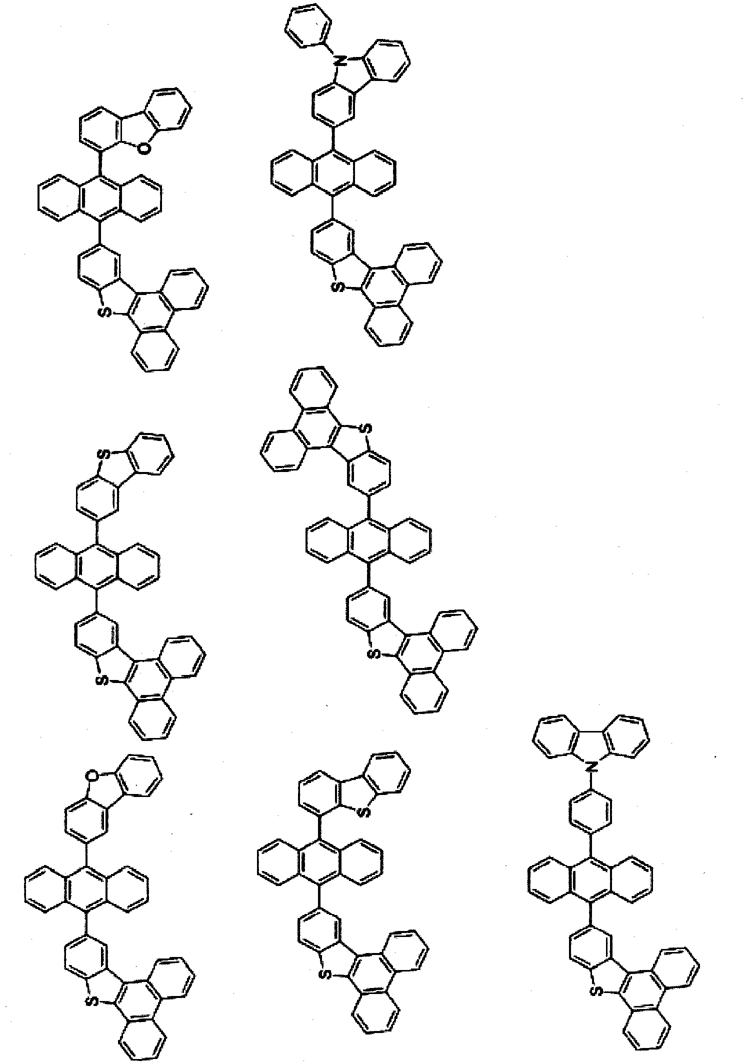

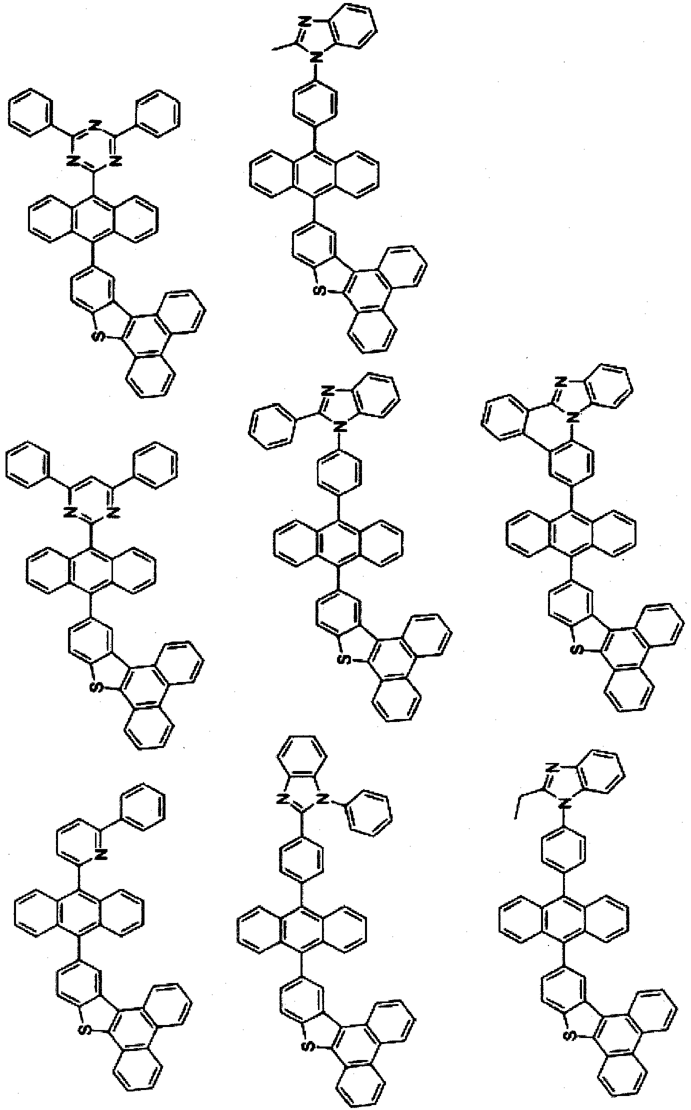

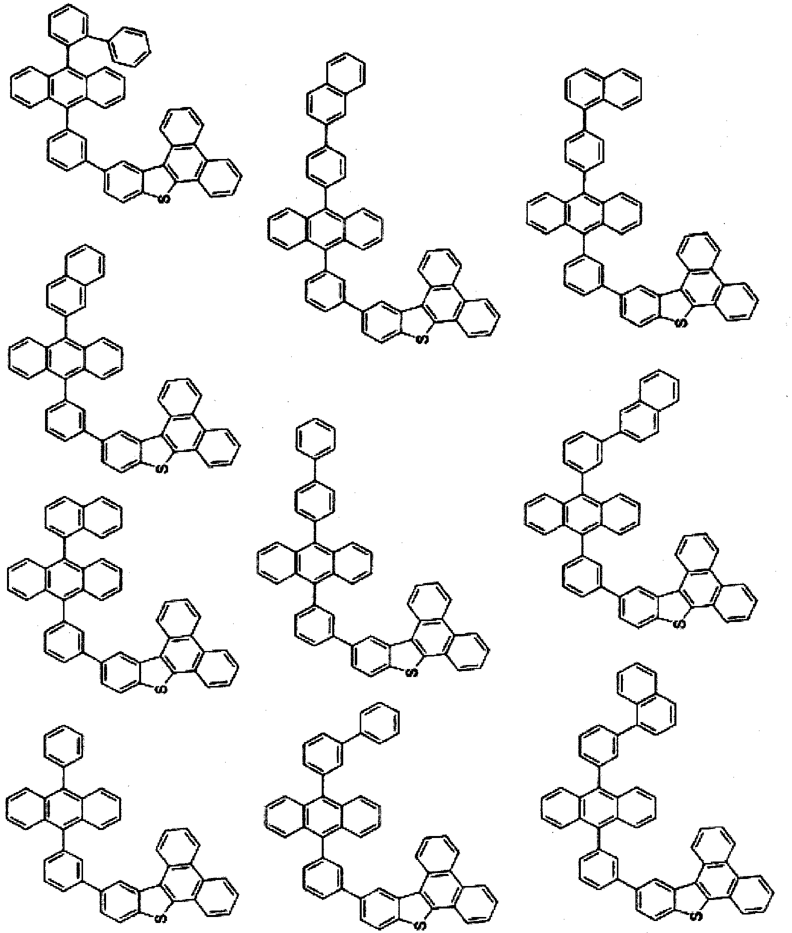

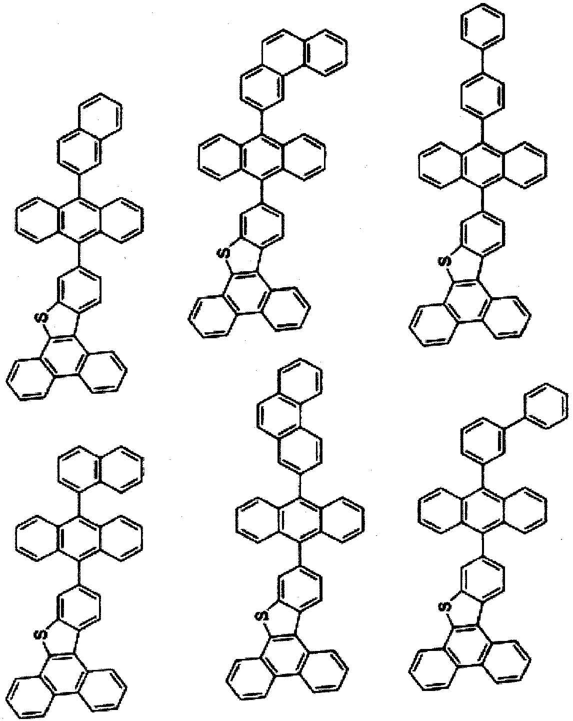

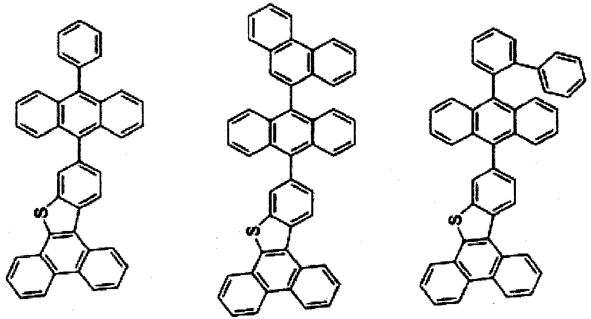

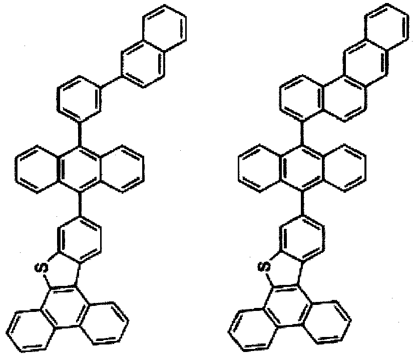

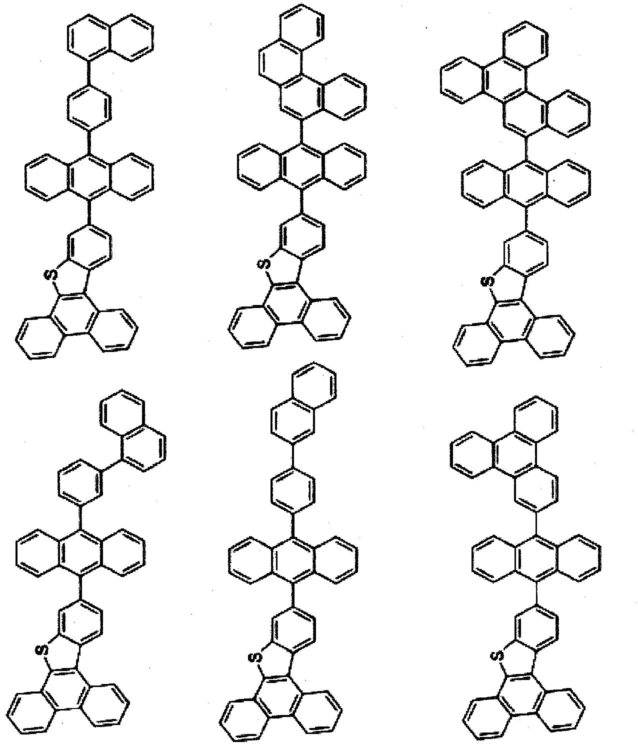

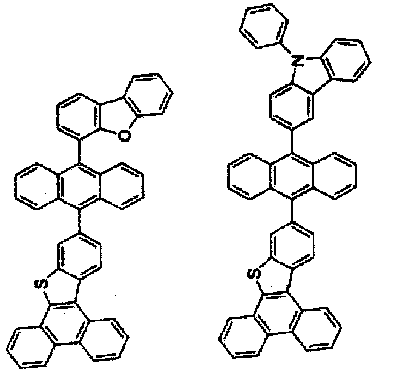

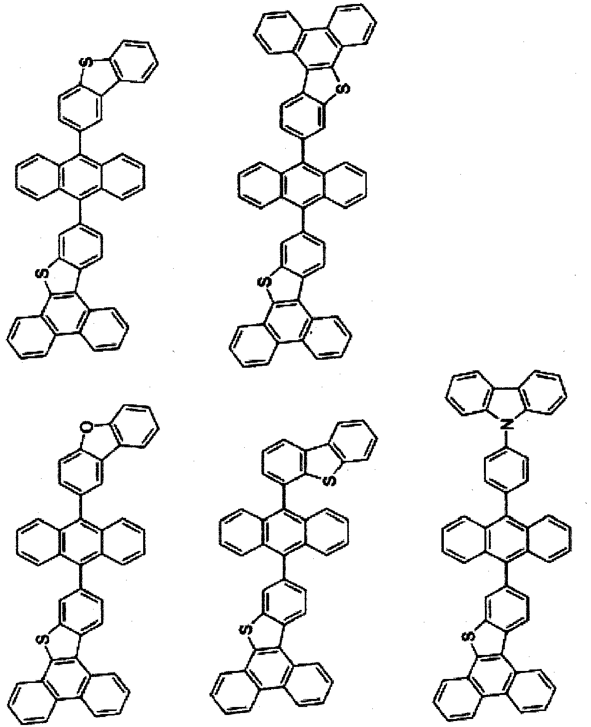

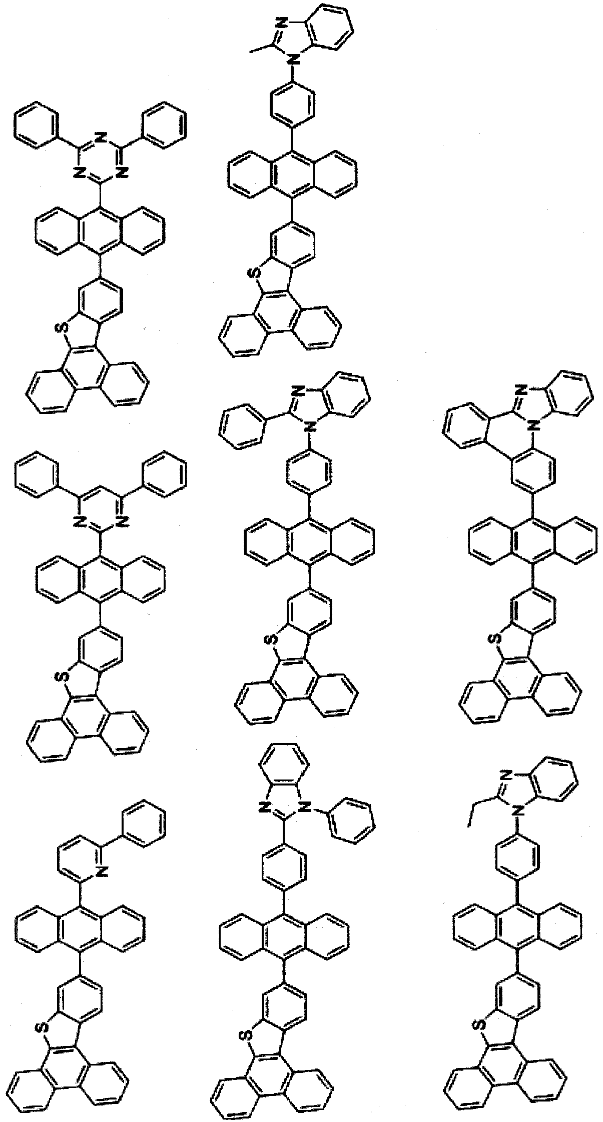

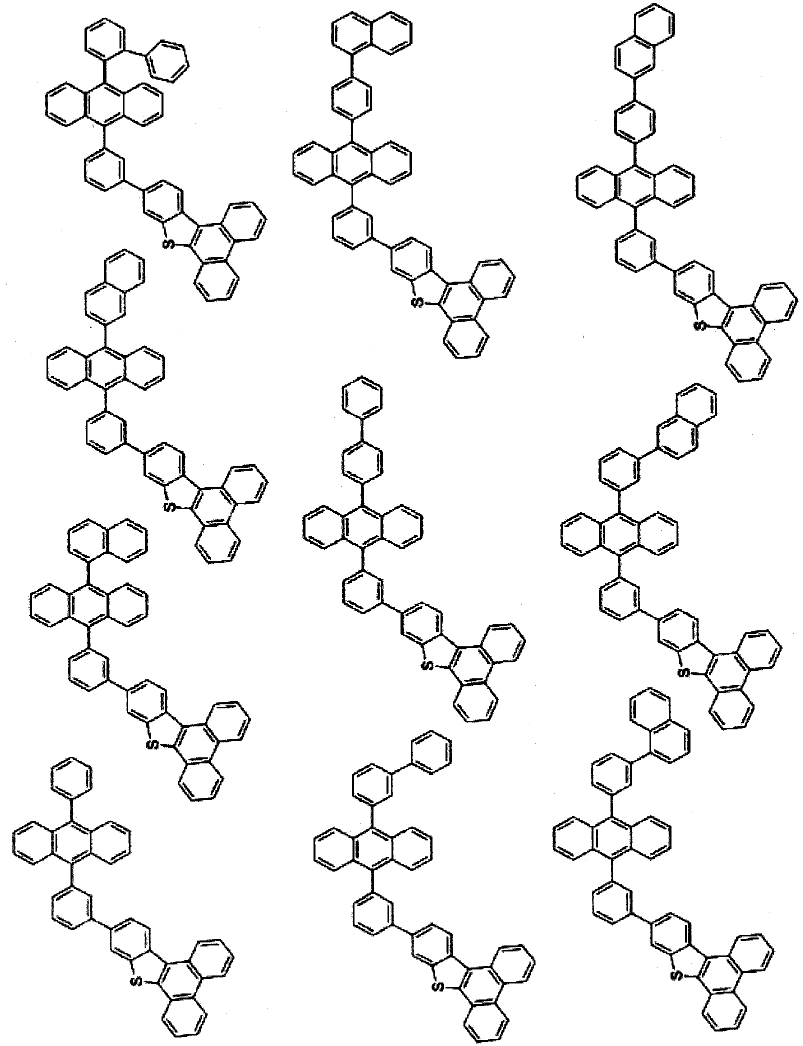

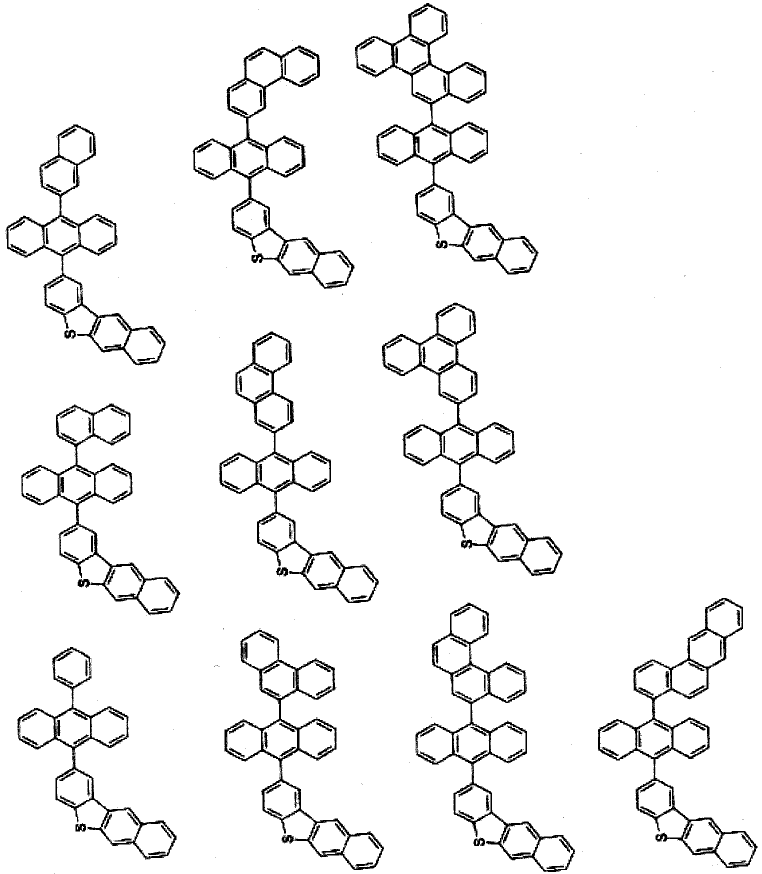

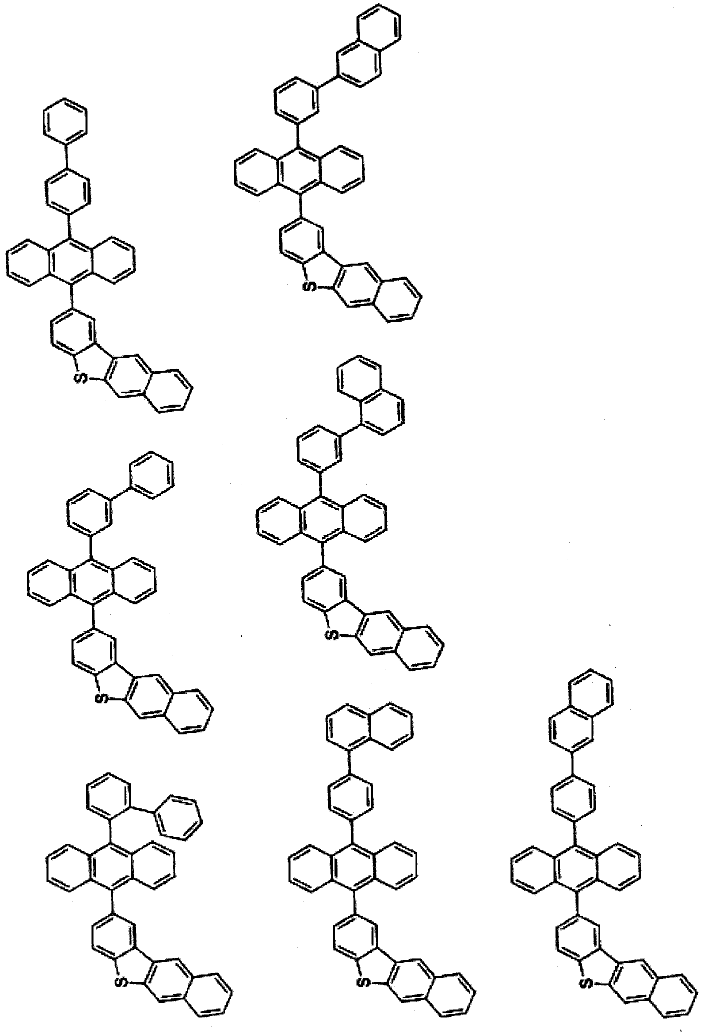

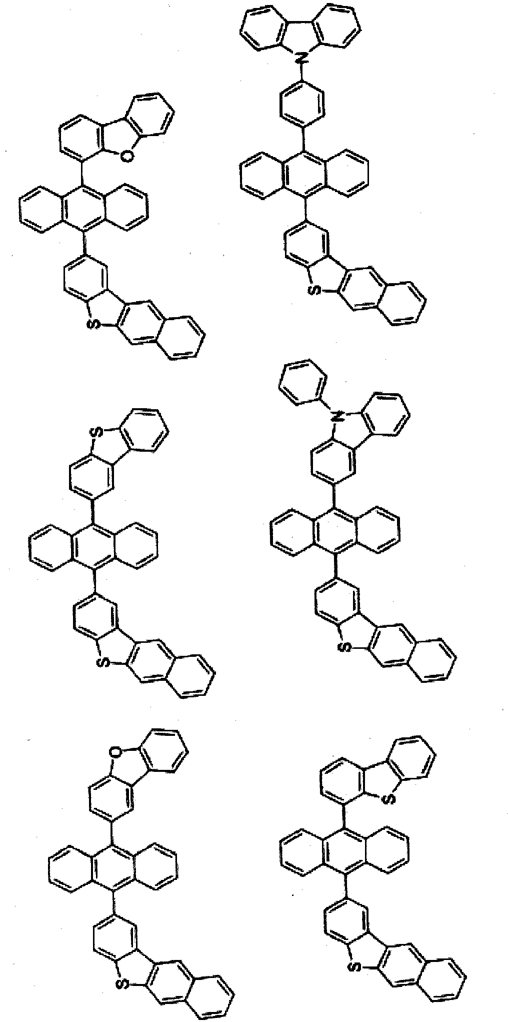

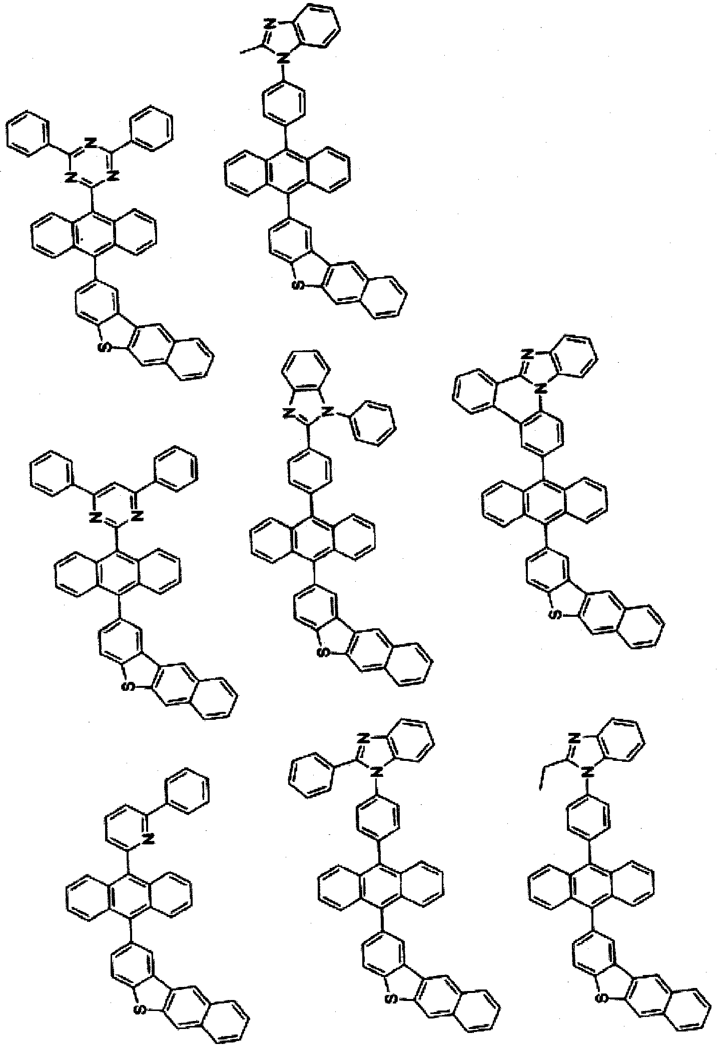

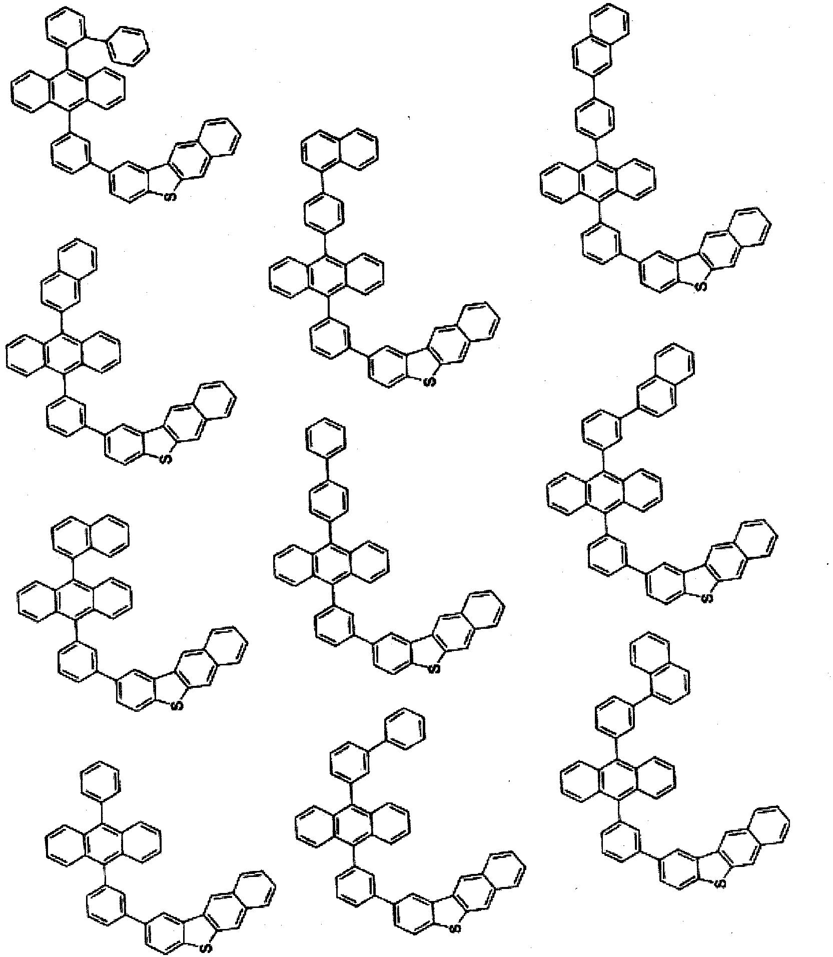

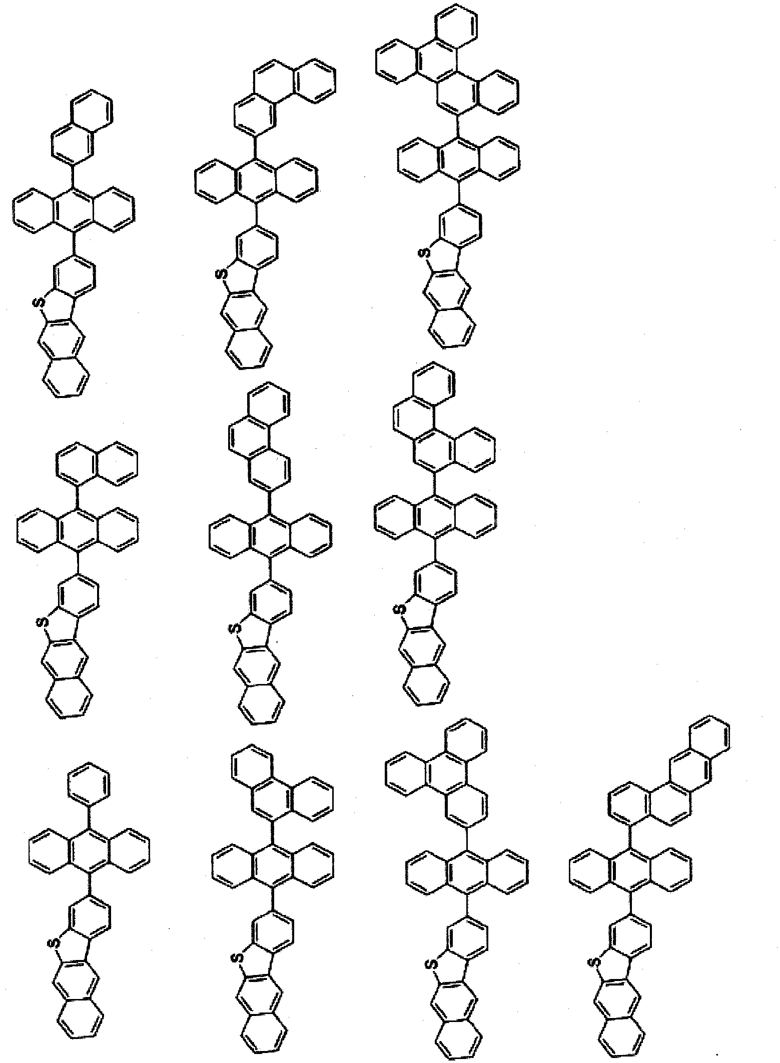

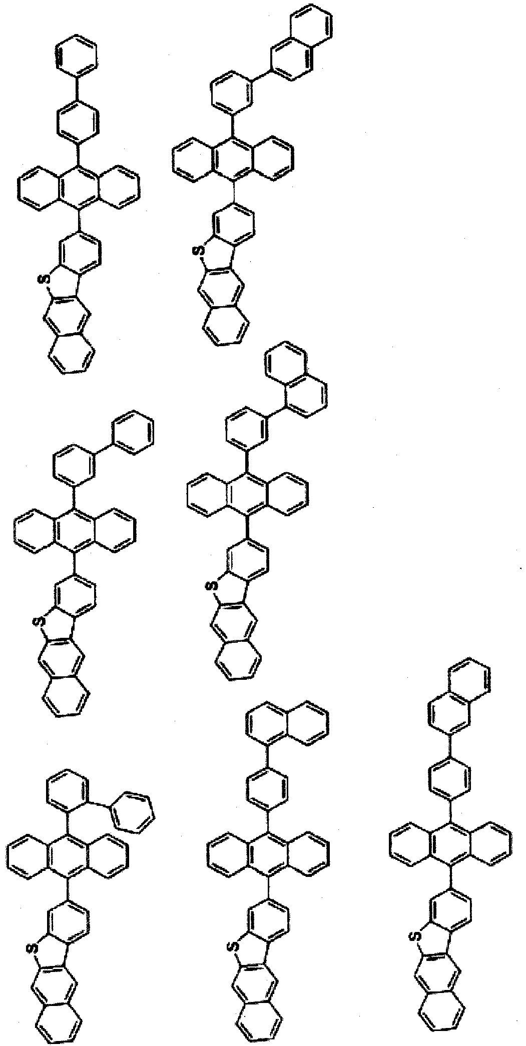

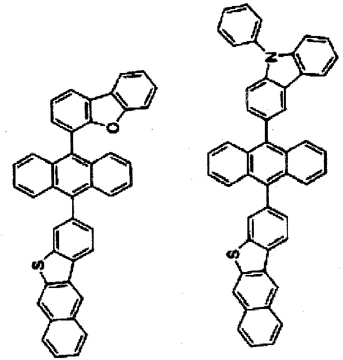

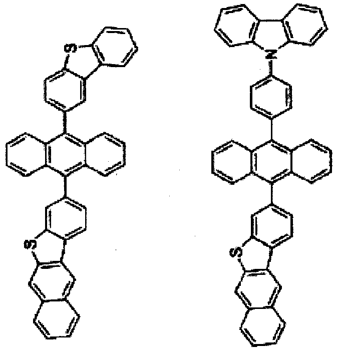

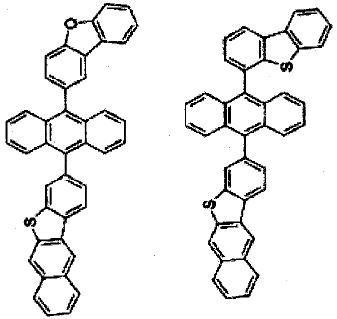

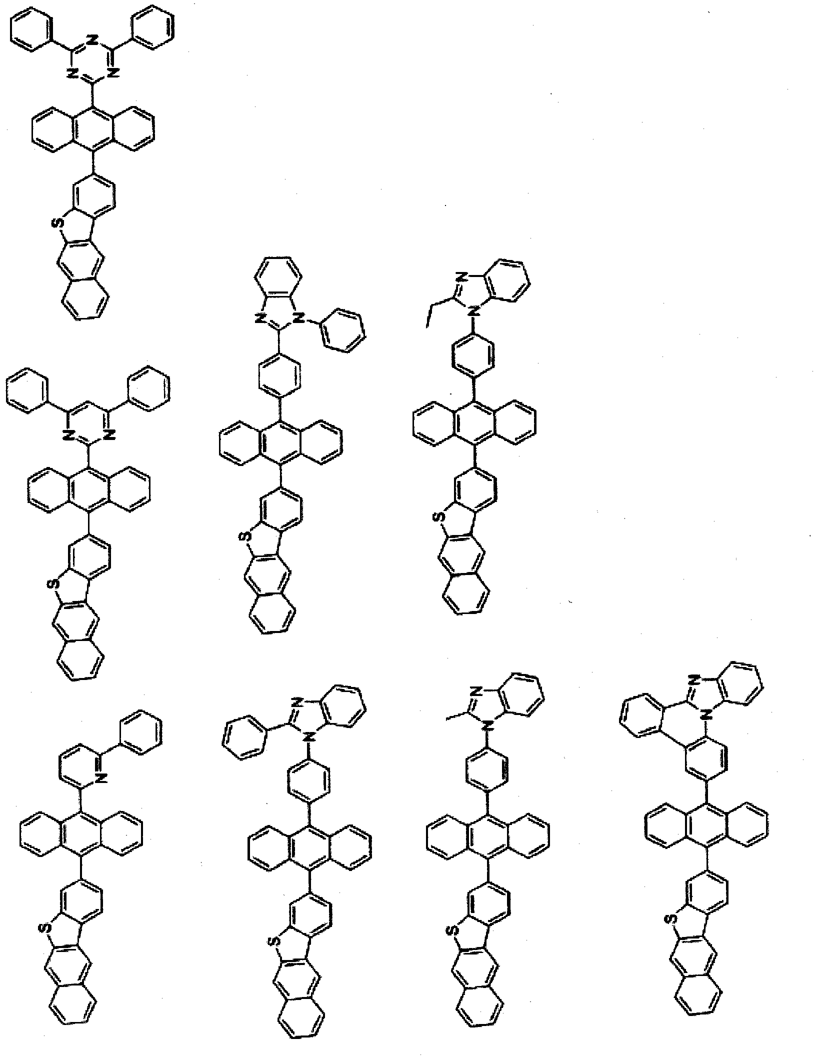

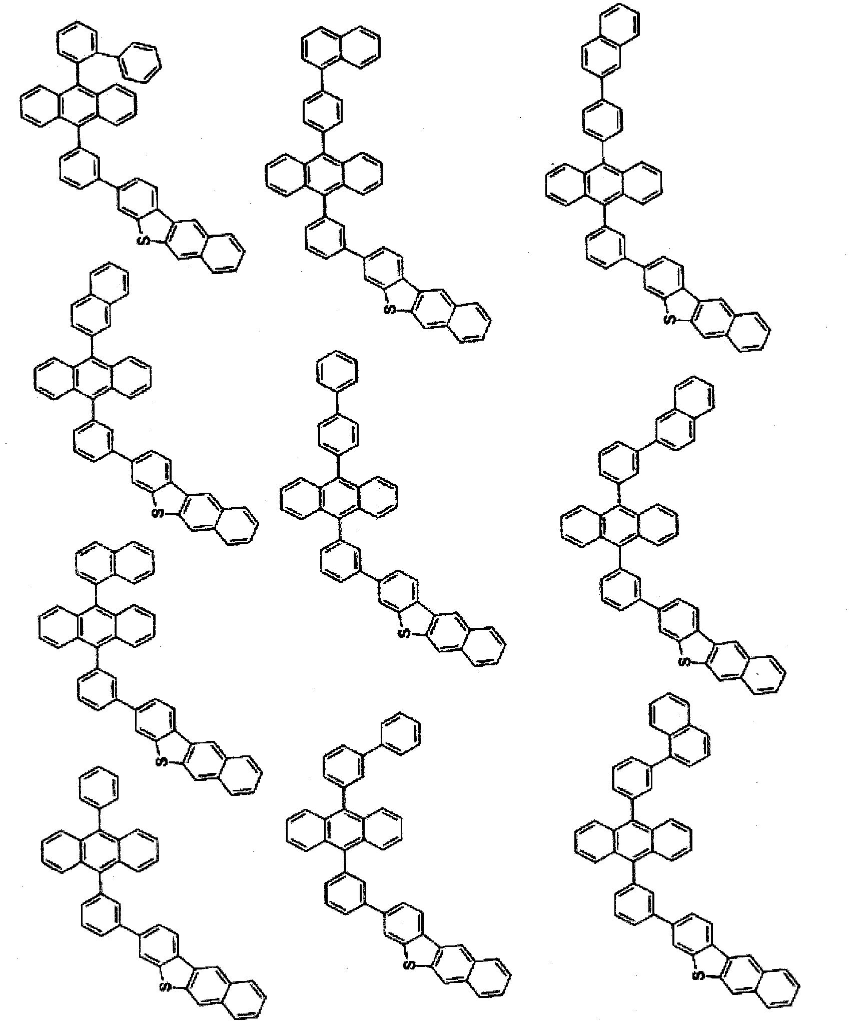

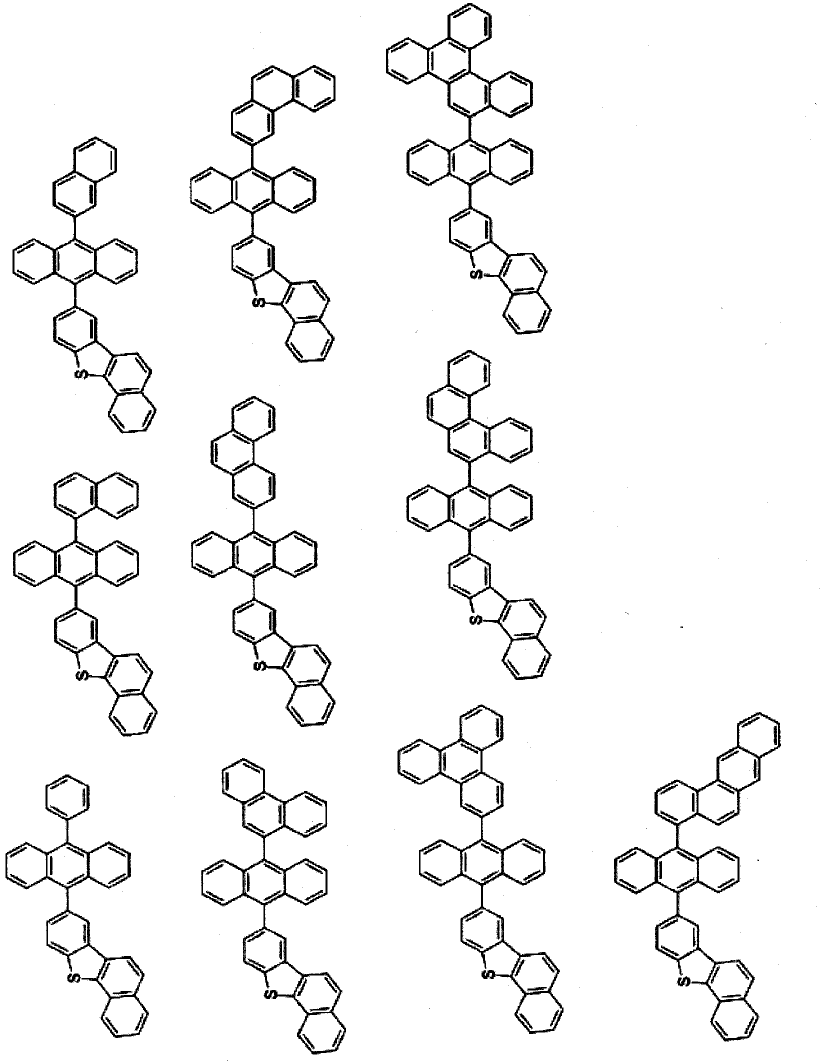

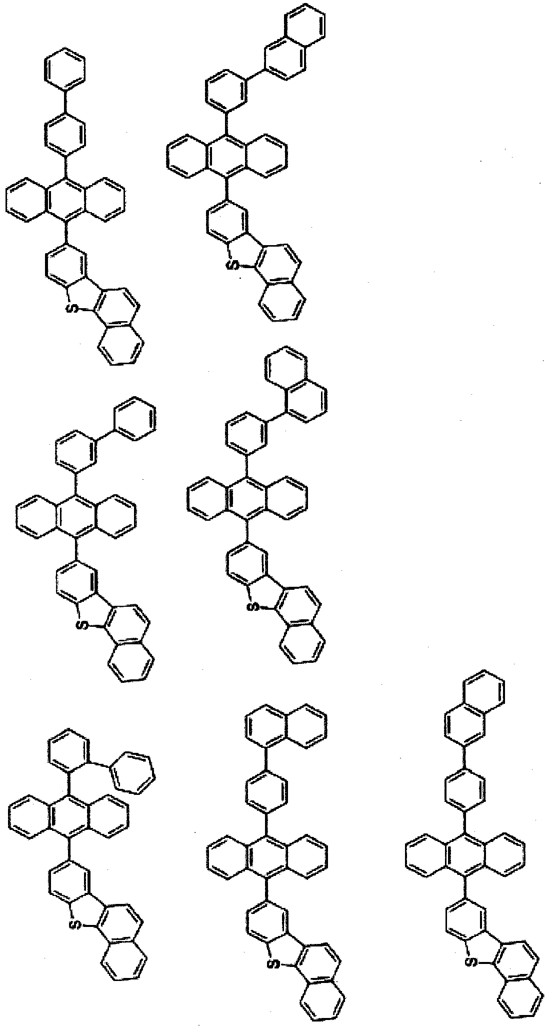

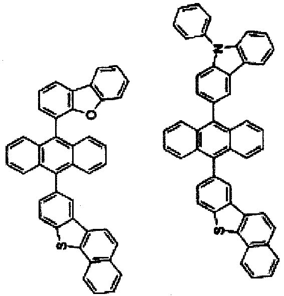

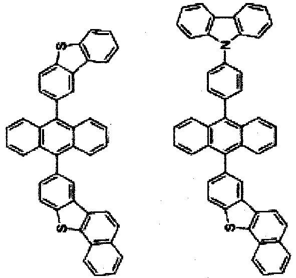

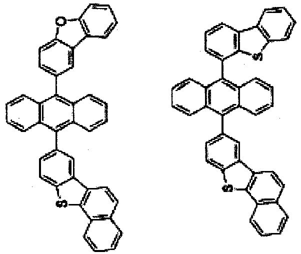

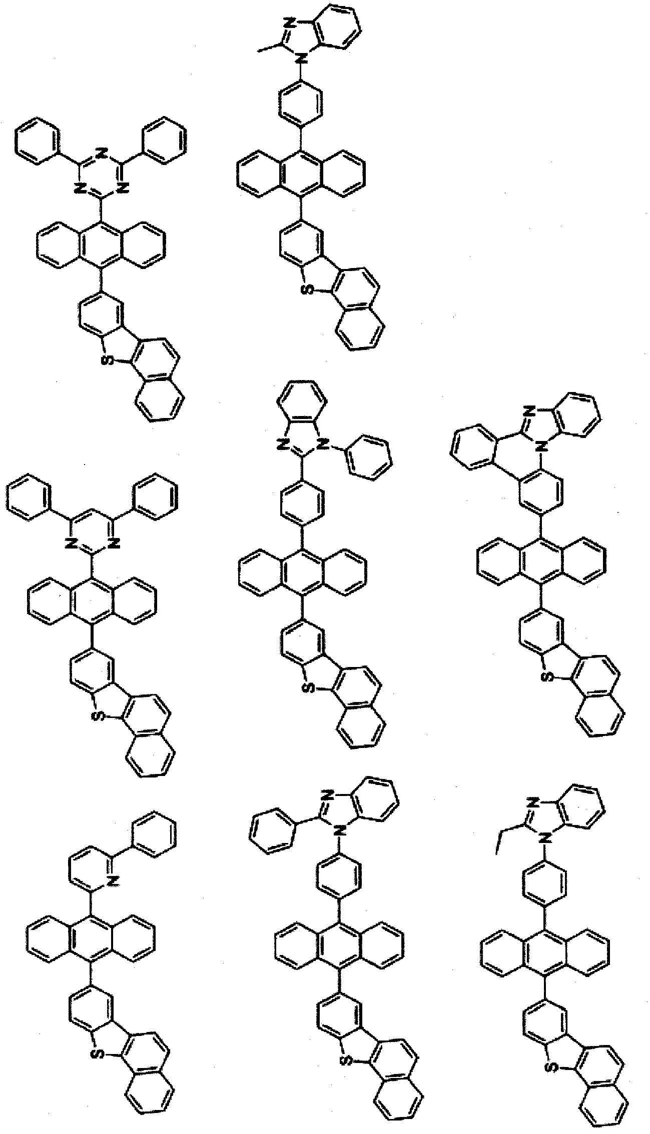

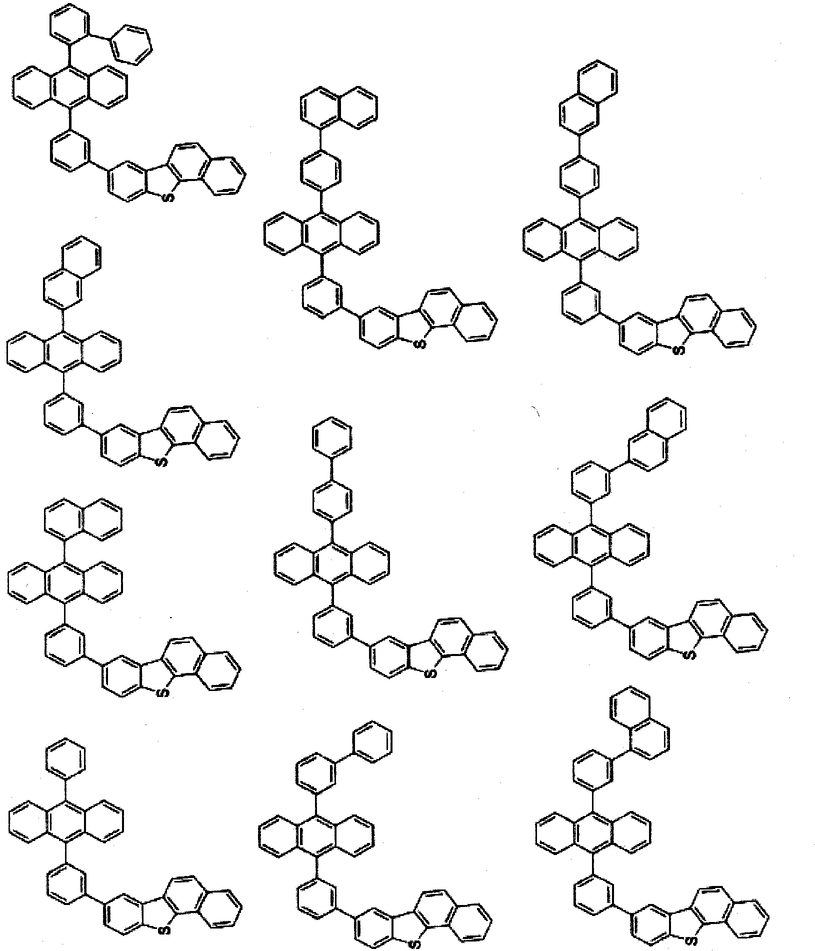

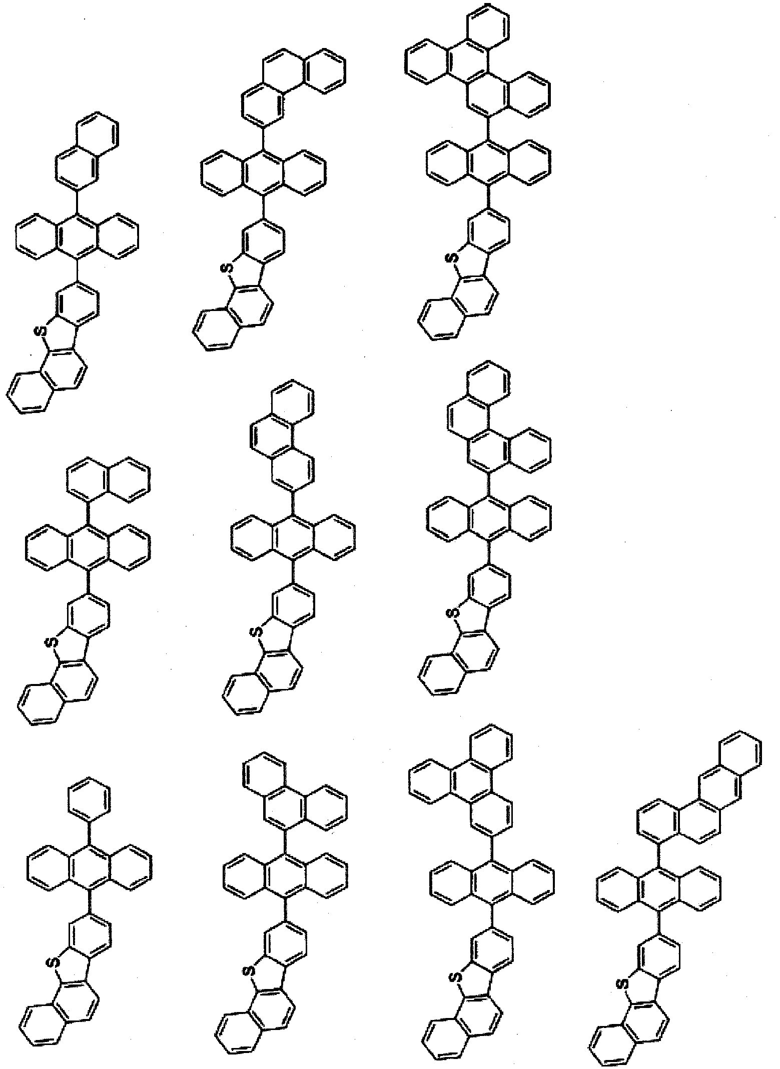

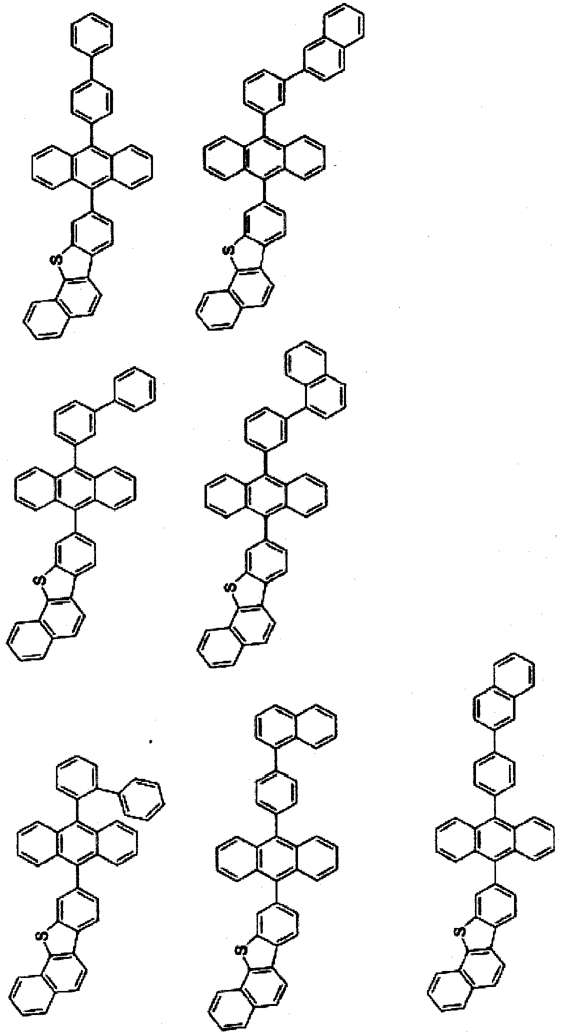

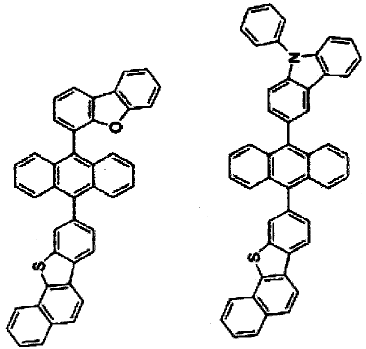

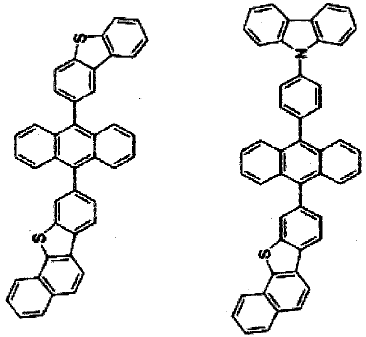

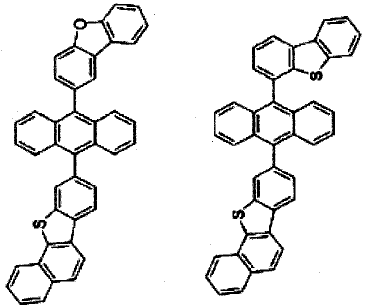

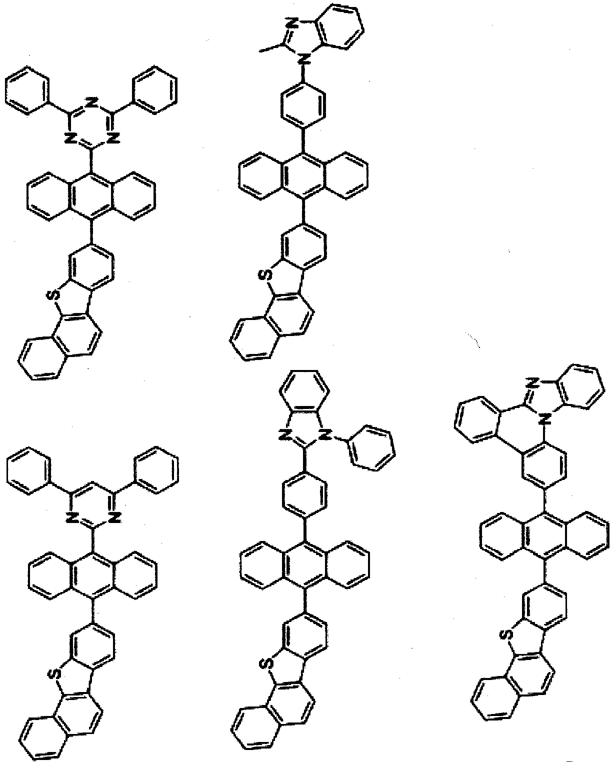

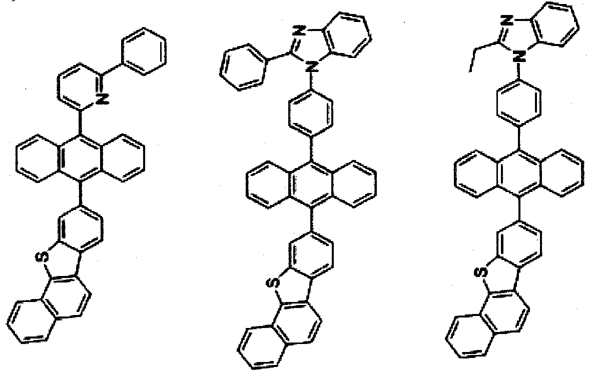

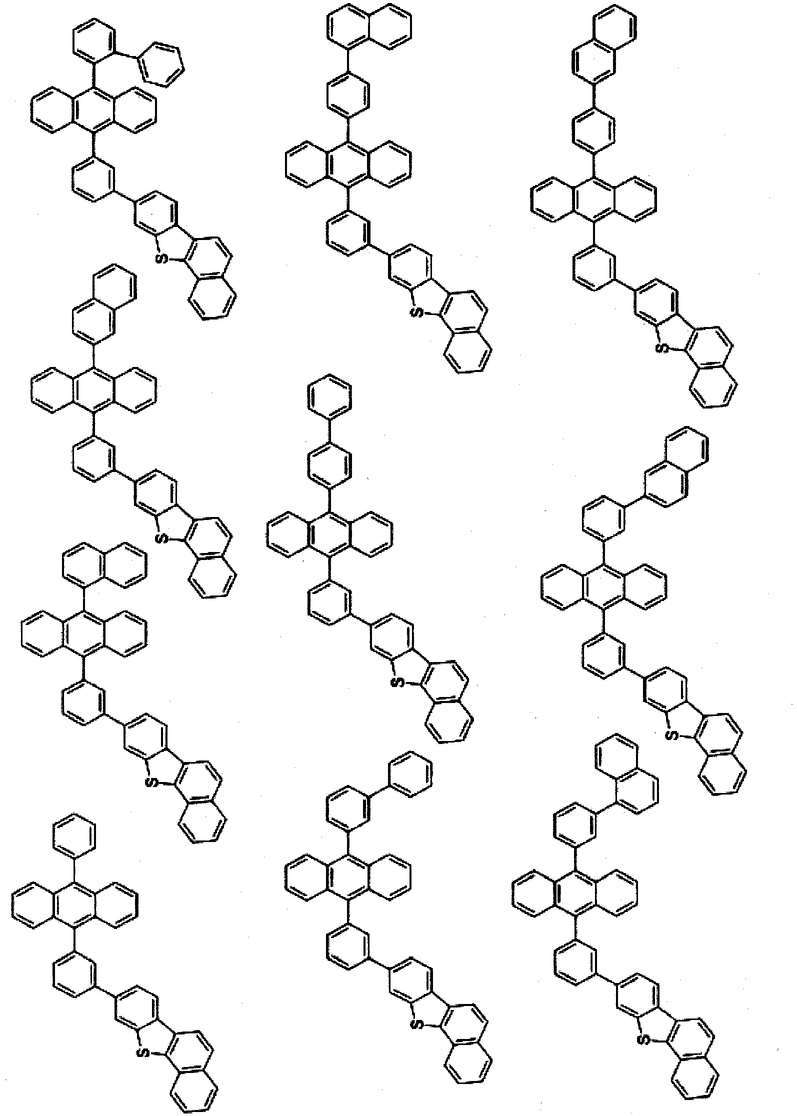

When the emitting layer described above includes the compound represented by the formula (1) and the compound represented by the formula (11), a content of the compound represented by the formula (1) is preferably 1 mass% or more and 20 mass% or less based on a total of the emitting layer.

Further, when the emitting layer described above includes the compound represented by the formula (1) and the compound represented by the formula (11), a content of the compound represented by the formula (11) is preferably 80 mass% or more and 99 mass% or less based on the total of the emitting layer.

One aspect of the organic EL element of the invention preferably has a hole-transporting layer between the anode and the emitting layer.

One aspect of the organic EL element of the invention preferably has an electron-transporting layer between the cathode and the emitting layer.

Specific examples of a typified element configuration of the organic EL element of the invention include structures such as
(1) an anode / an emitting layer / a cathode,
(2) an anode / a hole-injecting layer / an emitting layer / a cathode,
(3) an anode / an emitting layer / an electron-injecting-transporting layer / a cathode,
(4) an anode / a hole-injecting layer / an emitting layer / an electron-injecting-transporting layer / a cathode,
(5) an anode / an organic semiconductor layer / an emitting layer / a cathode,
(6) an anode / an organic semiconductor layer / an electron barrier layer / an emitting layer / a cathode,
(7) an anode / an organic semiconductor layer / an emitting layer / an adhesion improving layer / a cathode,
(8) an anode / a hole-injecting-transporting layer / an emitting layer / an electron-injecting-transporting layer / a cathode,
(9) an anode / an insulating layer / an emitting layer / an insulating layer / a cathode,
(10) an anode / an inorganic semiconductor layer / an insulating layer / an emitting layer / an insulating layer / a cathode,
(11) an anode / an organic semiconductor layer / an insulating layer / an emitting layer / an insulating layer / a cathode,
(12) an anode / an insulating layer / a hole-injecting-transporting layer / an emitting layer / an insulating layer / a cathode, and
(13) an anode / an insulating layer / a hole-injecting-transporting layer / an emitting layer / an electron-injecting-transporting layer / a cathode.

Among the above-described structures, a configuration of (8) is preferably used, but the configuration is not limited thereto.

Further, the emitting layer may be a phosphorescent emitting layer or a fluorescent emitting layer, and may include a plurality of emitting layers. When the organic EL element has the plurality of emitting layers, the organic EL element may have a space layer between the respective emitting layers for the purpose of preventing excitons generated in the phosphorescent emitting layer from diffusing into the fluorescent emitting layer.

Fig. 1 shows a schematic configuration of one example of the organic EL element in the embodiment of the invention.

An organic EL element 1 has a transparent substrate 2, an anode 3, a cathode 4, and an organic thin-film layer 10 arranged between the anode 3 and the cathode 4.

The organic thin-film layer 10 has an emitting layer 5 described above, and may have a hole-injecting-transporting layer 6 or the like between the emitting layer 5 and the anode 3, and an electron-injecting-transporting layer 7 or the like between the emitting layer 5 and the cathode 4.

Further, the electron barrier layer may be provided on a side of the anode 3 of the emitting layer 5, and a hole barrier layer may be provided on a side of the cathode 4 of the emitting layer 5, respectively.

Accordingly, electrons or holes can be confined in the emitting layer 5 to enhance generation probability of the excitons in the emitting layer 5.

Moreover, the "hole-injecting-transporting layer" herein means "at least one of the hole-injecting layer and the hole-transporting layer", and the "electron-injecting-transporting layer" herein means "at least one of the electron-injecting layer and the electron-transporting layer".

A substrate is used as a support of an emitting element. As the substrate, glass, quartz, plastics or the like can be used, for example. Further, a flexible substrate may be used. A term "flexible substrate" means a bendable (flexible) substrate, and specific examples thereof include a plastic substrate formed of polycarbonate or polyvinyl chloride.

For the anode formed on the substrate, metal, alloy, an electrically conductive compound, a mixture thereof or the like, each having a large work function (specifically 4.0 eV or more), is preferably used. Specific examples thereof include indium oxide-tin oxide (ITO: Indium Tin Oxide), silicon or silicon oxide-containing indium oxide-tin oxide, indium oxide-zinc oxide, tungsten oxide, zinc oxide-containing indium oxide, and graphene. In addition thereto, specific examples thereof include gold (Au), platinum (Pt), or nitride of a metallic material (for example, titanium nitride).

The hole-injecting layer is a layer including a material having high hole-injection properties. As the material having high hole-injection properties, such a material can be used as molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, an aromatic amine compound, a ladder-type compound such as a fluorene derivative, or a polymer compound (an oligomer, a dendrimer, a polymer or the like).

The hole-transporting layer is a layer including a material having high hole-transportation properties. For the hole-transporting layer, an aromatic amine compound, a carbazole derivative, an anthracene derivative, or the like can be used. A polymer compound such as poly(N-vinylcarbazole) (abbreviation: PVK) and poly(4-vinyltriphenylamine) (abbreviation: PVTPA) can also be used. However, a material other than the above-described materials may be used as long as the material has higher transportation properties of holes in comparison with the electrons. It should be noted that the layer including the material having high hole-transportation properties may be formed into not only a monolayer, but also a layer in which two or more layers formed of the above-described material are stacked.

The electron-transporting layer is a layer including a substance having high electron-transportation properties. For the electron-transporting layer, such a material can be used as 1) a metal complex such as a lithium complex, an aluminum complex, a beryllium complex and a zinc complex; 2) a heteroaromatic compound such as an imidazole derivative, a benzimidazole derivative, an azine derivative, a carbazole derivative and a phenanthroline derivative; and 3) a polymer compound.

The electron-injecting layer is a layer including a material having high electron-injection properties. For the electron-injecting layer, such a material can be used as alkali metal, alkaline earth metal or a compound thereof, such as lithium (Li), a lithium complex, lithium fluoride (LiF), cesium fluoride (CsF), calcium fluoride (CaF₂) and lithium oxide (LiOx).

For the cathode, metal, alloy, an electrically conductive compound, a mixture thereof, or the like, each having a small work function (specifically, 3.8 eV or less), is preferably used. Specific examples of such a cathode material include an element belonging to group 1 or group 2 of the periodic table of the elements, namely, alkali metal such as lithium (Li) and cesium (Cs), alkaline earth metal such as magnesium (Mg), and alloy containing the metal thereof (for example, MgAg and AILi).

In one aspect of the organic EL element of the invention, a method for forming each layer is not particularly limited. A conventionally-known method for forming each layer according to a vacuum deposition process, a spin coating process or the like can be used. Each layer such as the emitting layer can be formed by a publicly-know method such as a vacuum deposition process, a molecular beam deposition process (MBE process), or an application process such as a dipping process, a spin coating process, a casting process, a bar coating process and a roll coating process, using a solution prepared by dissolving the material in the solvent.

In one aspect of the organic EL element of the invention, a film thickness of each layer is not particularly limited, but is ordinarily preferably in the range of several nm to 1 µm generally in order to suppress a defect such as a pinhole, keep low applied voltage, or improve the luminous efficiency.

The organic EL element of the invention can be used for a displaying component such as an organic EL panel module; a display device of a television, a mobile phone, a personal computer or the like; and an electronic device such as an emitting device for lighting, a lighting appliance for a vehicle, or the like.

### Examples

Next, the invention will be described in greater detail by way of Examples and Comparative Examples, but the invention is not limited by the Examples.

### Example 1 (Synthesis of BD-1)

A scheme of synthesis of BD-1 is shown below.

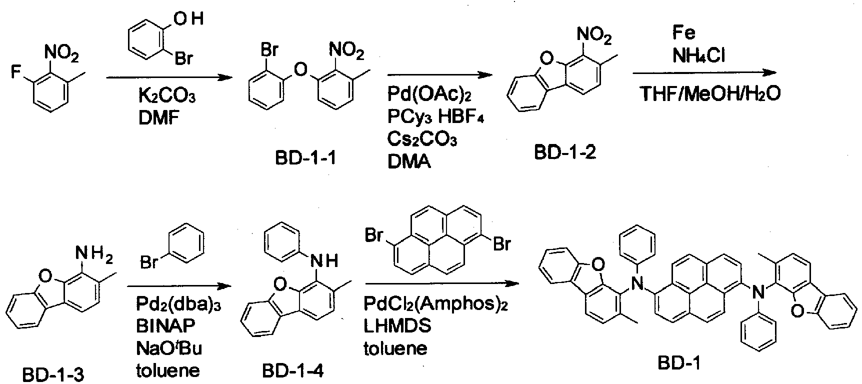

### (1-1) Synthesis of BD-1-1

Under an argon atmosphere, a mixture of 22.8 g of 1-fluoro-3-methyl-2-nitrobenzene, 25.4 g of 2-bromophenol, 40.6 g of potassium carbonate and 500 mL of dimethylformamide (DMF) was stirred at 120°C for 2 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by silica gel column chromatography to obtain 40.3 g of BD-1-1 (yield: 89%).

### (1-2) Synthesis of BD-1-2

Under an argon atmosphere, a mixture of 40.0 g of BD-1-1, 2.91 g of palladium acetate (Pd(OAc)₂), 9.56 g of tricyclohexylphosphine tetrafluoroborate (PCy₃HBF₄), 127 g of cesium carbonate and 400 mL of dimethylacetamide (DMA) was stirred at 130°C for 3 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by silica gel column chromatography to obtain 15.3 g of BD-1-2 (yield: 52%).

### (1-3) Synthesis of BD-1-3

Under an argon atmosphere, a mixture of 7.00 g of BD-1-2, 10.3 g of iron powder, 9.89 g of ammonium chloride, 80 mL of tetrahydrofuran (THF), 40 mL of methanol (MeOH) and 40 mL of water (H₂O) was stirred at 65°C for 4 hours. The resulting reaction liquid was cooled down to room temperature and subjected to Celite filtration, the resulting filtrate was washed with a saturated aqueous solution of sodium hydrogencarbonate, hexane was added thereto, and the resulting mixture was stirred and filtrated to obtain 4.48 g of BD-1-3 (yield: 74%).

### (1-4) Synthesis of BD-1-4

Under an argon atmosphere, a mixture of 4.42 g of BD-1-3, 3.20 g of bromobenzene, 0.373 g of tris(dibenzylideneacetone)dipalladium (Pd₂(dba)₃), 0.508 g of 2,2'-bis(diphenylphosphino)-1,1'-binaphthyl (BINAP), 3.92 g of sodium-t-butoxide (NaO^{t}Bu) and 100 mL of toluene was refluxed under heating for 16 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by silica gel column chromatography to obtain 4.24 g of BD-1-4 (yield: 76%).

### (1-5) Synthesis of BD-1

Under an argon atmosphere, a mixture of 4.15 g of BD-1-4, 2.60 g of dibromopyrene, 0.102 g of bis(di-t-butyl-4-dimethylaminophenylphosphine)dichloropalladium (Pd(Cl)₂(Amphos)₂), 14.4 mL of lithium bis(trimethylsilyl)amide (LHMDS) (1.0 M THF solution) and 50 mL of toluene was refluxed under heating for 3 hours. The resulting reaction liquid was cooled down to room temperature and filtrated, and the resulting residue was purified by silica gel column chromatography to obtain 4.49 g of BD-1 (yield: 83%).

### Example 2 (Synthesis of BD-2)

A scheme of synthesis of BD-2 is shown below.

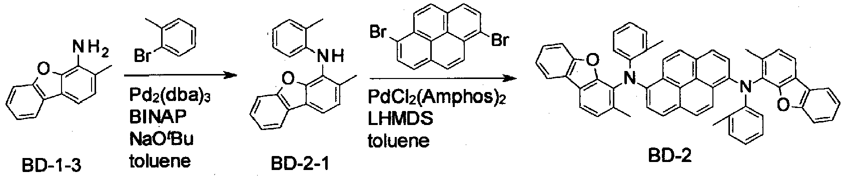

### (2-1) Synthesis of BD-2-1

Under an argon atmosphere, a mixture of 4.42 g of BD-1-3, 4.00 g of 1-bromo-2-methylbenzene, 0.428 g of tris dibenzylideneacetone dipalladium, 0.582 g of BINAP, 4.50 g of sodium-t-butoxide and 80 mL of toluene was refluxed under heating for 16 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by silica gel column chromatography to obtain 5.75 g of BD-2-1 (yield: 86%).

### (2-2) Synthesis of BD-2

Under an argon atmosphere, a mixture of 5.53 g of BD-2-1, 3.30 g of dibromopyrene, 0.130 g of bis(di-t-butyl-4-dimethylaminophenylphosphine)dichloropalladium, 18.3 mL of LHMDS (1.0 M THF solution) and 30 mL of toluene was refluxed under heating for 5 hours. The resulting reaction liquid was cooled down to room temperature and filtrated, and the resulting material was washed with water, methanol and toluene to obtain 3.32 g of BD-2 (yield: 47%).

### Example 3 (Synthesis of BD-3)

A scheme of synthesis of BD-3 is shown below.

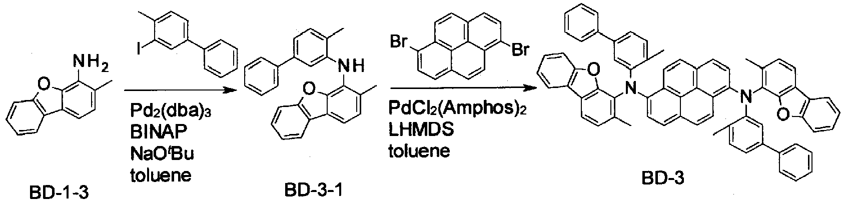

### (3-1) Synthesis of BD-3-1

Under an argon atmosphere, a mixture of 5.39 g of BD-1-3, 7.30 g of 3-iodo-4-methyl-1,1'-biphenyl, 0.455 g of tris dibenzylideneacetone dipalladium, 0.618 g of BINAP, 4.77 g of sodium-t-butoxide and 80 mL of toluene was refluxed under heating for 10 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by silica gel column chromatography to obtain 7.22 g of BD-3-1 (yield: 70%).

### (3-2) Synthesis of BD-3

Under an argon atmosphere, a mixture of 3.39 g of BD-3-1, 1.60 g of dibromopyrene, 0.0629 g of bis(di-t-butyl-4-dimethylaminophenylphosphine)dichloropalladium, 8.89 mL of LHMDS (1.0 M THF solution) and 30 mL of toluene was refluxed under heating for 16 hours. The resulting reaction liquid was cooled down to room temperature and filtrated, and the resulting residue was purified by silica gel column chromatography to obtain 1.75 g of BD-3 (yield: 43%).

### Example 4 (Synthesis of BD-4)

A scheme of synthesis of BD-4 is shown below.

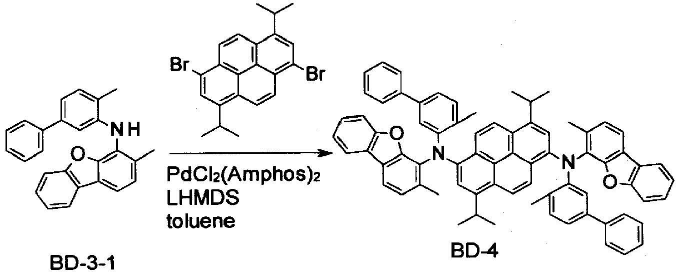

### (4-1) Synthesis of BD-4

Under an argon atmosphere, a mixture of 3.44 g of BD-3-1, 2.00 g of 1,6-dibromo-3,8-bis(1-methylethyl)pyrene, 0.0638 g of bis(di-t-butyl-4-dimethylaminophenylphosphine)dichloropalladium, 9.00 mL of LHMDS (1.0 M THF solution) and 30 mL of toluene was refluxed under heating for 20 hours. The resulting reaction liquid was cooled down to room temperature and filtrated, and the resulting residue was purified by silica gel column chromatography to obtain 2.05 g of BD-4 (yield: 45%).

### Example 5 (Synthesis of BD-5)

A scheme of synthesis of BD-5 is shown below.

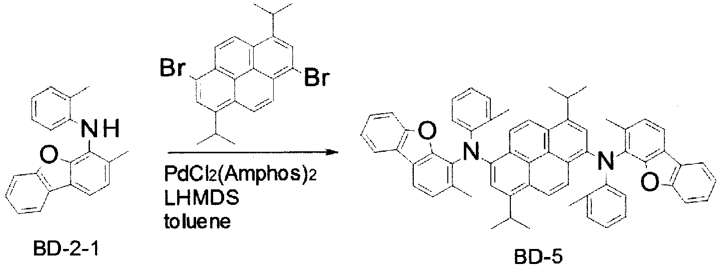

### (5-1) Synthesis of BD-5

Under an argon atmosphere, a mixture of 2.31 g of BD-2-1, 1.70 g of 1,6-dibromo-3,8-bis(1-methylethyl)pyrene, 0.054 g of bis(di-t-butyl-4-dimethylaminophenylphosphine)dichloropalladium, 7.65 mL of LHMDS (1.0 M THF solution) and 30 mL of toluene was refluxed under heating for 17 hours. The resulting reaction liquid was cooled down to room temperature and filtrated, and the resulting residue was purified by silica gel column chromatography to obtain 2.25 g of BD-5 (yield: 69%).

### Example 6 (Synthesis of BD-6)

A scheme of synthesis of BD-6 is shown below.

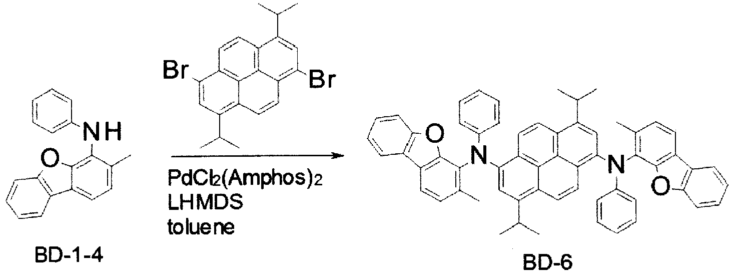

### (6-1) Synthesis of BD-6

Under an argon atmosphere, a mixture of 2.17 g of BD-1-4, 1.70 g of 1,6-dibromo-3,8-bis(1-methylethyl)pyrene, 0.054 g of bis(di-t-butyl-4-dimethylaminophenylphosphine)dichloropalladium, 7.56 mL of LHMDS (1.0 M THF solution) and 30 mL of toluene was refluxed under heating for 12 hours. The resulting reaction liquid was cooled down to room temperature and filtrated, and the resulting residue was purified by silica gel column chromatography to obtain 2.29 g of BD-6 (yield: 73%).

### Example 7 (Synthesis of BD-7)

A scheme of synthesis of BD-7 is shown below.

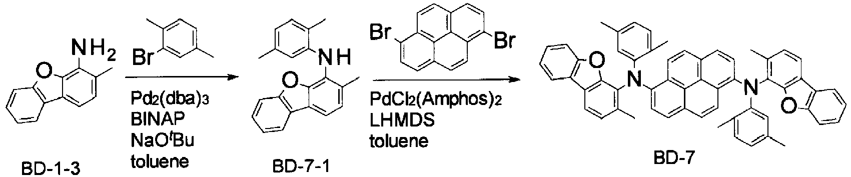

### (7-1) Synthesis of BD-7-1

Under an argon atmosphere, a mixture of 4.00 g of BD-1-3, 3.42 g of 2-bromoparaxylene, 0.338 g of tris(dibenzylideneacetone)dipalladium, 0.460 g of BINAP, 3.56 g of sodium-t-butoxide and 60 mL of toluene was refluxed under heating for 3 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by silica gel column chromatography to obtain 4.17 g of BD-7-1 (yield: 75%).

### (7-2) Synthesis of BD-7

Under an argon atmosphere, a mixture of 2.42 g of BD-7-1, 1.38 g of dibromopyrene, 0.054 g of bis(di-t-butyl-4-dimethylaminophenylphosphine)dichloropalladium, 7.65 mL of LHMDS (1.0 M THF solution) and 30 mL of toluene was refluxed under heating for 17 hours. The resulting reaction liquid was cooled down to room temperature and filtrated, and the resulting residue was purified by silica gel column chromatography to obtain 2.57 g of BD-7 (yield: 68%).

### Example 8 (Synthesis of BD-8)

A scheme of synthesis of BD-8 is shown below.

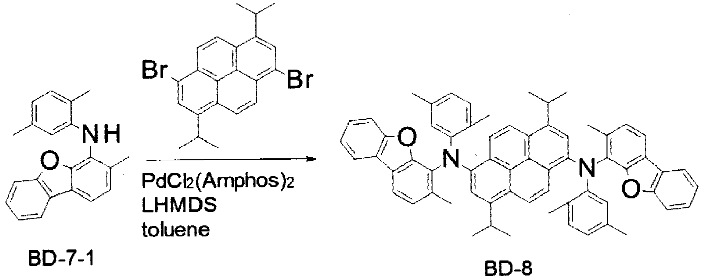

Under an argon atmosphere, a mixture of 2.17 g of BD-7-1, 1.68 g of 1,6-dibromo-3,8-bis(1-methylethyl)pyrene, 0.054 g of bis(di-t-butyl-4-dimethylaminophenylphosphine)dichloropalladium, 7.56 mL of LHMDS (1.0 M THF solution) and 30 mL of toluene was refluxed under heating for 12 hours. The resulting reaction liquid was cooled down to room temperature and filtrated, and the resulting residue was purified by silica gel column chromatography to obtain 2.29 g of BD-8 (yield: 73%).

### Example 9 (Synthesis of BD-9)

A scheme of synthesis of BD-9 is shown below.

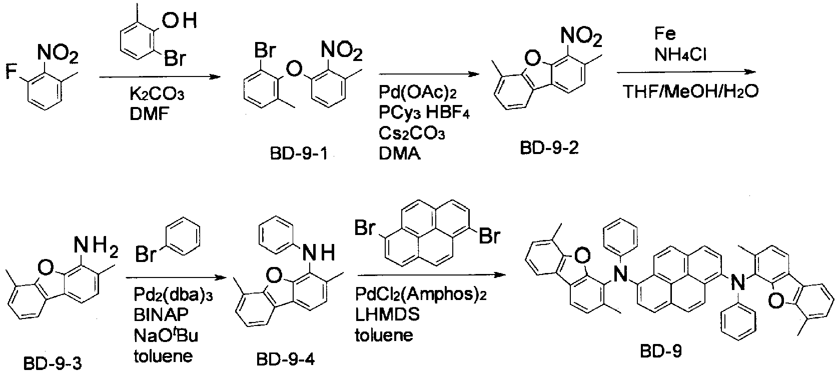

### (9-1) Synthesis of BD-9-1

Under an argon atmosphere, a mixture of 30.0 g of 1-fluoro-3-methyl-2-nitrobenzene, 36.1 g of 6-bromo-o-cresol, 53.3 g of potassium carbonate and 650 mL of dimethylformamide (DMF) was stirred at 120°C for 5 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by column chromatography to obtain 34.2 g of BD-9-1 (yield: 55%).

### (9-2) Synthesis of BD-9-2

Under an argon atmosphere, a mixture of 30.0 g of BD-9-1, 2.08 g of palladium acetate, 6.83 g of tricyclohexylphosphine tetrafluoroborate, 91 g of cesium carbonate and 280 mL of dimethylacetamide (DMA) was stirred at 130°C for 8 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by silica gel column chromatography to obtain 8.97 g of BD-9-2 (yield: 40%).

### (9-3) Synthesis of BD-9-3

Under an argon atmosphere, a mixture of 8.00 g of BD-9-2, 11.1 g of iron powder, 10.7 g of ammonium chloride, 90 mL of tetrahydrofuran (THF), 45 mL of methanol (MeOH) and 45 mL of water (H₂O) was stirred at 65°C for 4 hours. The resulting reaction liquid was cooled down to room temperature and subjected to Celite filtration, the resulting filtrate was washed with a saturated aqueous solution of sodium hydrogencarbonate, hexane was added thereto, and the resulting mixture was stirred and filtrated to obtain 4.77 g of BD-9-3 (yield: 68%).

### (9-4) Synthesis of BD-9-4

Under an argon atmosphere, a mixture of 4.00 g of BD-9-3, 2.70 g of bromobenzene, 0.315 g of tris(dibenzylideneacetone)dipalladium, 0.429 g of BINAP, 3.31 g of sodium t-butoxide and 80 mL of toluene was refluxed under heating for 16 hours. The resulting reaction liquid was cooled down to room temperature and subjected to extraction with ethyl acetate, and then an organic layer was washed with water and dried over anhydrous magnesium sulfate, and a solvent was distilled off under reduced pressure. The resulting residue was purified by silica gel column chromatography to obtain 3.42 g of BD-9-4 (yield: 63%).

### (9-5) Synthesis of BD-9

Under an argon atmosphere, a mixture of 3.40 g of BD-9-4, 2.00 g of dibromopyrene, 0.079 g of bis(di-t-butyl-4-dimethylaminophenylphosphine)dichloropalladium, 11.2 mL of LHMDS (1.0 M THF solution) and 40 mL of toluene was refluxed under heating for 3 hours. The resulting reaction liquid was cooled down to room temperature and filtrated, and the resulting residue was purified by silica gel column chromatography to obtain 3.09 g of BD-9 (yield: 72%).

Known alternative reaction or raw materials according to an intended product are used in copying the above-described reaction, whereby a compound within the scope of the present invention can be synthesized.

### Example 11

### (Preparation of organic EL element)

A 25 mm x 75 mm x 1.1 mm-thick glass substrate with an ITO transparent electrode (anode) (manufactured by GEOMATEC Co., Ltd.) was subjected to ultrasonic cleaning in isopropyl alcohol for 5 minutes, and then subjected to UV-ozone cleaning for 30 minutes. A film thickness of ITO was adjusted to 130 nm.

The glass substrate with the transparent electrode after being cleaned was mounted onto a substrate holder in a vacuum vapor deposition apparatus. First, a compound HI was deposited on a surface on a side on which the transparent electrode was formed so as to cover the transparent electrode to form a compound HI film having a film thickness of 5 nm. The HI film functions as a hole-injecting layer.

Subsequent to formation of the HI film, a compound HT1 was deposited thereon to form an HT1 film having a film thickness of 80 nm on the HI film. The HT1 film functions as a first hole-transporting layer.

Subsequent to formation of the HT1 film, a compound HT2 was deposited thereon to form an HT2 film having a film thickness of 10 nm on the HT1 film. The HT2 film functions as a second hole-transporting layer.

BH-1 (host material) and BD-1 (dopant material) were co-deposited on the HT2 film to be 4% in a proportion (mass ratio) of BD-1 to form an emitting layer having a film thickness of 25 nm.

HBL was deposited on the emitting layer to form an electron-transporting layer having a film thickness of 10 nm. ET as an electron-injecting material was deposited on the electron-transporting layer to form an electron-injecting layer having a film thickness of 15 nm. LiF was deposited on the electron-injecting layer to form a LiF film having a film thickness of 1 nm. Al metal was deposited on the LiF film to form a metal cathode having a film thickness of 80 nm.

An organic EL element was prepared as described above. Compounds used therefor are shown below.

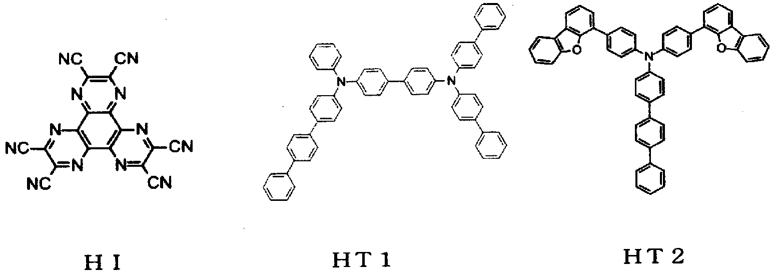

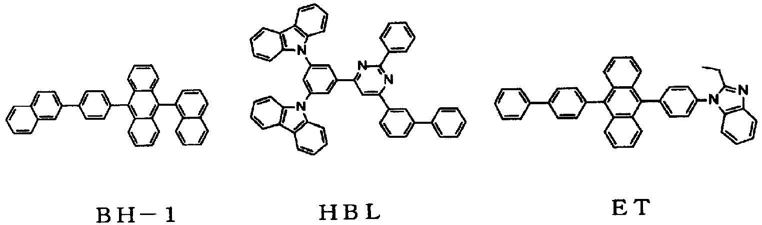

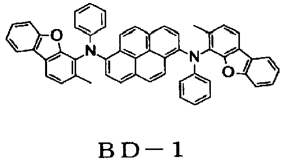

### (Evaluation of organic EL element)

Initial characteristics of the thus obtained organic EL element were measured by driving at a constant current of 10 mA/cm² of DC (direct current) at room temperature. The measurement results of voltage are shown in Table 1.

Furthermore, voltage was applied to the organic EL element to be 10 mA/cm² in current density to measure an EL emission spectrum by using Spectroradiometer CS-1000 (manufactured by Konica Minolta, Inc.). External quantum efficiency EQE (%) was calculated from the resulting spectral radiance spectrum. The results are shown in Table 1.

### Examples 12 to 17 and Comparative Examples 1 to 2

An organic EL element was prepared and evaluated in the same method as in Example 11 except that a compound shown in Table 1 was used as a dopant material. The results are shown in Table 1.

Compounds used therefor are shown below.

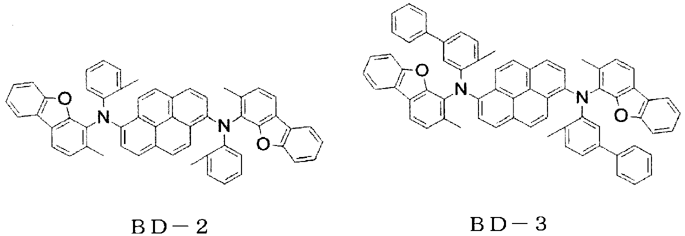

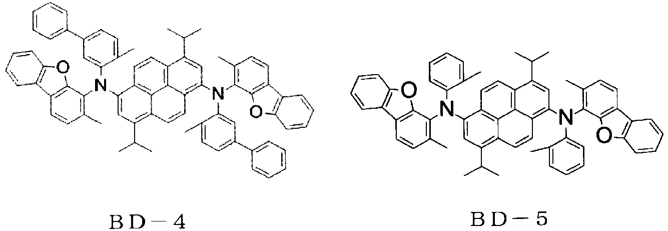

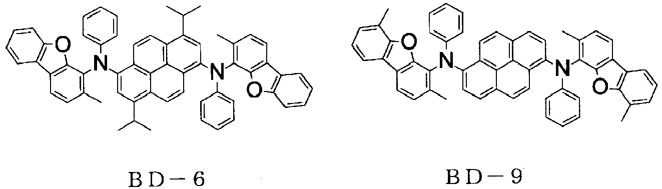

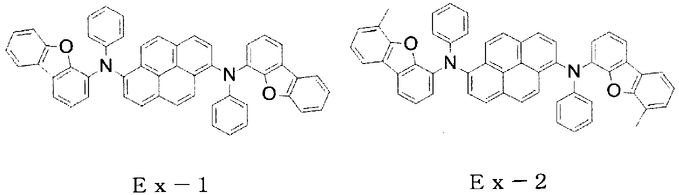

**[Table 1]**

| | Dopant material | Voltage (V) | EQE (%) |
|---|---|---|---|
| Example 11 | BD-1 | 3.67 | 9.4 |
| Example 12 | BD-2 | 3.66 | 9.2 |
| Example 13 | BD-3 | 3.65 | 8.9 |
| Example 14 | BD-4 | 3.66 | 9.3 |
| Example 15 | BD-5 | 3.65 | 9.1 |
| Example 16 | BD-6 | 3.67 | 9.4 |
| Example 17 | BD-9 | 3.66 | 9.1 |
| Comparative Example 1 | Ex-1 | 3.66 | 8.5 |
| Comparative Example 2 | Ex-2 | 3.66 | 8.6 |

From the results in Table 1, the organic EL elements using BD-1 to BD-6 and BD-9 in Examples 11 to 17 had higher external quantum efficiency in comparison with the organic EL elements in Comparative Examples 1 and 2.

The results described above show that, if the compound according to one aspect of the invention is used for the organic EL element, the organic EL element is excellent in luminous efficiency. The reason is estimated as described below.

It is considered that BD-1 to BD-6 and BD-9 each have a substituent in a 3-position of dibenzofuran of a side-chain substituent of a pyrene structure, whereby rigidity of an amino group to which dibenzofuran is bonded is increased to prevent vibrational deactivation from an excited state, leading to high luminous efficiency.

The compound according to one aspect of the invention has the substituent in a specific position of dibenzofuran, dibenzothiophene or the like of the side-chain substituent of the pyrene structure, whereby the rigidity of the amino group to which dibenzofuran, dibenzothiophene or the like is bonded is increased to prevent the vibrational deactivation from the excited state, leading to the high luminous efficiency.

Several embodiments and/or Examples of the invention have been described in detail above, but those skilled in the art will readily make a great number of modifications to the exemplary embodiments and/or Examples without substantially departing from new teachings and advantageous effects of the present invention. Accordingly, all such modifications are included within the scope of the invention.

The entire contents of the description of the Japanese application serving as a basis of claiming the priority concerning the present application to the Paris Convention are incorporated by reference herein.

## Claims

1. A compound, represented by the following formula (1): 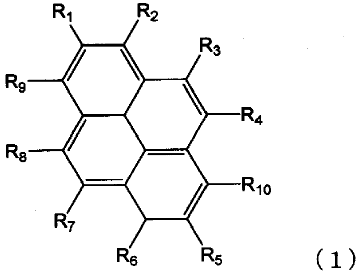 wherein, in the formula (1), R₁ to R₁₀ independently represent a hydrogen atom, a halogen atom, a cyano group, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a substituted or unsubstituted alkylthio group having 1 to 50 carbon atoms, a substituted or unsubstituted arylthio group having 6 to 50 ring carbon atoms, or a group represented by the following formula (2); and at least one of R₁ to R₁₀ is a group represented by the following formula (2): 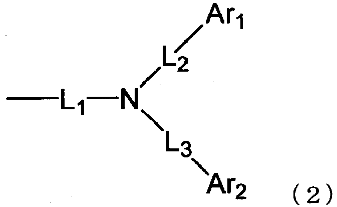
wherein, in the formula (2), Ar₁ and Ar₂ independently represent a substituted or unsubstituted aryl group having 6 to 50 ring carbon atoms, a substituted or unsubstituted heteroaryl group having 5 to 50 ring atoms, or a group represented by the following formula (3), and at least one of Ar₁ and Ar₂ is a group represented by the following formula (3);
L₁, L₂ and L₃ are independently a single bond, a substituted or unsubstituted arylene group having 6 to 30 ring carbon atoms, or a substituted or unsubstituted heteroarylene group having 5 to 30 ring atoms;
when the number of Ar₁, Ar₂, L₁, L₂ and L₃ is two or more, two or more of Ar₁, Ar₂, L₁, L₂ and L₃ may be the same with or different from each other: 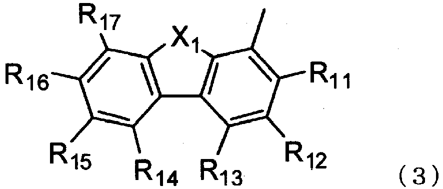
wherein, in the formula (3), R₁₁ represents a substituted or unsubstituted alkyl group having 1 to 50 carbon atoms, a substituted or unsubstituted aryl group having 6 to 50 ring carbon atoms, or a substituted or unsubstituted heteroaryl group having 5 to 50 ring atoms; R₁₂ to R₁₇ independently represent a hydrogen atom, a halogen atom, a cyano group, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a substituted or unsubstituted alkylthio group having 1 to 50 carbon atoms, or a substituted or unsubstituted arylthio group having 6 to 50 ring carbon atoms; and R₁₂ to R₁₇ may form a saturated or unsaturated ring between adjacent substituents; and
X₁ represents an oxygen atom or a sulfur atom.

2. The compound according to claim 1, wherein X₁ is an oxygen atom.

3. The compound according to claim 1 or 2, wherein L₁ is a single bond.

4. The compound according to any one of claims 1 to 3, wherein Ar₁ is a group represented by the formula (3), and Ar₂ is a substituted or unsubstituted aryl group having 6 to 50 ring carbon atoms.

5. The compound according to any one of claims 1 to 4, wherein L₂ and L₃ are a single bond.

6. The compound according to any one of claims 1 to 5, wherein two of R₁ to R₁₀ are a group represented by the formula (2).

7. The compound according to any one of claims 1 to 6, wherein R₁₂ to R₁₇ do not form a saturated or unsaturated ring between adjacent substituents.

8. The compound according to claim 1, represented by the following formula (4A): 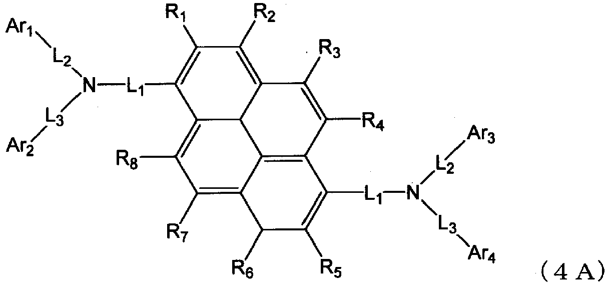
wherein, in the formula (4A), R₁ to R₈ are as defined in the formula (1);
Ar₁, Ar₂, L₁, L₂ and L₃ are as defined in the formula (2);
Ar₃ and Ar₄ independently represent a substituted or unsubstituted aryl group having 6 to 50 ring carbon atoms, a substituted or unsubstituted heteroaryl group having 5 to 50 ring atoms, or a group represented by the formula (3); and
at least one of Ar₃ and Ar₄ is a group represented by the formula (3).

9. The compound according to claim 1, represented by the following formula (4B): 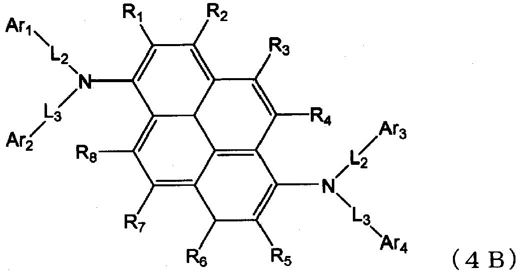
wherein, in the formula (4B), R₁ to R₈ are as defined in the formula (1);
Ar₁, Ar₂, L₂ and L₃ are as defined in the formula (2);
Ar₃ and Ar₄ independently represent a substituted or unsubstituted aryl group having 6 to 50 ring carbon atoms, a substituted or unsubstituted heteroaryl group having 5 to 50 ring atoms, or a group represented by the formula (3); and
at least one of Ar₃ and Ar₄ is a group represented by the formula (3).

10. The compound according to claim 1, represented by the following formula (4): 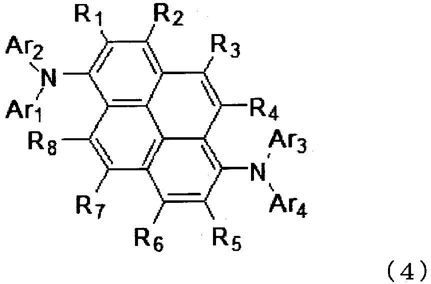
wherein, in the formula (4), R₁ to R₈ are as defined in the formula (1);
Ar₁ and Ar₂ are as defined in the formula (2);
Ar₃ and Ar₄ independently represent a substituted or unsubstituted aryl group having 6 to 50 ring carbon atoms, a substituted or unsubstituted heteroaryl group having 5 to 50 ring atoms, or a group represented by the formula (3); and
at least one of Ar₃ and Ar₄ is a group represented by the formula (3).

11. The compound according to any one of claims 8 to 10, wherein
R₁ to R₈ are independently a hydrogen atom, or a substituted or unsubstituted alkyl group having 1 to 50 carbon atoms;
Ar₁ and Ar₃ are independently a group represented by the formula (3);
Ar₂ and Ar₄ are independently a substituted or unsubstituted aryl group having 6 to 50 ring carbon atoms;
R₁₁ is independently a substituted or unsubstituted alkyl group having 1 to 50 carbon atoms, or a substituted or unsubstituted aryl group having 6 to 50 ring carbon atoms; and
R₁₂ to R₁₇ are independently a hydrogen atom, a substituted or unsubstituted alkyl group having 1 to 50 carbon atoms, or a substituted or unsubstituted aryl group having 6 to 50 ring carbon atoms.

12. The compound according to any one of claims 8 to 10, wherein
R₁ to R₈ are independently a hydrogen atom, or a substituted or unsubstituted alkyl group having 1 to 18 carbon atoms;
Ar₁ and Ar₃ are independently a group represented by the formula (3);
Ar₂ and Ar₄ are independently a substituted or unsubstituted aryl group having 6 to 18 ring carbon atoms;
R₁₁ is independently a substituted or unsubstituted alkyl group having 1 to 18 carbon atoms, or a substituted or unsubstituted aryl group having 6 to 18 ring carbon atoms; and
R₁₂ to R₁₇ are independently a hydrogen atom, a substituted or unsubstituted alkyl group having 1 to 18 carbon atoms, or a substituted or unsubstituted aryl group having 6 to 18 ring carbon atoms.

13. The compound according to claim 11 or 12, wherein Ar₂ and Ar₄ are independently a substituted or unsubstituted phenyl group, a substituted or unsubstituted biphenyl group, or a substituted or unsubstituted naphthyl group.

14. The compound according to any one of claims 1 to 13, wherein R₁₁ is a substituted or unsubstituted alkyl having 1 to 8 carbon atoms.

15. The compound according to any one of claims 1 to 14, wherein R₁ to R₈ are a hydrogen atom.

16. The compound according to any one of claims 1 to 15, wherein the compound is a material for an organic electroluminescent element.

17. An organic electroluminescent element, comprising:
a cathode;
an anode; and
an emitting layer arranged between the cathode and the anode,
wherein the emitting layer includes the compound according to any one of claims 1 to 16.

18. The organic electroluminescent element according to claim 17, wherein the emitting layer further includes a compound represented by the following formula (11): 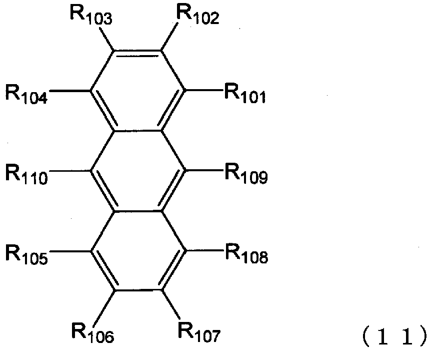
wherein, in the formula (11), R₁₀₁ to R₁₁₀ are independently a hydrogen atom, a halogen atom, a cyano group, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a substituted or unsubstituted alkylthio group having 1 to 50 carbon atoms, a substituted or unsubstituted arylthio group having 6 to 50 ring carbon atoms, or a group represented by -L₁₀₁-Ar₁₀₁,
at least one of R₁₀₁ to R₁₁₀ is the group represented by -L₁₀₁-Ar₁₀₁,
L₁₀₁ is a single bond, a substituted or unsubstituted arylene group having 6 to 30 ring carbon atoms, or a substituted or unsubstituted heteroarylene group having 5 to 30 ring atoms,
Ar₁₀₁ is a substituted or unsubstituted aryl group having 6 to 50 ring carbon atoms, or a substituted or unsubstituted heteroaryl group having 5 to 50 ring atoms,
when the number of L₁₀₁ is two or more, two or more of L₁₀₁ may be the same with or different from each other,
and when the number of Ar₁₀₁ is two or more, two or more of Ar₁₀₁ may be the same with or different from each other.

19. The organic electroluminescent element according to claim 18, wherein at least one of R₁₀₉ and R₁₁₀ is a group represented by the -L₁₀₁-Ar₁₀₁.

20. The organic electroluminescent element according to claim 18, wherein R₁₀₉ and R₁₁₀ are independently a group represented by the -L₁₀₁-Ar₁₀₁.

21. The organic electroluminescent element according to any one of claims 17 to 20, comprising a hole-transporting layer between the anode and the emitting layer.

22. The organic electroluminescent element according to any one of claims 17 to 21, comprising an electron-transporting layer between the cathode and the emitting layer.

23. An electronic device, comprising the organic electroluminescent element according to any one of claims 17 to 22.
